# EUROPEAN PATENT SPECIFICATION

(11) **EP 2 417 270 B1**
(45) Date of publication and mention of the grant of the patent: **20.09.2017**
(21) Application number: 10762298.7
(22) Date of filing: 06.04.2010
(51) Int. Cl.: C12Q 1/68, C12N 15/11, G01N 33/52

(54) **DIGITAL QUANTIFICATION OF DNA METHYLATION**
DIGITALE QUANTIFIZIERUNG VON DNA-METHYLIERUNG
QUANTIFICATION DE LA MÉTHYLATION DE L'ADN

(30) Priority: 06.04.2009 US 166970 P
(43) Date of publication of application: 15.02.2012
(73) Proprietor: The Johns Hopkins University, Baltimore, MD 21218 (US); Case Western Reserve University, Cleveland, OH 44106-7285 (US)
(72) Inventor: VOGELSTEIN, Bert, Baltimore, MD 21208 (US); KINZLER, Kenneth, W., Baltimore, MD 21015 (US); LI, Meng, Baltimore, MD 21234 (US); DIAZ, Luis, Ellicott City, MD 21075 (US); PAPADOPOULOS, Nickolas, Towson, MD 21204 (US); MARKOWITZ, Sanford, Pepper Pike, OH 44124 (US)
(74) Representative: Tombling, Adrian George
(86) International application number: PCT/US2010/030084
(87) International publication number: WO 2010/118016

(56) References cited:
- US-A1- 2005 106 593
- US-A1- 2007 065 823
- US-A1- 2008 145 898
- MENG LI ET AL: "BEAMing up for detection and quantification of rare sequence variants", NATURE METHODS, NATURE PUBLISHING GROUP, GB, vol. 3, no. 2, 1 February 2006 (2006-02-01), pages 95-97, XP008126872, ISSN: 1548-7091, DOI: 10.1038/NMETH850
- DIEHL FRANK ET AL: "BEAMing: single-molecule PCR on microparticles in water-in-oil emulsions", NATURE METHODS, NATURE PUBLISHING GROUP, GB, vol. 3, no. 7, 1 July 2006 (2006-07-01), pages 551-559, XP002510523, ISSN: 1548-7091, DOI: 10.1038/NMETH898 [retrieved on 2006-06-21]
- W.-D. CHEN ET AL: "Detection in Fecal DNA of Colon Cancer-Specific Methylation of the Nonexpressed Vimentin Gene", JNCI JOURNAL OF THE NATIONAL CANCER INSTITUTE, vol. 97, no. 15, 3 August 2005 (2005-08-03), pages 1124-1132, XP55000481, ISSN: 0027-8874, DOI: 10.1093/jnci/dji204
- MENG LI ET AL: "Sensitive digital quantification of DNA methylation in clinical samples", NATURE BIOTECHNOLOGY, vol. 27, no. 9, 16 August 2009 (2009-08-16), pages 858-863, XP55034577, ISSN: 1087-0156, DOI: 10.1038/nbt.1559
- DRESSMAN, D. ET AL.: 'Transforming single DNA molecules into fluorescent magnetic particles for detection and enumeration of genetic variations' PROC. NATL. ACAD. SCI. USA vol. 100, 11 July 2003, pages 8817 - 8822, XP002327377
- KOJIMA, T. ET AL.: 'PCR amplification from single DNA molecules on magnetic beads in emulsion application for high-throughput screening of transcription factor targets' NUCLEIC ACIDS RES. vol. 33, no. 17, 06 October 2005, pages 1 - 9, XP008164954

### SEQUENCE LISTING

<110> Vogelstein, Bert Kinzler, Kenneth W. Li, Meng Diaz, Luis Papadopoulos, Nickolas Markowitz, Sanford
<120> DIGITAL QUANTIFICATION OF DNA
   METHYLATION
<130> 001107.00770
<160> 29
<170> FastSEQ for Windows Version 4.0
<210> 1
   <211> 106
   <212> DNA
   <213> Homo sapiens
<400> 1
   ctcgtcctcc taccgcagga tgttcggcgg cccgggcacc gcgagccggc cgagctccag 60
   ccggagctac gtgactacgt ccacccgcac ctacagcctg ggcagc 106
<210> 2
   <211> 23
   <212> DNA
   <213> Homo sapiens
<220>
   <221> misc_feature
   <222> (1)...(23)
   <223> n = A,T,C or G
<400> 2
   gttgtttagg ttgtaggtgn ggg 23
<210> 3
   <211> 25
   <212> DNA
   <213> Homo sapiens
<220>
   <221> misc_feature
   <222> (1)...(25)
   <223> n = A,T,C or G
<400> 3
   ctcntcctcc taccncaaaa tattc 25
<210> 4
   <211> 33
   <212> DNA
   <213> Homo sapiens
<400> 4
   cgccaccctc cgcagccatg tccaccaggt ccg 33
<210> 5
   <211> 35
   <212> DNA
   <213> Homo sapiens
<400> 5
   ggccatcgcc accctccgca gccatgtcca ccagg 35
<210> 6
   <211> 21
   <212> DNA
   <213> Homo sapiens
<400> 6
   tggcacatat acaccatgga a 21
<210> 7
   <211> 21
   <212> DNA
   <213> Homo sapiens
<400> 7
   tgatggtttc caatttcatc c 21
<210> 8
   <211> 21
   <212> DNA
   <213> Homo sapiens
<400> 8
   tggcacatat acaccatgga a 21
<210> 9
   <211> 24
   <212> DNA
   <213> Homo sapiens
<400> 9
   tgtccctaca aaggatatga actc 24
<210> 10
   <211> 20
   <212> DNA
   <213> Homo sapiens
<400> 10
   gggtggacgt agtcacgtag 20
<210> 11
   <211> 19
   <212> DNA
   <213> Homo sapiens
<400> 11
   cctcctaccg caggatgtt 19
<210> 12
   <211> 18
   <212> DNA
   <213> Homo sapiens
<400> 12
   ctgtaggtgc gggtggac 18
<210> 13
   <211> 19
   <212> DNA
   <213> Homo sapiens
<400> 13
   cctcctaccg caggatgtt 19
<210> 14
   <211> 19
   <212> DNA
   <213> Homo sapiens
<400> 14
   cctcctaccg caggatgtt 19
<210> 15
   <211> 18
   <212> DNA
   <213> Homo sapiens
<400> 15
   ctgcccaggc tgtaggtg 18
<210> 16
   <211> 45
   <212> DNA
   <213> Homo sapiens
<400> 16
   tcccgcgaaa ttaatacgac ctcgtcctcc taccgcaaaa tattc 45
<210> 17
   <211> 23
   <212> DNA
   <213> Homo sapiens
<400> 17
   gttgtttagg ttgtaggtgc ggg 23
<210> 18
   <211> 45
   <212> DNA
   <213> Homo sapiens
<400> 18
   tcccgcgaaa ttaatacgac ctcatcctcc taccacaaaa tattc 45
<210> 19
   <211> 23
   <212> DNA
   <213> Homo sapiens
<400> 19
   gttgtttagg ttgtaggtgt ggg 23
<210> 20
   <211> 20
   <212> DNA
   <213> Homo sapiens
<400> 20
   tcccgcgaaa ttaatacgac 20
<210> 21
   <211> 23
   <212> DNA
   <213> Homo sapiens
<400> 21
   gttgtttagg ttgtaggtgc ggg 23
<210> 22
   <211> 23
   <212> DNA
   <213> Homo sapiens
<400> 22
   gttgtttagg ttgtaggtgt ggg 23
<210> 23
   <211> 20
   <212> DNA
   <213> Homo sapiens
<400> 23
   tcccgcgaaa ttaatacgac 20
<210> 24
   <211> 22
   <212> DNA
   <213> Homo sapiens
<400> 24
   tcggtcggtt cgcggtgttc ga 22
<210> 25
   <211> 22
   <212> DNA
   <213> Homo sapiens
<400> 25
   ttggttggtt tgtggtgttt ga 22
<210> 26
   <211> 35
   <212> DNA
   <213> Homo sapiens
<400> 26
   gtgygggtgg aygtagttay gtagtttygg ttgga 35
<210> 27
   <211> 94168
   <212> DNA
   <213> Homo sapiens
<400> 27
   aacagtgaga gggccctcac gtatacactg atccatgggt aaatggaacc gagagagtgg 60
   cattgtctct gcttttagat tcttgccagt aacaccactg taagtgagtg aaggcttaac 120
   tcttttgttt ttggcttgtg gccttaatca gctactttaa cgctcttgac agctgagctt 180
   tctctttccc agctctgctg agaccctgac aacaacactg aataatgtcc taaaaaattg 240
   atagtttcat ttatctgcaa agacatattt tttgtgtctc actgcctctt cccactccga 300
   ttacattatt atttctacac tgcaaagctt tacaacattt tcagctattt tctaaccata 360
   gttttcctcc taacacggca gcttcattcc tgggttttct actttgattc atctcttgct 420
   cagctagatt aagtgatgca gtcatctgtt ccagaagggc tcatggatgt tatattgtaa 480
   atttcattct agaaaaattt ttatctattt ttattaagag tttttgttcc actggagtca 540
   ttctcctctt cagaggcatt aatgatattt atcacggatc actctgtctt cggtagctat 600
   catttactct acagtatgta actggagaaa gtgaagaaag agaacagaga ttatttttga 660
   agcaataatg gtcaaacccc ccaaatttga tgaaaattat aaataagaaa agcaccacaa 720
   ggtccaagca ctacaaataa agaaaaacat acagagcaca tcaaaataaa acttttcaaa 780
   acacgtgata aagataaaat cttaaatgta gacaaagaaa gacacaatat attaagagga 840
   atgaaagcag aaattattac agaccttaag tcagaaatta agaaacccag ataacactgg 900
   aaagtattaa aagaaaacaa acagattttt acaaaaatat ctttcaaaat gaatgcaatc 960
   aaagattcag tgggccaggc acagtggctc atgcctgtaa tcccagcact ttgagaggct    1020
   gatatgggag gaccagttga gcccaagagt ttgagaccag cctgggcaac atagtgagac    1080
   cctgcctctc caaaaaagaa aaaaaaatga gtaaaaatta tatttcacag tgaagacaaa    1140
   ataaaacact tttttactca aaagcaccaa ggtaatttgt ccctttccac atataaccaa    1200
   caagagacac taaagaaggt ttttcaggaa gaagaaaaat tatatcacat gcaaactttg    1260
   atctaaaaca caaggataaa aagtgctgaa aattgtaggt atatagataa atataaaaga    1320
   ctttgtgctt tttaaaagtt ctttaataga tatttgacta tttaatccaa aaaacagtaa    1380
   caatgtatgt tggtgcctat aacattcgta gcagtaaagt gcaagacaaa atatcaaaat    1440
   agaccagtag gggaaattgg aggaatactg ttaaaattta aacataaagt ggtataatgt    1500
   tatttgaagg cgggcttcca caagtaaaag atgcatcttg taaaccctag agaatccaac    1560
   aggaaaataa aacaaagata gatatagata tttagatatt taaaaataaa tttcagttta    1620
   cattgaataa atattatgac taattgtctt taaaaagaca ttagtgaaga ataatgaatt    1680
   ttttaaaaat ccaaataaaa atagtgacaa tgtaaaatgg taaaaacact tcaaaaactt    1740
   tgaaaatttg gtattttctc ataaagttta ataaatgtag gtatttatcc acaataaatg    1800
   aaaactaaga tccacacaga tttgtacata tatgttcatt gcaactttac ttgtaatggt    1860
   aaaaaactag aaactactta aatgtctata ataatgtgaa tacatacaca tattgtagta    1920
   tatgatattc tactgtacaa aatatcactc aacaataaaa aagagcaaat tattgatata    1980
   caacaaaaca tgatgaattc caaaaccatc aacactaaac aaaagcagtt ccactcaaaa    2040
   gaactacact cttatgattt tatttgtgat atactaggaa gggaaaactt aatccataat    2100
   gaaaagaaag cagatccatt gtcgcctggg ttggggttgg agactgacag aaatgtggca    2160
   ctaagaacaa tttagcatga tggaagtgtt ctaagtcttg attgtggtgg tagtagttac    2220
   acagttgatg attgagtcga aactcaaact gcccacttaa aattacgtac aataaactac    2280
   atatttattg catgtaaatt atgagaaatt tcatgagaaa tttgtttgta aaatttttca    2340
   tgataaatta ggagaaacaa cacaatttct tttttttttt tttttttttg agacaaagtc    2400
   gaccaggctg gagtgcagtg gcgcgatctc agctcactgc aacctccgcc tcctgggttc    2460
   aagcgattct cctgcctcag cctcccaagt agctgggact acaggcatgt gccaacacac    2520
   ctggctaatt tttgtatttt tagtagagac caggttttgc tatgtcagcc aggctggtct    2580
   cgaactcata acttcgtgat ccacctgtct cggcctccca aagtgctggc attacaggcg    2640
   tcagccaccg cgcccagcca atttcacatt atttaaaaaa taactcaaca taaatcagtg    2700
   tttcaatact tgtgctgaag tcaccacagg tgaaaaaggt taaccttgac aggtttgcta    2760
   aaataagaag cattcagtaa aagccaatga ttttctccct tgttcttact gcatgctata    2820
   tatattgcat aagtatttaa taaaagtaaa tactttttcg aagttcaata tcaccacaga    2880
   accaagcatt tagaagtcaa gcatatcctt acatttcaat tatttctctg aaagaccaaa    2940
   atattgatct ccctgtctat agctggactt ttttttaaaa gcatgctatc ttctgatagg    3000
   atataaaatg ttaatctcct tctcctgaac tcttaaattt atatatgctt tatcttccaa    3060
   ttaagacaac ggaaaccagt attaggcagt gtttttcaat cacatttccc ttgctacata    3120
   cttattaaaa caatgacaat tactcacttc aatcgtcttg gcaagtaact gtgtgtgagg    3180
   aaaattatac ttgtatactt cattagcgac agtgttgaca tcaatggcag ccaccacttg    3240
   tgcaggtata cagctttctg taatgataaa tggagtatct tgaaaagaaa agtccttaag    3300
   ttcattatag aaagaaatca aaatagcaga atttagtaat ccatttcctt atttcttcat    3360
   ttattagcct catctcagac tgtcagtgga aggtcaggcc aggggaagcc aggcatgaag    3420
   tgaaatattt gaacatggac ttctggttca tctctctaaa cagcattcca atactaaaga    3480
   gaagatttaa atattttagt gcttacctct tatatttgct ttcctccctc attaagagac    3540
   acacttataa gtcaaataga aagttttcct aagttaaaac aaactaaaaa ttgaaaagtg    3600
   agatatattc taaaagccct atgatttaga actttatata aataaactac catttgttct    3660
   gcaccttctc acattaatac aatgttgctt cacagtacca ttgaaaatgc tttaattgtt    3720
   tttttagtaa cagctttatt gagatgtaat tcacatttga caaaattcac ccaattagag    3780
   cttacataag tttttaaatg agatgaacaa ttaattggta tccaaatgta aatggttcat    3840
   ttaccaacat tttaaatgtt gacagaccag aagagaatat aagaagaata agggaaataa    3900
   ttataaagga atgccttcac tgcccttctg tcttgctggt gaagaaaacc attttcaaaa    3960
   tgttttcaga tccagagaat tctagcatat tctttaatgt tagccgaaat tggaactcca    4020
   caattgtagc cgtagctata ttttacaatg accaccctct ttccccaaat atgtataaaa    4080
   ccaattatgt gcatgccctg acccaagtca ggagaaagtg tgaagtttta aaaatgaaag    4140
   tatttgagac ttcagaaaat catcagagaa agcaatacaa tattacatag tagatccaag    4200
   tagccgattc tttaatacca ttagttgatt aatagttagc tgattagctt accccaaata    4260
   tcaaaaaagg aaaaaaaaaa aaaaagagtg gtgtgaggaa ctaggactca tgcagcaggc    4320
   acccggaaat aaaagcagac cacaggagac agggcaagga gaaaggctca cacacgctca    4380
   gaaaacaatc ataaccttcc actgaggcct agaagagatg aggccaggaa gaaaagaaag    4440
   gcacaaatgt ctccaaggat tgatactaaa tggctgctag gctcaagaca ctcagcaggt    4500
   atcctgctga aaagtaagat cccctgcgtg ctgcaaactt ggtggtctaa tcagtaatgc    4560
   catagacagc tgcctcttct gcattgaagt gcaataacta gttactggcc ttagtataaa    4620
   ataaatttga ggttataaat tgtatctgtt aaagccatca gatcttgaaa cataaatcaa    4680
   gaaggcttct aaacaaaaga aaatattaat atataaggat ccatttgcaa acacaaaact    4740
   atcataattc aagtcaagaa ttaagctact actaatcact gtaagagagc tgaaaattaa    4800
   ataaacttca ttgtactatt ttactgtttt gttaaatatg ttgtaatcag catgatgtag    4860
   cctgcttgta tttccaccat atatgaagca aactgggatt gcctgacatt ggttaatagg 4920
   acttgtatct tttttcaggc agagtgaaaa tgtacagtga tgtgctgaat ctgcactgta 4980
   atcaataatt aattttatgt tcttgtgtta tatgcctgga ggtcataaaa actgacaaaa 5040
   atcaactgcc aaatgagtat ttcatatggt gatccaaaaa ctgatgaact gaaatttctt 5100
   tctcaggaaa gaacagattt tcaaagccaa taagcaaatg cactgtttgc atggattgat 5160
   gcacatattt tagactgtta taaaattagg acctactgca taacttaaaa agttatttct 5220
   attaagagaa atggatgtac taagatatta ctgacactaa ttcaacttgc cttctcaaaa 5280
   tagaattggg gggtggttat tgtttctaag gaaaaggtta aacttacaga taagttttaa 5340
   aaagcaaacc tgtcaattct gtcaaaatct taacttgaat tccaaagact agttgccttg 5400
   atatttctta gtatacagtt taaatctaca atctccaaaa ttctttactt ttaagttaca 5460
   tatatagatt ttgaaaatgt ataattaaaa atgtcaaagt gaatataatt ctatagaatt 5520
   ttgcctatgt gagctccatc acttgagtga tccaagagta gtaattctca aaaaggaggc 5580
   agaatttggg aaagggattc gtgagtatct tttcattgaa acagaggaag aggatgtgtt 5640
   ttcagtactt tgatgtatta ctttgcctag actttattca tgatatttca gttattattc 5700
   aggatttcat atttattaac aggaagcctg gtttaaaaaa aaaaggtgag atatcctact 5760
   tagagaaaca catttatttt atttatttat ttatttattt atttattttt tgagatagtg 5820
   tctcactctg ttgcccaggc tggagttcag tggcatgatc tcggctcact gcaacctcca 5880
   cctctcaggt tcaagcaatt tgattctcct gcctcagtct ccagagtaac agaaactaca 5940
   gacacttgcc accacaccca gctaatttct atatttttag tagggacagg gtttcaccat 6000
   attggccagg ctggtctcaa actcctaacc tcaagtgatc tgcctgcctc agccttccaa 6060
   agtgctggga ttacaggtgt gggccaccac gcttggctga aaaacacagt acaaattatt 6120
   ttaaaaggtt gtcagattgg ctccgaaagg aatacataag gacatcacgc ttccatttat 6180
   atgaggacta aatttaaaag gtagatttag ttatttcaag agagacttct aggtaagatg 6240
   ttagaaatgt cactgaggat acatacataa ttcttgcttt caatatgctt cttacaaaaa 6300
   tagacaagaa acacacacac acacacacac acacacacac acagacattt gaatagcaat 6360
   actttccttt ctttttctga tgattcttct ttctggaaac gctgtaggga agaacttttt 6420
   gttggtagta gtgaaaagct ttacaagttc acactcttct tgactttcct aaccaaataa 6480
   ataaaaaaat ttaactgttc ttgtgtagtt attaatgtgg gaaaaaaatc aatatttaca 6540
   agatgtaaat gcaaatgtaa aaaatgtaaa tgtaaagacg ttatgttaga taatataatg 6600
   cagtttgaag ttttgaggag gaccttgaga aagaaaatta aggcataacc agtccacttt 6660
   gaaataagga aagaaatttt taaaattcta ttccttcttt attatttatt ttctaatcta 6720
   tatctcatat aggtgagtaa ctactgaatt actcattata ctcacacctg agacatttaa 6780
   tcaagtatgt taacactgac atctagttgt aagatgaaag atcagttgaa tacatccaat 6840
   gtcctttcat tcaaagtgct ggcttagtta ggaataaaag tactaaagaa cgtacagacc 6900
   cataaggatc tgaacttaaa attgctgcca aatgttttat cactattagt aaagtatatt 6960
   gtttggttct ttttaaaagt tcttgtattt tgctgggtga ggtggctcac acctgtaatt 7020
   ccagcacttt gggaagccaa ggagggcaca ccatttgagg ttcggagttt gagaccagcc 7080
   tggccaaaat agtgaaacct catctctact aaaaatacaa aaattagcca ggcatggtgg 7140
   cacacgcctg taatcccagc tactcaggag gctgaagcag gagaatcgct tcaaccctgg 7200
   aggcagcggg tacagtgagc tgagattgca ccattgcact ccagcctaga tgacagaatg 7260
   agactctgtc tcggaaaaaa aaaaaaaaaa aaagttcttg cattttaaaa attgctgata 7320
   aattatttcc aagaataaaa taaaaaatac attaaaaata tttttcaaag atccagtttc 7380
   cttagaaagt ttccatagtc agctaactgt ccaaacaaaa tttactttgt gtcaagacat 7440
   accttagatt attattgtta ctaacaccag actaaatata agagaccacc agccaaaata 7500
   cggataggaa ctgctggaga tctatgaaat tatcacgtcc ttgacactat atcttaggag 7560
   aatttatttt tcaacaaaat accaaaagac ataccagagc cagagaagtg gtggaagaga 7620
   gaaatatggc aaattaatgt aaaaaaaaag gagcacagtg aaggaatcta caaggaatgg 7680
   aaaataaaga tgttatgtca ggtagtataa tgcaatttgg agttttgagg aggaccttga 7740
   gaaagaaaat taaggcatga gtaatctttc cggaacgtct ctagaaagtc tatttaaagt 7800
   catgttgatg tcatgaggta gctaatacag cttctccagc ccctcaaccc acaacctcgc 7860
   accagccctg aagctttcct tggcagaaga gaagagaaaa agcaatttag ctattcactt 7920
   tcagcagggg ctacagaaag gccacatagc ccagcattac ccttagtcct gaggatgcag 7980
   ggacctcaat ttccaagggc ataaactggt ctctaaaaag aatataggta acgaacgcct 8040
   aggatacagt aaatgctcaa taattattaa aattcatatt cactgcctgg catttatctg 8100
   gaatattata gattaaatag ataaaataat aatgataatg attattttaa ctaaaattta 8160
   aaaatcaaaa ttaaaacaat cattatcatt ataattaagg ttagaaaatt agaccagtga 8220
   caaaagctta agactcatac tgttcatctg ggagatgaat tgcaggagaa atatttttga 8280
   aagagtttca agtaatgtaa gaagagatct tcacaaatga caaaaactag ctgattccaa 8340
   tctctactaa taaaaaaaaa aaaaaaaagg aaaaacagaa acgggctaaa atttaatccc 8400
   aagagactag cattgatggg aaaagctttc ctgaccttgc tatcaaaccc tagaatgaaa 8460
   gagagaaatc ggaggtattg gggaaaggat ttttttaaca cagggcagaa aaattttctt 8520
   cccaggatat ttattacttg ttttctccaa tttggaggag gatagtccca ctaagcaatc 8580
   cctaaagaca ctttctagcc ctgatactct atgagcattt aagtaagaag tataggtctt 8640
   cagatcatct tttactacct gccattattg acaaagatct tggccttttc accctaattc 8700
   actggttaga aactaaagaa ctgatacaag agaaatgctc ttttatggct atgtttgtct 8760
   attaatttgc ctttcctaga tactacgttt aaaataacta ttaaagaata aaaatagtaa 8820
   gtgaactacg acaaacaata taaattttct ctttctgccg ggtgtggtgg ctcatgcctt 8880
   taatcctagc actttgggag gctgaggcgg gtggatcacc tgaggtcagg agtttgagac 8940
   cagcctggcc aacatggcaa aaccccgtct ctactaaaaa tacaaaaatc agctaggcat 9000
   ggtggtaggc acctgtaatc ccagctactc agggcctgag gcagaagaat cgcttgaacc 9060
   cacaaggtgg aggttgcagt gagccgagat tgcgccactt cactccagcc tgggcaaaag 9120
   agcggaactc tgtctcaaaa aaaaaaaaaa aaaactctct ttccatattc ggtacagaga 9180
   aaacctgatt tgtgggaatc aacatgaagt aacattgcat taatgcttaa ttttttcctt 9240
   ctacttataa ctgtctatta ggagagaaac tagtatttcc tagacaaagc tgcaccttag 9300
   gtgaaatagt gccgtgcttt ttttcatgat aatcaatctt taattccctt gtccctcttc 9360
   ccatttcaaa ctacctagca aaatgctaaa ctgaacacta agcaaatgag tacttaagaa 9420
   acagtatgaa agatggatag caaacaacat atttttccta tttcagaact caaggaactt 9480
   cacaatttct ccttagtgct cagtctcttt gaagtaaatt ttacatcaaa ttaaattttg 9540
   ctaatgaaat tttactaata tgttttacta atgactcaaa aatatgattt ctagttatac 9600
   tatctctaca aaaattattt gcatatccca acatttattt cttcctcctc ttcaataacc 9660
   aaacatcaaa tttattcact gtggcaatgc accctgctaa aaggatatct gccacccctc 9720
   tacagctaga tgcagccatg gactaaagtc agactaatga gacagaataa gtgcatcaca 9780
   ctctcagaga gtgttctata aaggaagaga gtggtagttc cctatgctct ctggcctttc 9840
   cactggaggt ttggaaagca gatgtaatga cttgagctcc gtttgctgtg acggaccatg 9900
   aaggctgatc cgtgcactct catcacactc cagttcactc tgctgtcaca tttttcccag 9960
   gacagtggct ccctcactag actactcaat catctcagtg ctgacaccat gtctcattca 10020
   tatgggtact ctcagcaccc aggcattgca gtaattggca cagaataggc gctcaataaa 10080
   cgtctattaa ttgaatacat acaagagaat aagtgaatta tacttcctaa aatcaactcc 10140
   agattattac tgattaccct ccttgttctc ctgtttctga gaacactgct agcttaaaaa 10200
   gtatttaata tgtctgaact tatatgatta tctatctcaa tgctcatttt taagtaattg 10260
   tttgacattt aggtcaaatt ttttaacacc tacaatgaag ttcccaggaa acctacaaaa 10320
   ttctatttat gttacaaatg tcttaaatat agtcccaata gtgctattgt caggcagtaa 10380
   gtaaatatct gtgagattac ctatcatgta ctgagcactc tactgaggat acaaaatgag 10440
   tggacatttg ttttctgttt taccagcatc tggcccctcc ttttctggca atagtcccag 10500
   attttctttg ggggaaactt acctcactca tgctctgttg aagtggttca gatggcctca 10560
   aatccccttc tcctagcttc tagaagcctc ggtcaagtgc ctgagccagt aagactagcc 10620
   agaccggttt caggatgggc atgcaactca aaataagcca attaggcaca ataagaggtg 10680
   acccctggct ttttcttgtg taactattaa tagcaagagt agcttgatct ttcttaccac 10740
   gttaaacctt ggatggagat aagttcagag gctactacag gcattttgat gcaataaggg 10800
   gagaacatat ataaaaatgc acccaaccta gaagaaacaa ggctgagata catggagaaa 10860
   aaaactggtt tctggtgaca tgatttgagc ccctgcttca aactctaccc ctaactgggc 10920
   ctacatttgg acctggtcaa ttaaatgagt ccagagttac aacttttagg aaaccagtct 10980
   gagctaagtt tctgtcactt gcaaataaca cttctaagtg gcaatcaggt gaacacaaac 11040
   taatatgcca tttgttccta tgaggagaag catttcaaat tccaaactgg caaacagagc 11100
   aatatgaatg caaaagtcct gtaaataagt aattaattaa ccatacatct acaaaataga 11160
   agtattcttc ttgataaaac tgttctaaat tgaagaaaga aggtcaagag tcttcaattt 11220
   ggggagtgaa gacttcctta tggtaatacc caatcactta tttctatata tggatactaa 11280
   atacaacaaa tacagagaga gagctactta taaactatag ctcccatgtc ttttaaggag 11340
   cacagaatat tttcagttgc ccagtctcat caattcttac aaagctcaag tcctgaaagt 11400
   aaaacagtta aaatagaaat ttcattacaa gcaattcaaa aataccaaat tttaaatgat 11460
   cgataaacat ataaaagatg ccaaacttca ccaataatga cagaaatcca cattaaccag 11520
   ttttacctag cagcttggca aagattggat aaatgaacac tgtctaatgc cagcaaggat 11580
   ttaagaaaat gtgctgtcct tcatgtcatt tgtggtataa attgacataa ttcttctgat 11640
   tattagacct agttatacta catctggaaa tgttcgctaa ggaaaattgt aaacaagtat 11700
   gtaaagataa gagtgtatca ttacatacat gtatgaaaat agagatgtta aaaagataac 11760
   atccatattt actgtcacaa agcaatatcc atattattta ctacatagag cagttacaga 11820
   atatcttgag taaaaatcca agttttataa agtgtatgtg tatgtgtatg tgtgcacctt 11880
   aagacagaga aaacagccaa tatcaacaga ggctatcccc tggtggtgag atttggaggt 11940
   aattcttctt tctttaaact cttttgaata attcgatttt tttctcattg agaatatact 12000
   attattttat ttatatcttt agttactttt ttgagatgga ctcttgctct gtcacccagg 12060
   ctggagtgcc gtggcatgat ctcagctcac tgcaatctcc acctcctggg ttcaagtgat 12120
   tcttctgctt cagcttcctg agtagctgag actacaggtg tgtgccacca cgcccggcta 12180
   atttttgtat ttttagaaag agacagggtt tcaccacatt ggccaggctg gtctcgaact 12240
   cctgacctca tgatccacct gcctcggcct cccaaagtgc tgggattaca ggcatgagcc 12300
   accgcacctg gcaagaatgt actattttta taattggcaa aaagcaaaca aaaaaatgac 12360
   tcttattttg gaacgaaaga attcaatgct gctcttggtt ttgaccctga gaaacaacaa 12420
   aaggaaagta acaaggtcag acaagaaagg tttggctctt ttctcaaggc atgccaattg 12480
   tgctgtgacc tgtcacaggc acatacacac cagtaaacaa ttataactca ttataattac 12540
   aacaattgta actcagtgca ttattttacc tgctaactca acaataatca aaagaaaaac 12600
   taaaaattaa actaccttat gaaagtcttc taaagatcat caagtagcta gcatacgccc 12660
   aaaaaatgta gagctgaaag taacctattc acatgcttat agaagtcaaa agatataaac 12720
   tacttaaaaa gtcaagaaat aatagaaaca catgtgatgc agtcaattac aggtgtgaac 12780
   aacaaagact ttgattttgt tttacataca tttgttttga gtgaatgacc actcatgaat 12840
   ggttccttac tataaaatgt ttacggtcct ctttactgat gctttctcat aacttatgag 12900
   gtgatcattt ctgacatatt tatggaaaag tcaatgaaaa aatgagaagt gacataaaca 12960
   ttagtactct atatctagtt aactgaattt tttaaagact tgtaaaaata taattatttt 13020
   aatattttat aacatatagt ataatatata taataattaa ctcacaaaga tccctaaaat 13080
   tagcctaaga aaatcctctc taaatttgtc ttcccattct gagcctcctt tgctttatag 13140
   ttagccctcc ttgaaagctt ctaattttca tcattattga ctcaataaaa tttcaaatcc 13200
   tatcctgcaa aacactagca gtataatttc tgctacagat acctctaaaa ggagagttaa 13260
   gttttacaat tttgcactag gccagaaagt ataggcttgt cataagacct atgcacttca 13320
   actgcctcac tcatttcccc tttccaagtc tcctcctaac tatatcattc tctacaaatc 13380
   tttttccact aagccacaca gtattcagca ttaggcattc ttattttatt ttttatcaaa 13440
   gctatcttag ggacagtctt atggaatttt ttattttgtg tgtgtgttca tacgtcctga 13500
   tgaagattta gaatatggat atctgtttgt gtatttcttt ctctctttct ctgctagaat 13560
   tctgcattta aatcctagct tggctatttc cagttgcgtg atttaggcaa gtttctttgt 13620
   gtctttaagc ctcagtatct tcacctatta agaggataat aaagtacccg caccatagaa 13680
   ttttctgagg attaaatgag ttaataacta taataggaca tggacatctt tggggattac 13740
   tatttgatta gtgtctggca tataataaat gtttgccatt attattattg aaagtgcaaa 13800
   gactttttaa aaatgagaat atcaaatgct agaaaggacc aggtgtgatt taaaaatcgt 13860
   cagtgcggca cttctcaaaa gaagacattt atgcagccaa aagacacatg aaaaaatgct 13920
   catcatcact ggccatcaga gagatgcaaa ttaaaaccac aatgagatac catctcacac 13980
   cagttagaat ggcgatcatt aaaaagtcag gaaacaacag gtgctggaga ggatgtggag 14040
   aaataggaac acttttacac tgttggtggg actgtaaact agttcaacca ttgtgggagt 14100
   tggtgtggcg attcctcagg gatctagaac tagaaatacc atcccattac tgggcatata 14160
   cccaaaggat tataaatcat gctgccataa agacacatgc acacgtatgt ttattgcggc 14220
   actattcaca atagcaaaga cttggaacca acccaaatgt ccatcaatga tagactggat 14280
   taagaaaatg tggcacatat acaccacgga atactatgca gccataaaaa ggatgagttc 14340
   atgtcctttg tagggacatg gatgaagctg gaaaccatca atctcagcaa actattgcaa 14400
   ggacagaaaa ccaaacacca cacattctca ctcataggtg ggaattgaac aatgagaaca 14460
   cttggacaca gggtgggaac atcacacacc ggggcctgtt gtggggtgcg gggaggaagg 14520
   agggatagca ttaggagata tacctaatgt aaataacaag ttaacaggtg cagcacacca 14580
   acggcacatg tatacctatg taacaaacct gcatgttgtg cacatgtacc ctagaactta 14640
   cagtataata aataaataaa taaataaata aataaataaa taaatatggt caatgcagtt 14700
   gtgagcatca attaacagtc aatatttcaa tgaatatttg gtggtataca tttaaaaccc 14760
   aaaaaattac tttggatttt ttagttttat gttttggcac atttcttaag gtgtgatagg 14820
   caaaataata atccccaaag atatccacat cttaatccct gaaacctgtg aaaatgttac 14880
   cttacatggg aatcaggatt ttgaagatgt gattaaactt gagatgagga gattatcctg 14940
   aattatgcac gtgggcccag tataatcaca ggaaggactg aggaaggtta aaatcagaga 15000
   aggggatttg gagatggagt agaggtcaga gagaaggctt tgggatgcta cacttctggc 15060
   ttcaaagata gaaaaaggga taatgagcca cgtactatgg gcagccccta gaagccagaa 15120
   aagccaagca aatgaattct cccctagaac ctccagaagg aatgcagccc tgttgataac 15180
   ttgattttaa gacttctgac ctccagaatt ataagataat aaacctgtgt tgctttaagc 15240
   tactaagttt atgtttgtta caacagcaat aggaaactca tacatagata ataattagag 15300
   atgtaaagaa aatatttata tataggataa gtgatgctat tttatttata atagtgcaaa 15360
   tttatgaatg atctgaatgt cccagaatag atgattaatt cgaacaaata ctgtactttc 15420
   ataaagtgga gtatgataca accattaaaa gtttataaat tctacattta atggcaagcg 15480
   aaaatattca agatattcta agtaaagaaa gaaagctata atttatttta atgttcatgg 15540
   tcacattaga ataaagattt taatctaagc cttcaccagt gtttatgtct tcaccagtgt 15600
   tatgtcagtg tttatgcatt agcatcattg gtgagttttt taaattttct tctctatttg 15660
   tgtagcattg gtattgccca gtagagcaaa atctctggaa aaaggaacat gactgtctcg 15720
   ttcaatgctg tatcctcacc atacaacaca gtaagcaaca aagaaaaatc actccatagt 15780
   tatgtactga ataaatgaat attttcagtg tgtcccaatt ttttaacaaa tgtattacta 15840
   atattgctaa aaataaaaaa caagaaatgc acataataat aatttttaaa tattcaactt 15900
   cactagtaat caaaaattaa gataacaatt tcacctagta aattatatgg gttaacatat 15960
   tatattggca tatttatggt cacagagacg ttactgaata taaactagta tgtcctttta 16020
   aaaaaataat ttgacaatca tcaacaacct ttaaaatgtt catgtactat agcttattca 16080
   ttaattctac aaatatttgt tgagcatcca acatacaatg gtgcttttag atccaaggat 16140
   acagtggtga acaaaataga acaaaatgca acaaaaaaat cccaccctca aggagcttac 16200
   attctagaag aggaggcagt caattttaaa gataaacaag tacaatacaa aatatgatag 16260
   gcagtaataa gtgttaaagt gaaaaacaaa acaggccaga ggatagggag gcagggcaga 16320
   ggatacaagt tttggtactg agaacagttc actgtgcatc tttcctgacc tttttctgtg 16380
   tctaaacaca aatacattac acacataccc actgtatgtg cccaaacata agacaactag 16440
   aataaacgtt aatcccctat tttccccatg aaaatatcca aaattagatt tctggccatt 16500
   ttcattatat aaatgtttaa ggatgtagcc aaaatagtca aatgacttca tattgtataa 16560
   tttataattt aaacatactt tattttttat attcagttta attactattt ttacattaaa   16620
   attaagttct acattagtaa acttaatttt actaatgttt tacattagta aaacaaactc   16680
   tgttttttga actttttttt aactggcatg aagaattaca gctcttaggt gttaacatgg   16740
   gaagggaaaa gggtatttat cccatttaac aggtctggct agcttgtatt caataaatat   16800
   gtgcttgaag aagccatctg aggtaatctt taggatctgt tatgaattta taaagatata   16860
   attccaggta atatctttaa aaaatagaga aaggatttat cccctgacta ctgaacggga   16920
   caatgcttat gaaaaccttt taccaaccct gtcaagtgtt tattcatgct ctgcaaaata   16980
   ttttcagcaa gtatttcctg tgagtttata accaggttca tttatgaatt caagacatat   17040
   ttattgagca tgactgacgc tggcgctggc tctggctcca ggcagagatg ataaaacaac   17100
   gaatccctgc tcccacagag cttcacacaa ggagaagcct ccaacatatt ttatacagca   17160
   ctatcttagc atgttcattt ggaaatattg tatattttct actcaaagtt catgaaatga   17220
   ttttattcta ctttctcatg tgcctctttg aaatcagggt cactatcatc aacagagcca   17280
   ccttttaaat catctaacag tgttcaagga cacaccaact ttccttcagt cagagttact   17340
   tgagaactag ccctttttga gttcgtgtat aatgatgtca ctggaaattt tatgcccacc   17400
   acaaataccc agtagctatt tgtgatttcc gcgaagtaat cacctactgt attgctttat   17460
   aaagtaattt tcaaaaggct tgtttacaat aacatccaac acttgtaatt gtgaggtcat   17520
   cctcctaaaa attacttcta gatctttaaa ttcatttcta tggtttctgg ccaattccat   17580
   tgagtgacct caataagcat ctagaaatga tattaagttt cttcaggcta atgtagttta   17640
   tccaagcagt actttattga tttgctattt ttgaaaagtt tagtaattga gtaatatgaa   17700
   gagatttcct ttcagaagac taaattataa ggccatactc tctttctgaa gtataatttt   17760
   tcaggtaaaa tgaatatctc atatagaaga atttatgcca aacatgtcta taagagtgta   17820
   tgtatatata tttgtatttt gtttttgttc ttagttaaaa tactgggatc acaagcgttt   17880
   tgaatttaca gtcatgaagg aaatgcaaaa gatgatgtct tcatagcgaa aaacccttgg   17940
   attttaaata tagtgccttt gccatatatg gatatgcaaa gccataaaaa atttagaaaa   18000
   atggtagatt cacagctttt tcttcaaacc atggtaccaa cattctgaca gttcaaaatt   18060
   aatgaaaaga aatctcactg gatggagaac aagctagaga ttaagtcaca atcaacaaag   18120
   aacatcattt aatctgaata ttcaaataat atagttttaa caattatctc agcaaacaat   18180
   aacacaaaaa taaattgaga acacaagaaa aacatccatt ctgaagaagc agagaaatta   18240
   ttctggtact ttattaccat ctaaacctta gcgatctatt aaagtttagc atagttttaa   18300
   aaattctagt taatttaaaa tcatatgcta acatatgatt tcaatttaac tagaattttt   18360
   aaactacaaa tcctttgaat tataggttct ttttgaacca aaattcaact ctaatgaagg   18420
   catctgccaa agtctatggg gtcctgtttg tgctaaaaga ctctggcatt tgtttcttga   18480
   gtctcattca ttgagtacta gagggtaatt aacttcatta ttgagtttga acaaaaatca   18540
   gtaatggatg cacaagaact atttaatatt aatgttcagc tctctaatag agctgtttta   18600
   ctttacacaa aggggtttgg ctaggtttgt gttgatttaa agtctcagaa tgaaaataat   18660
   tagtggatat aagtaaaaga aaccattggt ccaggcgaag taacggtaat ttttaaaatt   18720
   ccaacataca ctttacctag tcgctaaaaa tgagatataa ttttacgctt ttagacaaga   18780
   ggagagctgg ttggcactat tcctgggtag cacttttcac caatatttct gataatgttc   18840
   catgggaaag caaaaggcaa cagagattct aaaaaggttc cagacaccaa actgtacatg   18900
   taaccttgac cttatttatc attatgataa taatcaccgt gtaattatgt gtagatttgc   18960
   tctcatacat caacttcttt tcaactttga aaaataaaga gttcatattt cttgacagga   19020
   ataaaagagc aaaaaaaaaa aaaaaccact taaacttaat tattttggcc atagctcact   19080
   gttagcatat gatttaaatt ttacagtgag taaagaccaa tttattctca aaagccctga   19140
   ttattttgac aatatttaaa atgttttaaa taaaaagaac aaaaatgttt tatcatgtat   19200
   tacagaaaaa tatttgttat ctattaaaat ctcacacaca accatgccta caaaaactct   19260
   tcatgattca gacttaatac ttaagtttaa acttatcaca gaaggatgat atctacatca   19320
   agctgcagat atttcacagc aagcatgcct atagttcatt ttttcatttg ttcattcacg   19380
   cattcaacaa gtatataaaa ggattattcc agacaaagcc tatcctatca tggagcctac   19440
   attctagagg agagagacag acaataaaca aacaagttta aattatgcct aacaataaaa   19500
   agtactatgg agaaaactaa aaaccagagc aagacagcat gggagaaggg agtggttcta   19560
   gttttatgta ggtcggttac agaatgtctc tttagcagca acctggaaga gatgagggga   19620
   tgggcctgac agatatcagg gagaagcact ttctaggaca gagagaagag acacagcaaa   19680
   ggcccagaga tgggaacatg ctggttttgt aggaagaaca accaggaggc cagggagcct   19740
   ggagtagagt gagcaagggg acagccacag gagatgtggc cccagagaga aagtgtctct   19800
   agcccaagca ggcctgggga cccattcagg actctggttt ctgagtggtc ggagaagctc   19860
   ctcaggagtt ttaatcacag taataactta tcagatatat tttttaaaga tcactctggc   19920
   cactctactg aaaacaaact tgcaatgcca gagcagaagc aagatcagta aagaggctac   19980
   tgcaataatg ctttgtccag ggtcccagaa aagtagctca ggccaagctt agctgcagat   20040
   gctttatctg gaaggaaaac tccgaagcgg caagagttag ggcagtgagg cagagaagga   20100
   gggagtgcat tagcacgtcg gtcaatgctt cccagaaacc cagccaactg ctctggcaat   20160
   gagggatttg gtagagccag gctggatggg atgaccacac ctcagagctg tcaggggagg   20220
   gctgtatctg ctgtgggaga tgatgagatg ccctgcagta gagacgctac tgggtctgaa   20280
   atagcacttc ccatcttctc actggcattt gagcaaggag caccattgcg ggaggaggga   20340
   gagagtgtat cttctggctc ctttctcatc ctgtctgtta ttggtcaaaa gtccccccaa   20400
   agcgtgactc cccacctccc tgctacctac acacacaagt caggttttgt gatctgactc   20460
   ctcaagacag ctactgagca gacagagtgc atgcccacag cctgacactt cacctgagtc 20520
   caggtacagg ggtcgacaat cattctgagg gtcaagctcg gccatcaccc cctgacagtg 20580
   tcagatgagg caaggtggca ttagtcaggg tgtgacactg aaccagcagc cccaggccag 20640
   gaggcaggtg gggccaagca aatccgggca gcgcaaaaat taagtctgac aagacaggag 20700
   agagattata gatcaggtgg taaaggtgaa aatggagaga aatgatagag ttataaatat 20760
   agtctgaagg taaagcctgt agaactggct gatgtgggtg aaaaagaaga aacaagatga 20820
   ctccaaggct caggtgtggg caactagaag aacaaagcca cctgttattg gggtgaggca 20880
   ggttacaaga gtggcaagtt ttggaaggga tcagagacca gtagttcagc tttgggcatt 20940
   ctaggagaaa acagagatag taagaatgac aatacctgcc taatagaaat attacagaga 21000
   agcttatttc aaagttcgtc caaattatga acatagttgg ctaatatgta tagccctggc 21060
   gcatcttttc tttcacaacc ccagcatctc cctcagctcc atcactgccc ttttgttgcc 21120
   catcagacat ttccggaaac acgcactaag gagcactgtt ttcacaatgt aggtgtgctc 21180
   tccttccccg caaaacaaaa gggtgggcga ggggcaggag cagcagggtc cttgcccaaa 21240
   tataaataca tatgggaagc actgttttaa gccaaacagc ttctttactt ctcaaaacct 21300
   gatacatttc acgtgtcttg agaaggtctc aaggaaagat gttgtgtgga gagtttctaa 21360
   acgtatttga acatcaaggt acactgaata aacaaaatcc atatgatcct ctcaaaagat 21420
   gtggaaaaag ctttcaataa aatccaacat ttcctcatga taaaaacccc tcaagaaagt 21480
   aggcatcaaa ggaacatacc tcaaaataat aagagcatct atgacaagcc cacagccaac 21540
   atcatactga gtgggcaaat gatggaagca ttccccttgc gaactgaaac aagacaagga 21600
   tgcccattct caccactcta ttcaacatag tattggaagt actagccaga gcaatccctc 21660
   aacagaaaga aataaaggag ccaggcacag tggctcacac ctgtaatccc agcactttgg 21720
   gaggttgggg tgggctgatc acctgaggtc aggagttcga gaccagcctg gccaacatgg 21780
   tgaaaccccg tctctactaa aaatacaaaa ttagccaggc atggtggcgc atgcctgtag 21840
   tcccagctac ccaggaggct gaggcaggag aatcgctgga acccaggagg cagaggttgc 21900
   agtgagccaa gattaagcca ctgcactcca ccctgggtga cagagtgaga agacgtcaaa 21960
   cgatctctct ttgcagatga tattatttta tacctagaat actctaaagt ctccaccaaa 22020
   agtctactgg aactaataaa tgacttcatc aaagcttcag gatactaaat taatgtgcaa 22080
   aaattagaag catttctata cacaaataat gttcaaactg agagtcaaat caagaatgtg 22140
   atcttcttta taacagccac aaaaaacata taaaaatacc taggaataca tctaaccaag 22200
   gaggtgaaag atctctgcaa ggagaatgac aaaatactgc taagagacat catacaggtg 22260
   acaaacaaat ggaaaaacat cccatgctta tggattcaaa aaatcaatgt tgttaaaatg 22320
   gtagtactgc ctgaagcaat ctacagattc agtgctattc ctgtcaaact actagcatca 22380
   ttttttcata aaattggaac aaactattct aaaactcata tggaaccata taaaagcctg 22440
   aatagccaaa gcaatcctaa gcaaaaagaa caaggccaga ggtatcacat taatggactt 22500
   caaactatac tataagacta cggtaaccaa gacagcatgg tactggtaca aaacagacat 22560
   atcagacaaa cgaaacagaa tagataaccc agaaataaag gcacacacct acagtcatct 22620
   gatcttcaac aaagtagaca aaaaaaatgg ggaaaggact cactatttaa taaacggtgc 22680
   taggatagct ggctagccat atgcaggaga atgaaactag acccctacct ttcaccatat 22740
   acaaaaatta actcaagatg gattaaagac ttgaacgtaa gacctcaaac aataagaatt 22800
   ctagaagaaa acctaggaaa caccactctg gacattggcc ttggaaaaga atttatgact 22860
   aagtcttcat aaacgattgc aacaaaaaca aaaattgaca agtgggacct aattaaacta 22920
   cagaccttcg gcacagctaa agaaactatc aacagagtaa acagacaact cacagaatgg 22980
   gagaaaatat ttgcaaacta tgcatctggg aaaggtctag tatccagaat ctaaaagaaa 23040
   cttaaacaac tgaacaagca aaaaacaaac aaccccactt aaaaactagg caaaggacat 23100
   gaacagatac ttctcaaaag aagtggccca tcaacaaaat gaaaaaaaat agtcatcatc 23160
   actaatcttc aagaaatgca aatcaaaacc acaataggac agcatctcaa accagtcaca 23220
   atggctacga ttaaaaagtc aataaaaagc taggcgtgat ggctcatgcc tgtaatccca 23280
   gcactttggg agaacgaggc aggtggattg cttgggatca ggagttccag accagcctgg 23340
   tcaacatggt gaaaccctgt ctctactaaa aatacaaaaa ttagccaagc gtggtcgtgg 23400
   gcacctgtaa tcacagctac tagggaggct caggcaggag aatagcttga acccgggagg 23460
   cagaggttgc agtgagccaa gattgcacca ctgcactcca gcctgtgcaa caaagcaaga 23520
   atccgactca aaaaaaaaaa aaagtcaaaa aaaaaaaaaa acgcagatgc tggcaaggct 23580
   gcggagaaaa gggaaccctt atgcactgtt gatgggaatg taaataaatt cagccatggt 23640
   ggaaagcagc ttggaaattt ctcaaagaac ttaaaacaaa actaccattc aacctcgcaa 23700
   gctcattact gagtatacat gcaaaagaaa gcaaaatcat tctaccaaaa agacacatat 23760
   acttgcatgt tcatcacagc actagtcaca atagcaaaga cgtgcccatc aatggcggac 23820
   tgaattaaga aaatgtggta tatatacacc attggaatac tacgcagcca taaaaaagaa 23880
   caaaatcata tcctttgtag caacatggat gcatctggag gccattatcc ttagtgaatt 23940
   aacacagaaa cagaaaacca aatacctcat gttctcacta ataagtggga gttaaacatt 24000
   ggatactcat ggacaaaaag atggcaacaa tagacaccgg ggactactag agagagaagg 24060
   ggggcagcaa aggttaaaaa actaactgtg ggtactatgc tcagtgcctg ggtggtggga 24120
   tcattgatac ctcacatctg agcatcacac aatataccta agtaacaaac ttgcacatgt 24180
   accccttgaa tctagaagct gaaaaaaaaa aaatttaccc ctaaccccaa aaaatacttt 24240
   gcagtgaagt aagcacacat tattcctcac aagaaaagat aagtaattca attgtctaat 24300
   gggaagttaa agttctcttt tactgaatac tcaaaattca tcttacgtat ttcttttttg 24360
   tatttcaagt ttattaactt ccacagctgc cagagaagaa atgataaatt tattttaata 24420
   ggacaacttt ttacgttgct tcactttaaa tatgacttgc tatataagag taactagaag 24480
   gaaagaggca tgaaaaggaa aattttataa tcccaaagac aggtatgtat aactgaaaaa 24540
   aaaattaagc ggttcatagg ccagccccta acaactgatg ctataaaaat atccttaggt 24600
   gacattcata tctacataca aatacacttc atcgtaatgt agttgcctga ttcagttaca 24660
   aacacaccca gttacaagct ggtctagtag actcccaagt tggcatgtaa gtcgttgagg 24720
   aacaaagaca aaagagattc agaaaatgaa taattaatac gtgacaaatg agatgcttag 24780
   cattttaaaa gaaaatatta aggctgagta attcttacta aacacaatga agtattatgg 24840
   ggtaaaactc aaagccaact ccttggaaga gctttagaag ctatttggca gacaggcctt 24900
   tttgctttta aggctgactc caatatttga ctatatagaa aacagtcatc cagctagagc 24960
   tttcctttat tcaccacctt aaaccacact ttactcttct cctgatacta cattttaaaa 25020
   tgagaaaaat acagaatctt atttgcatat atttgcaggt gaccttgcag gctccactga 25080
   tatataacct gtgacactac ttcttccact gctttctgaa agaagatatt ttcacttgga 25140
   agattctcat tagccaaaac tttgtagact gtgcagacat aaatatcaaa gtacacataa 25200
   gtagtttctg tataggtcaa atatgtattt aaaaatgtaa tagacattgt tatactgtgg 25260
   ccatctagaa tctttgccta tgtttcctgt gtttgaaaat ttgccacagt atggatcctt 25320
   ccttccccaa atagaagcca gaactcacct tctcagcttc ccttgtctgg ccgtggtatc 25380
   tagtgtctac caaccacctg catccacatg ataagacctc agtttagagt tagcagtgtg 25440
   aggagagact tcaattcaga ggtagcaatg gaagaactta aacaaacgag gaaacccatc 25500
   tttctggcaa gggaggtggt aagaggcatc cacctctcaa gggaaaactg cgacagcact 25560
   tctggcttcc agaacccagc actgttggtg taagctgtga tgtcgggata aagcttcact 25620
   gtggccatac ccacactata gtgtgggcag tgctcctgtt cacctaacct gtttgttggg 25680
   tgtcctggag attctgtgag tttccaaatg tttttaattt tttttttcct gattatgtta 25740
   gctagagtgg attctgtggg ttgcaactaa gaactacata taatacaaaa aaagaatctg 25800
   ggctaaataa accttcatca tttgataaaa cgatagcaga aggattatca attcaatagt 25860
   aacctcaata gtaagctaga aagagtgtgg tactgtgcta gaggctgtac aggaacagaa 25920
   aggaacagca aagaatgtta catttaagcc atgtaaatac atgggaaaat agatataaat 25980
   aaagttaaat gaaatttaga aaatacagaa gcatctatgt agtgactgta aaaatgcaac 26040
   agcatactgg atatgctgat aaggatgaaa aacttttatt gacatatttt ttaaatgttc 26100
   actgggtttg aggcaaatct atcatgttgc taaagaaatt aaatatactt tttataactg 26160
   aaaattcagt aatgtgggga aaaatatttg caaatcatat atccaataag acattaagat 26220
   ccagaatata taaagcctac aactcaataa caaaaagaaa attccaaaat gggcaaaggc 26280
   cttgaacaga cacttctctg tattgaagaa aacaaatgac taacaggcac atgaaaagac 26340
   cctcaacaac ccggcacagt ggctcacacc tgtaatccca acactttggg aggccaaggt 26400
   gggtggatca cctgaggtca ggagttcgag accagcctgg ccaacatggt gaaaccccat 26460
   ctctactaaa aatacaaaaa attagccagg catggtggca gggacttgta atcccagctg 26520
   ctcgggaggc tgaggcagga agatcgcttg aacccgggag acggaggttg cagtgaactg 26580
   agatcgcgcc actgcactcc agcctgggca agaagagcga aactccatct caaaaaaaaa 26640
   aagaaagaaa aagaaaagat gctcaacatc tttagttatt aagaaaatgc aaatcaaaac 26700
   cacaatagga tatgatttca tatccatcag aatggctatt atttttaaaa aatggaataa 26760
   caagtggaga atatggaggt agaggaaacg gagtgctcat gcaatggaaa attgtgccgc 26820
   tgctgcggaa aacaggctgg tggttcctca agagttacac agtattatca tatgatccag 26880
   caattccact gctaggtcta taccccaaaa cttaaaagta aggactcaaa caatacttat 26940
   ataccaatat ccaaagcagc actactcaca atggtcagca ggtaattgat acaactcaag 27000
   tgtctatcaa cagatgaatg gatacacaaa attgtacagc tatacaatgg aatattattc 27060
   aaccacaaaa agaactgaga tattgataca ggctacaaaa taaatgaact ttgaagacat 27120
   tattctaagt gaaataagcc agacacaaaa ggacaaatgt attattctac ttatgtgtgg 27180
   tacgcagagt agacaagttc ataaagacaa agcagaatca aggttaccag ggactggggg 27240
   gaaagggaat ttggagttat tgtttaacag gtacagagct ctgtttgaga tgattaaaaa 27300
   gttttagaaa tgaacaaggg tgatggttgc acaacattgt gaatgtactt aatgccattg 27360
   aactatacat ttaaaaatag tttaaatgat acatttcatg ttgtgtttta ccacaataaa 27420
   aaaattccat tatgaacctt atctagaaat cagtcaaaaa tgaagttcaa tattattcct 27480
   tttgtgctgc ttattatatt atttaatgta tagatccaag gaagttagta gttctaacca 27540
   gaacaacggc aacatagcat agttaaaagg tcaggtaggt agacctaggt taaaatacca 27600
   gttctaccgc taactagttt attacatact taaaaaagga cctgaattta aaagatccct 27660
   aataaacact aaagttagct gctgttatta gtctttatta ttatattaaa aaccacctgg 27720
   aaaggagcaa tgcattttat tacaggtaaa ttttaaactt tagtacatca aataagtgaa 27780
   catgggaagg caaacaagtg tggaaaaata tttgcatcaa tatgattaag agtctatatc 27840
   cctaatatgc agatttccac cgaaaagatg agagagcaga gaggtacagg gaggcaggaa 27900
   agttgctttg gatcacaggg tcagccaggg aaggccaata tatgaactaa gacatgaaat 27960
   gtgaaccgaa gctagagtga aaaagtctgg ggttagggag cactctggat gaggcaacag 28020
   caaaagcaaa ggccagaaca tcgaagaact tcgagggttt gaggagtaga gagaaagaaa 28080
   aacagtaagc ctcgacaaga aaggctgagg aaagaattca gggaatcgtt taattagaat 28140
   ttcagctttg ggtgttgacg gtgaagcggg atgctgaagg gagagtggca gataaaattg 28200
   aagagaagac agaagccaga actcacaagc ctgttaagat gggactgtga ttacaatctc 28260
   aaaagcaacc atttaagcaa cgaagtaaca tgatctggtt tactgtgcaa atgatcatgc   28320
   tgcctgctag aaggaaaacg gagtagggag gcagaatgga agtaaggaga gaagctggga   28380
   agctatgtta ataatttaaa ttacagaact taatagcttg gattcacaac agtagaactg   28440
   gcaatagaaa aacgtgtaca aattcacatt gcattggcta ataggagaga aaatatttta   28500
   ctctttcatt aatcccaaaa ctgtcactta aaattacaca atttccccta acaaattagc   28560
   aaggattaaa aattgataat acatatccct gatgaatatg tagttaaggc actcaaaaat   28620
   ctctgaaaag caatttgcta tcctctctct caaaagcctt aaaaattctg tttttcactt   28680
   agtaactagc acacagttaa gtcttactgt tgaattaatt ccaacagtta gtaaacatgc   28740
   ttaaagagaa aatcggcatt atgctcaaga atgtaactgt agcacactgt acttgcaaaa   28800
   aaactgaaga atgatctaaa tattcgacac ttaaggaatg gctgaattac agcttatgat   28860
   aaagtactaa gcaattcatt aaggagtttc aagatatggg aaaacgcgga cattatatca   28920
   ctttagagaa ttaacaaaga gtaaatagta ctagagtttt gctttgatgc tgctaagttt   28980
   taaaatgaga aataaagata gccggcaaac aagtactaaa aacttaatga tcattaacct   29040
   agtctagaat tgaaactcaa taaatctgct caaaaaactt atacgctaaa cccattaaat   29100
   attgtctgta tcttacagtt actcttgtta ctcttggtcc taaataagga agcagggaag   29160
   tttcagttcg gtggttgtct tagtaattta agaataaaca tgcaccttca aagcctgttt   29220
   gtcattctgt atccccagct cctagcacaa aatctggcac agaataggag ctcaataaat   29280
   aattttgagt gaatatcacc aaaagtaaaa ctttcagttc caactcagca tgaaagaaat   29340
   caaaaacctc aaaatcaaag acgtgcataa aagtaatagt ttgcttgata atatctcgtg   29400
   atgtcgtttt aaagtaatag tgataataat gtataatatt tggagtggtt acaatgtatc   29460
   aggcactaac aagcacttga cagactttaa tccttataat aatcattctc ttttaaaacc   29520
   aggaaacagg ttcatcactg ttcacgaagt agcccaacgt cacactgcca ccaagctgga   29580
   actcagttct ctggcatcta aacgcagaac tacagaagac acgtttcttt ccaacgatca   29640
   gacgtccaag ttcttcaagc ttcctctacc atcctattta gcattcgcaa acttctacct   29700
   tgaattgtag ctattatccc ttccaagatc catctctcac caggggagcc cctgacactc   29760
   cctccctccc cagctgggaa gccccccagg atgaaggacc gagtctccat gcttctcggt   29820
   ggctgcacac ttagaatggg ggcttggcac caggcgaggg tctcgcccct ttgataccct   29880
   ttgaggttga tttactgcct tgcgaatccc gactcccaac tgggcccttt gaggcgccat   29940
   gtgcggcccc tcgagccaga ctcggcgcca ggagaaggga gtcagcgtct ggaggggtgg   30000
   acacggcggc gcgcaggagc tgtttccagg acaaccgagg gccgccccac agtgtccgcc   30060
   ccacagtgtc cgccccttgc gtctcgccgc tccgcccctt cccggcgcgg gtgctccaac   30120
   cagcccccgt gagcgaatag agagaggggt gctagatgga ctctcacctc tcagcgcgtg   30180
   gtgcatgccg cccacgccgc tgtatagctc cagcacccgc aggggctcca tccccgcgcc   30240
   tcagccgccg cagccccgga gctaggcctg ccggtccgtc gctcctcctc cctccatcgc   30300
   gccgctccct cagcctctgg ttccgcggcg tttcagttcc cccgtctcct accgctccag   30360
   gctcagggct tcagagggcc tcgcggcctg gggattggca agggtctaga ggggaacaaa   30420
   ggacggaaaa caataaggct tcccgaatga atggcagcca ctctgtagcc tgatttacgc   30480
   tccatttcct tctttggtgt caggggcctt gattggaaaa gaaatccaga gttcagtaaa   30540
   ggggagaaaa ctgccttggt ttccaagtga attaacactg gttagaagcc agaaaaaaat   30600
   catttttcag acaagctgct tttctgtcac ttagaagcct gaacattcaa acattctttc   30660
   cacagtgtgc cttgtttcct gcctgccctg ggtcaataga gagaaattag accagcctct   30720
   cccctagaga agattccaga attataagta aggacacaag tccactcgca gcagagaaag   30780
   ccctgtgtgc gtgctgataa tacagaggat gtttgttgtg tctattagtg gggttaatta   30840
   tgttttagct tggaaattac acctacatag aatacacagt gaatatcatg taggattatt   30900
   tatgggtctt taagtgctat ggccttgatt cctcaggatg atccagtaac caactgttag   30960
   aggctcatgt tgacgaaaaa agccaaactc cgtaaaatgt ttgaagaggt ttattctgag   31020
   cacaaggtga ggaccgtgac ccatgacaca accccacagg gtcctgacaa catgtaccca   31080
   aggtggttgg gctacagctt gggattatat ggttttaggg agacataagg acattaactg   31140
   gtatatggaa ggtgtactta gtttggtcca taaagatggg acaactcaaa aaggggaagg   31200
   attccaggtt acaggtggat tcaaagattt tctgattggc agttggttga aagagttaag   31260
   ttgtgaccta aagacctgga atcaatagaa aggaatgtgt ctgggttaag ataaggtgga   31320
   gaccaaggtt cttattatgt agacgaagtc tcatacgtaa caggcttcag agagaataat   31380
   tggcaaatgt ctcttatcat gtgtaaaaag atgccagact cttagttaaa tatctcttgg   31440
   atcaggaaaa gacctggaaa gggaaaggga gtctctatag aatgtagatt ttccccacaa   31500
   gagatggctg tgtagggcca tttcaaaaag tgtcaaagaa atttatttta gggtaaaatg   31560
   ctttgattta gggcctgcta tctttcatgt gatgctatac tagagtcatg ttggaatttt   31620
   ggtatcttat tgctaagaag agtctgttat gtcggtatga agatctcttg ttttaataat   31680
   aatactggtc agttgtgcct gaactccaaa gagaggaaag tataatgagg catgtctgac   31740
   ttccctttcc cagcatggcc tgaactagtt tttcaagttt ctttggaatc cgcttggtgg   31800
   aggtgttcaa tcagtcagtt ggggggagct tagaaatttt gtggggggag cggtgtttac   31860
   agtcaattat aaataaaaat aatgtaccca tatggaggat agaaaatgtt cccgttgtga   31920
   agtgtttttc tctttgttcc caacactgcc acaggttttt gttaggggga acttgggggt   31980
   accacttgta ataacaataa ctgatttgtt agaaatgttg catgcctgct aatgtaatgc   32040
   tgttaacaaa taaaagtata accactgcat ccaaattata acattcactg gttccttttg   32100
   atgtggtcta tgaaattaac tttttaaatt gtacatgtgc attggccacg tgctttgctt   32160
   tttgttagtc actttcagat aagaagaggt gtatggtaga aagataccag ataactcagc 32220
   tttagccctg gcgttgccag ttaactgatt gtgtgacttt gagatgaccc tggtttcctc 32280
   atctgtaaga taaagcagaa gggaacagaa agagatggat tgttgagggt gttttagttc 32340
   caagaaatga attcatggct gaattcaaat tagaaaaaat gagacctgat ttggcaacgt 32400
   gttaattatt tattgaattt gaaatatgta gaaagaagac ttgttacctg ctttaagcaa 32460
   atgtactctt aaactaactc agcttacaga aaccatgtac acactgacat atgtataatg 32520
   catgaagcaa actttttaaa tatttattac atatagtacc agaaataaac atcagtacat 32580
   aaaatgtaaa gggaactata tagtcacagt gatttatata tagccaaaaa ccaatatatt 32640
   aaacaaatct ggaattttca gcattcgtgc tataaatcaa aataaatgtg acaaaagacc 32700
   ttacctgtag gggagagaca gtacagacac atggcagagt taatgaaaaa taccaacctc 32760
   ctgagaggaa ggagcctcaa accccttccc cacacaggct gattacaaac tgtttactct 32820
   gatacttaag gatcttcacg gtatagccct gaacttactt ttcttcctta acttttttct 32880
   cttcttgaac tctgctgtaa atgaaactac ttggtgttcc ccaaacatgc ccttcagttc 32940
   acttctctgc ttttgttctt tcccttctct agaaatctcc tagacttctt taagacctat 33000
   acttcaagtg gaaccttcct tctagactcc cagcctaaaa tatcctgaca cattgcaact 33060
   cctaggtctc cctcatcttc tgaaatacag atcacccttg tacttgtcat tcaatccttc 33120
   cagttattct agcccaattg tcacctcata tgtgccacat ctttatgtat tatttcctct 33180
   ctgaggctta tctcgtgata attatttcaa gaatgaaaac tctctccata atcataattt 33240
   acaactatct ccatggaagc atctgtagat attaggcccc ctgggattat gtatgcaaat 33300
   gtcttccaat cactggcaca aattacatga tttggtggaa gagtgggagg gctggtggag 33360
   gtggtacaat agggagtagc tgaggagaaa agtgaggtga gcagaatgaa agtaaatatt 33420
   gattactttt tccgaaagca aatcccgaag gctcataaga gagaagaaaa actctctagt 33480
   cccataattt cccataaagt catcctcaat gatatgtaca atactattta tattttaatt 33540
   agagaaacta gacatacttt taaaaaagca agcaaatatc aaaactggaa tgttagtata 33600
   tttactacat tttctctaaa agcagaaaat gcgtttcttt aaaaaaaaaa cacattaatt 33660
   aacacctgga aaataaaggc acatataaag catatcagaa ataattaagg ggcacatcac 33720
   aatactaagg accatagtaa aagagtaata gcacacactc aattattgaa ggcactgggc 33780
   ttggaatcta atgacatttt tatcttctac ccttatgtgt tctttcactg atcattaata 33840
   tctaattaaa tgaaataaag ttttccaagt gctgaaaatg ctttttcaat catctttgaa 33900
   tttccgtgat ccaggccact ctctttctca gcaaaacatg atgtggattc ctatacaggc 33960
   tcgcttacag tctgaggaca ccctgcttat gccaatttca gtcatgaata agttgcattc 34020
   tggctttata ccaaaaaaga aggtctttat gaattgaatg gaaaaagaaa aagtcatatg 34080
   agaagaaact gactataaga aatgtcctca agagagtaaa ttttacaaat ttgaattgga 34140
   aagaccaaca gagctcattt accacttctg agctcccact tatttgagaa tgccttctac 34200
   tattttcctg atccatgggc aataagcctc agcttgcaaa ctgctaatga aatgtttcat 34260
   ggtgaacagc tggaattatt agaaaattct tccttatagt ctcctaaatt ccagtttctt 34320
   ttagatttgc cctctgcact gattttaaag aattattcct cttctggctg ggcactgtgg 34380
   ctcatgcctg taatcccagc actttgggag gcagaggcgg gtggattacc tgaggtcagg 34440
   agttcaagac tagcttggcc aacatggcga aacccatctc tactacaaat acaaaaatta 34500
   gtcggttggg ggctgggtga ggtggctcac acctgtaatc ccagcatttg ggaggcagag 34560
   gcgggtggat cacctgatgt caggagttcg agaccagcct gaccaacatg gtgaaacccc 34620
   gtctctacta aaaatacaaa aattagtggc tcatggtggt gcatgcctgt aatccctgct 34680
   cctcgggagg ctgaggcagg agaatcactg aacctgggag gcagaggttg cagtgagcca 34740
   aaattgtgcc attgcactcc agcctggtcg acaagagtga aactccatct caaaaaaata 34800
   tatatatatt ttttctcttc tataatgatt tgctttgtgg ttaagaatgc aagcttcaga 34860
   gccacagtgt agacaggaag cacacgtctg ctgctgcttc tccctagcta ggtgacgtgg 34920
   agaaagtcac tacatccttt tttgagccaa agtgcacctc taaaatgtat aaataatagg 34980
   acatgcagtt gctatgaaga ttaaaagccc ttagcccggt gcctagcaca atacaaataa 35040
   atgtatgtta aataaagagt gcctgtgcaa tgacatcctc taagtgatgc attctcagtc 35100
   ctgtcggcta tgaactatca cttggcatga ttcagatccc tcgcattagt cacagtccag 35160
   cttggtgata cccattttag gagccaacac tgcatgaaac aaactgaaca tagatcagca 35220
   tatggtagag cgaaccagat ggctacagac attcaagggc tctagggcag ctttaggccc 35280
   tgtaggaagt gttcagggtt cctcggctta gaccatcgtg gagtaaagac tgaaagtctg 35340
   gaagctacct cagccctgac cgagtaggtg ctgagcatgg aaataaagaa gtcagacaat 35400
   agacagcaga attcatagta aaatagatac cgcttaactc ttaacttcca gaagaaccag 35460
   gattgctgtg aaggccagag ctacagctct gacaatcttc ggggaaaatt ttttcagtga   35520
   ccttaaccag gatgcatgaa ctggggagat ctggggtgca gaggagggga acctatctta   35580
   tagcagctag tcatgtgttg actctttcat acgctaggca ctgaggagat ttgcaattaa 35640
   aaaaatttaa aaagcagccg gagtggtgga tcttgcctgt aatcccagca ctttgggagg 35700
   cctggatgac agagcgagac tctgtctcaa aacacaaaca agcaagcaag caaacaaaca 35760
   aaaaacttac aaagtgcaaa actttataat ctatttcagg ccataaaagt gccaatagta 35820
   aaaaatgcaa ttgaatactt tcatttccta agcatagggg attttttcta atttaattga 35880
   ttctcactat ccaataggat aaccagtagc cacatttggt aaattaagtt taaattaatt 35940
   taattaacat taaaattgga ttaattatac taggcatata ccaagcgctc cgtattgctc 36000
   agcacagatg tagaatattt ccatcatcac agaaagttct atcgggcaga gctgctatgc 36060
   agtgatggtc ccttttagtt acagccttta ttaatagccc atcaataaat gaccagtgtt 36120
   ggaatgtgtg catcaattat tgcatactga gcaccaattt taaataaatg tcccccaaaa 36180
   tatggggcaa aatgaactca aattaaagca aaccttacag ctaaacattc tttctctttc 36240
   tctatcatcc agtcacctta ctgataaact gtcatggagg aaaagaaaca ggaacagcag 36300
   atgtgttctg tcagtggatg tgataaactt tcaagagaaa aagaacagat ggaaaatgaa 36360
   cttcatgatg tctttaagga gtagtacttt tttttttttt tttttttgag acagagtctc 36420
   actctgtcac ccaggctggg gtgcaatggt gcaatttcag ctcactgcaa gctccacctt 36480
   ccaggttcaa gtgattctcc tgcctcagcc tcccaagtag ctgggattac aagtgtgcac 36540
   caccacaccc agcttttata tttttagcag agacggggtt tcgccacgtt ggccaggctg 36600
   gtctcgaact cctgacctca agtgatccac tcaccatggc cccccaaaag cctgggttac 36660
   aggcatgagc caccgtgccc agccagaagt agtacttttc tgttataaag tatattctag 36720
   tgacttctga ctgtgaataa tgttatctta cacttatctc agaaggaata aaaagaaaac 36780
   atgtttttaa aaattaggat catgaatcat gaataaagtt aattatacat tttctgccaa 36840
   ataaaataga aaccatgccg tttgcctcat actttaccat ttcaaactac tcatagattt 36900
   ttaacatgga ttttgctctt ggaggctcat tctgaaattc tcaatttaaa aatattttgc 36960
   agcaaattta aaagatgatg tttcaaagta aatatagtgg gatccattct ttaaaactgc 37020
   tttcttaact tcacagaaaa ttagaactag aaagtatctt aaggactatt tgatacactg 37080
   ttttttaaac tatttttaga gataaagcct tttgttcaaa ccaaatctcc tctggaatgg 37140
   ggagcaaaga tagagaggta gagcaacagc tacactgtaa cttagtataa aagatactaa 37200
   gtagactcag ttggtcctgc ctgctgcccc tctacaccca ggcaaggcca gggatctaaa 37260
   ccaaagcaat cataggagcc acaatatgtc acttcctgag agcaggctga cctgaggact 37320
   gaggggaact tcagagatct atgaagcaaa cttgatctga tatggtaatg catgctataa 37380
   aagccagaca aaaatgttaa catagctgca caaaatagag actatctcag aaactaatgt 37440
   ttaacattga taacagatcc cctgcccaat cactcctgca aagacagctt gatacaggac 37500
   aaagaatatg gtccttagaa ctagatggat taccatggat cctgcctcaa atactccctt 37560
   tcttcacata ttaaacggga aatatttgat aacaaggtaa ctgtgtgtgt atatatatat 37620
   catgtgtaaa taattgtaca catatcaatt atatatgtgt acatatcaat atatacatat 37680
   atcatttata tgtgtacata cctatatata caatgtgtgt atatagacaa tgtatatatg 37740
   tacatttcaa tataaacaca atgtatataa gtgtgtatat atgtatatat atcagtatat 37800
   acatatgtac acacacacac tgatagatat atatcaataa tgtgcctaga acaataaata 37860
   ctggtttcct ttcactttct gaacaacttt gtcataatat agaacccatt tcttctaaca 37920
   atagggagaa ttattttcct agtatgtttt cataaagggt atactaccaa gagattaatt 37980
   tgcatattgc ctaacaagat gtctttctgt attgttttct attatagcct tcaatctttt 38040
   caggtggaaa aacacgaccc atgttgagaa tataggagac agaatttagt accttggaga 38100
   gctgttagta ctttgctcct tttctcttgt aatttatctg aacatttaga actgctcaag 38160
   gtaaccaaag aacacaaatc agacctactc acaccaggaa agggaaaaca cacatacaca 38220
   tacacacata cacaggcaca cactccttca tagtcttttt tctgcattgc tacatcatca 38280
   gaactgataa actaaaaccc agaaaaaatt caaagacaaa gacttttaat tttaccctac 38340
   ttaggtacta gtttaggtag acaacagtat cacttagaat tgttctcata tgatccaata 38400
   atgacaaata taactaaaat gtaataacac tttcatagta ctgccatgac tgtatctatt 38460
   cactctgtaa gaaaaaaact tcctattatt taaaattatt aaaacgtttc ttgtcagttt 38520
   caagtcactt acgtgagcat attacaacat acaagcacat ttcatttttc aaactttctc 38580
   ctctagcctc atgctctaga ggctgtcaac ctttacttta cagagttaaa cttattcctg 38640
   cagatcaaga ctaaaatgtt ttatttccat ctaaggtaat tactgctaag ttaaaatgta 38700
   taaatcctcc aatgggattg gtactatgtt tagtcactca gagaatactg gctgatgaaa 38760
   atagaccaca ttccaaaaga cagaaaaagt atgtttgact aataggaaaa taataataac 38820
   acaataatga cttgtatgtt tcaaataact ttataaatgt ttaaaatgct tattaaccca 38880
   ttttagacct atcggctttt ctttcttaat attcactttt ttacaaataa aaagtagaaa 38940
   acttcatgtc atttgctcaa agtcacataa gttaacattc tgcaggttaa cagatttatt 39000
   tgcaaatcac acaaacatat ttttaaaaga agggaattta gacgataaca ctagtttttt 39060
   gaggaaaaaa taggctggaa ctcatcaaat aaaaatgaaa gggagttagt atatatttga 39120
   ggaaggattt catattagga gttagacagg attctggtta ggaaagaatg tcaggaatct 39180
   gcatgtcttt tttttttttt ctcctttcat tttaggacca aatcctagaa tagagtgaaa 39240
   aaagaatgca acaaacagca ttatttatat atctttgcac agttttaaaa tcaaaaagca 39300
   gttttatcgt gttacttgtt ccctcgagag atgaggcatt attatttgga aaataaattt 39360
   ttgattatca cctaaagtga aagggactta ataaacaaac attggaaaag cccaaagtac 39420
   tgaaagctca atataactgc aagagtctct cttctgacca ttttttctct tttttgctaa 39480
   tagtaaaata ggcatgcagt tgtgcgacct cagctcactg caacctccac ctcctggatt 39540
   caagtgattc tcctgcctca gcctcctgag taggctggga ctataggcat gcaccaccac 39600
   acctggctaa tttttgtatt tttggtagag acgggtttca tcatgttggc cagactggcc 39660
   ttaaactcgt gaccttaact gttctgcctt tcttggcctc ccaaaatgct gggattacag 39720
   gtgtcagcca ccgtgcctga ccatagctaa cattttgagg tttctccccc atagtctttg 39780
   aactaattgt attttaccca aatcagaatc ttattgcata tgttttatac ctagctcttt 39840
   ttcacctaat atgttatcat tatcagttct gtacttcatt aaacacatca aagatatata 39900
   ccctttttaa atgctgactt tatttttctg aattttaaaa ataatacatg ctaatttcca 39960
   ggggtaaaac aaattacaaa agtatacaaa aattaaaggc acttccttct gttaaccttg   40020
   attccatcct cccccttaaa aaaaatttta agtttgttga atgtctttta ataaagatgt   40080
   ttatttccaa aactatgatg aggcagtcgc agacattttt gacatgagct cagagttgta   40140
   cgcatatgtt tatgtggagt tttcatattt aaaaatattt atgcctgaag tgcaaaaaat   40200
   aataatttaa agaaaataca taatacattt tgtttaattt gtcttaagac tgatgtattt   40260
   tatatacatg ggaatctgga atcatcttac aactgagaaa agttagcagt taacttcctc   40320
   tgaacgttac attttaaaca cattttagct cccaaaacaa tatgtcaggc ctcagatacg   40380
   cactctttca aaattctgcc tatgaggaaa atactaaata tgaacatttc aaaaataatt   40440
   cacctttaaa gctctatttt gtgattagac tgagtacctt gaaagtcatt attacaaccc   40500
   ttgcgtgtgt aaagcatctt ctcctcagca gcctcacctc ctacacgatg atggtctgta   40560
   aagtcagggc atccacaaag tgatttattc ccccttttac ctttaatatg gcgctataga   40620
   ccacattttg gccattgcag tgcagtagct caggtctaca atcccagtgt tttgggaaac   40680
   cagaacagca ggatcccttg aggccagaag ttaaagacca gcctttaaac aaaacaaaac   40740
   aaaacaaaac aaaacaaaaa aatttcatta gccaggtgtg ggggtgtgtg cctgtagtcc   40800
   caggtactca ggaggctgag gcaggaggat cacttgagcc aaggagtttg aggctgcagt   40860
   gagctatcat tgtaccactg cactccagcc tgggtgacta gtgagacctg tctctcaaaa   40920
   agaaaaagga aaaaaaaaat gagtatattt tgcctaaggt acccaaacac tagaaacata   40980
   ttccaaagtg aaatatagca gtataatgca agttttccaa aggaaggggg gcagaaatag   41040
   ttacataatt cttacaacca tatacttatg ttgaaggtat aaaagagttg aagattcttg   41100
   aagacaaaga gaaggtcttt ggagaaagtt atcagcatgc tatagaggct gtctgcttct   41160
   cacctcaagc ctgagacaag gagattcaga gaaccaccga gagagtttaa aatgtaacca   41220
   tctaaccttt gagtttgggg aggcaaagta attcccttgg atagatctga gccatacaag   41280
   acagaaagct gtcttaagcc ctgttcaagg ggtcactcac aagaaaaaaa ggtgtcatgc   41340
   aaaaaggaac aaggggtata ccactgaatg actcctaggg gctgtggcag cagtcacaga   41400
   gaagcaggac acaagggcat gtctgcatgc cagggaagct ttgagaaaga gaaagagagg   41460
   gagagacaga gagagagaga gagagagaga gaaagagaga gagagagaag aaaaccccaa   41520
   agaagcacct gctagaaaaa gggccagctt tgagcatatg ccaaaccttg aggtgtaaac   41580
   accagatcac caatagccct tgtcaagtaa gagttttctt atctcctgca cctctttccc   41640
   tctgtgagct ctaatcctgc agtggcaaga aaccttagtt agcaaaatga gggaagagga   41700
   agggaggcac ctaagtaggg agagtgagaa acccaggact tccctttcct aaggcacagt   41760
   caagccatgg gagggaggat attaactctt gggcaagatt gaaattttga aagttacata   41820
   catgtaagcg ttgtaaatta ctatgatcac ttttctcctt agaagtgatc aaagaatggt   41880
   ttggttttaa taaatgactg gaaaagtcac aggagctgca caaattttca tttgtaaaat   41940
   gatcgtttat caactgctct cttgtcttct tattccttcc tgcccttggt ttttggacac   42000
   aggttctttt ttctcctctt tatgccatag tttaataata tcatcaccaa ccatcaacac   42060
   ttattgagga tttactctag gccagacaat cgattatgta ttttatatac atttctcatt   42120
   tcatgcacat acaactgtta ttatcacctc cattttttat atcaagaaaa tgaggctctt   42180
   ccaaggtagc ataattacta agcataggag ctgagaaatt taccttgggg actctgactc   42240
   cagagtccca ctttttttcc ctgaaagaat gaatgaaatg tattttctca gttcaaagga   42300
   cacaaagtat caaatttgcc ttttttttta aacaaactct gtcttccaga ccggagtgca   42360
   gtggcatgat catagcttac tgctcactgc agcctagtcc tccagggctt aagtgatcct   42420
   cccacctcag cctcctgagt gactggtact acaggcatgc aacaccatgc ccagctaatt   42480
   tttaaaaatt tttcatagag ataggttttt gctatgttgc ccaggctggt cttgaattcc   42540
   tgagctcagg tgatcctccc acttcagcat cttgaagtgc taaaattata ggcatgagcc   42600
   accagacttg ctttctttaa taaaacagag ctctcaaaaa aatggatctc ccccatctca   42660
   tgagaaaatt gaaacattcc actgaaggag aattaacaga aaagtaaaca tagctacttt   42720
   ttgtaatcag aaaacttgct gatgtatact cttattccaa acccctggat gatgtcatta   42780
   tgtcaacagt cctttaggat tcctcaaccc agacataaaa taacagtcag agcactagca   42840
   tatccaaaaa tggtattgct ttatttttta aaaatatatt tcttttttga aatatcaatc   42900
   gaatcaattt tattctcaca acaatcctga gtaaatattt ttaaaaaata tattttttga   42960
   ggaaacagat taaaagcttt aaaaaatgtg attcactgaa aatcaaatag tgaatacatg   43020
   atagagtcag cactaaaaag gcagatatat caaagagaga ctgaaataag caactcattt   43080
   tttgcatcga cttcgaaaca cctctttacc atcagtgttt gtaataatca ttcttgatct   43140
   ttatacttca catttcttgg taatccaaat gcttcataaa ttctcttaca catcctgaat   43200
   ctcttgcttc attcagcgag ataattcctg gtgagcagga atgcatatgg ttttcagatg   43260
   tagaaactgg gatctccaga gacaaacaag ttggccatgg caatgggata caataatctt   43320
   ataggatttt acaaacctga tttgaagttt tgtcctaata tggaaaggta tgccttttct   43380
   cactagagag attattctag aattttagag ccataccaac atatttcacg gtaacctagt   43440
   gatctggatt gcccctctct gagaaagcaa agattctgca agcaattagt ccttcaaatc   43500
   aaattcctgg cttttacaaa ctgcttctct tttgaattcc aaaagttaac taattctttt   43560
   ctatctattc ttcatttcct cataagcgaa cactcaccag tttccccatt ttccaaccct   43620
   atggttttct tattctacgt ggccacagac aggaacaatt cactagcaca gtggttctca   43680
   gaatcacctg gtaagtcttg taaaaacaca aattgctggg ccccacgtac tcccagagtt   43740
   tcacattcag ttggtctaaa gtggggccta acagttggaa tttctaacaa attatccggt   43800
   gatgctgatg ctacaggtct gagtagcaca tttggaaaat cacttcacta gtacaccccc   43860
   gacgtgttgt cctgatggat ccttccaaaa aattgttcag atgccaaggc ttgtgaatcg 43920
   cacacatggt actcaatgcc tgggcctcac atcgaaggct tctatattga gaagaaaaga 43980
   aatatttgtc atgactcatt gcctttttca agctgttcca gctacaaaat taaaaccaga 44040
   aaaggactca attgaaagca aaataaaaac agctcaagta acactgtcca aattttgaga 44100
   cttatggcat gatttctgga attaggacaa aaggtatact agtgattcag gtaaatcctt 44160
   ggctgaccac actggaagct ggcatctcca aggctcccga caaaccctgg aacccaagcc 44220
   aagttgctcc tgcactaccc cagaagccag ctcaggtcct tggcttgtgg cttgcttttt 44280
   ctgtcaacag taagttcttc cataaagctc tccaaacaca ttagcaacct ataactcttt 44340
   taattaacta atttttctac aacttctcag ctatttttaa cacagaaaaa aatgtcaatt 44400
   gtcaagaaat aaatgctgtt gggccatcaa gcaggtaggc tgtaaatctg aaccgagaac 44460
   ctagccaatg aatttcctta ggatttcttc cttttgtaat agcaacactc agtggagatt 44520
   ttaattccat ttttcatata tttggggaaa aaatacggca ttttatgtca tagcataaaa 44580
   ttcttcagca ttccatttta gttattaaat aatgactagt atttgaaaca gcactgttac 44640
   attaagtaaa aaaaaattgg caagaaatgt caacttagac atttaataag ataatagttc 44700
   tgcttagaaa atgaggtgtt agttctgcct ttaacgtatc aacatattgt aggaaatact 44760
   gaccttttat tctgatcacc acgagtccat taaatttcaa aatgtgggac agccaataca 44820
   taatatacta ctaagtaata aatctatgtc atccaaaggg agctttgctg tctgagtcca 44880
   aaaaaaaaga aaacagaagc ataatggtaa ggttactgaa cactcaggtc ttgaaataag 44940
   tcattctgtt atgagcatga gtcaccaaaa ctacttcctt agtgataaaa gttgcctaag 45000
   taacatcaat gcctccctat gctaagaagg atatgggcag tgtgttgggt ttcagaccca 45060
   ctagtcatag tcatttcctt tacttaattg ctaactgccc ttcctggctg tagtttgggg 45120
   cctgtgtttt aggtagtaac ccagcaacta gttattatgc tatattttat caaggagaac 45180
   aaaacagtaa attttgtatt tccatggatt tcagagtaaa agtcaatgtt atttcaccaa 45240
   ggccctctga cttaagggag ttattgtgaa actgttcaac taggtctcaa tgtggccaca 45300
   tcattatgca agactcaaag gcaaaatatt taacagagaa aagaggagca gattgggagc 45360
   cctctgagag gaagggaggg gtggtattca tttctgcgtc cttagcacct agcagggagc 45420
   ctgctcacct aaagattcaa aacgcaggcc gcgcaggcgc agtggctgat gcctgtaatc 45480
   ccagcacttt gggtaggcca aggaggggtc acttgagctc gggagcttgc gactagcctg 45540
   acaacacggc aaaaccctgt ctctacaaaa tatattttaa gaaaattatc tgggcatggt 45600
   ggcatgtgcc tgtagtccca gctgctcaag aggctgaggt aagaggattg cctgagccca 45660
   ggaggtcgag gctacagtga gctgtgatgg agtcactgta ttctagcctg ggtggtattg 45720
   caagctcctg tctcagaaaa aaaaaaaaga ctcaaaatac atccaccaaa tggggtttgc 45780
   tgcttctttt caaaataatc caatctatga agtaattgcg ccactgctga gttaggattt 45840
   catcaggctt tgctgttatt gatgttgttg ttgctgttga gatggagttt tgctcttgtt 45900
   gcccaggctg gagtgcagtg gtgtgacctc agctcactgc aacctccacc ttctggtttc 45960
   aagtgactct cctgcctcag cctcccgagt aactgggatt acaggtgcct gccaccacgc 46020
   cctgctaatt tttgtatttt tagtagagac ggggtttcac catgttggcc aggctggtct 46080
   cgaacacctg acctcatgat cctctcacct cggcctccca aagtgctgcg attacaggca 46140
   tgagccattc atcagacttg ttaacacata tatgcctttg tttttccctt atatttacca 46200
   tgggactgtg gtatttgctg ctgttcccct tgcagacatc tgaagtatat taagcatgct 46260
   ctattttaaa atctgacata caaaagcaat gagattagta ttgcatgaga aaatttaaaa 46320
   actggatagt aagcacacat gtattatcag tatcgctgtt gggtttggcc ttgagaatct 46380
   tggttgccta taattccctg agcctcctct catttccaaa aactccccca taattcttgg 46440
   attgcacaat agtactacca tgaaggacta aaatgttaat aaatctccct aaaaatcaaa 46500
   ctaatagatg gtaagtatag ggagaagttg tttttaactg atataagtat tgcattccaa 46560
   aaatgtggtt ttatttcttt tctactagca aatggggcaa agcattttta ttcacttaat 46620
   catatgcaag attcaaatgc cataattatc cattcattct ggtgggttga gtcagaatta 46680
   agaaatgaga tctccacaga aaacccctaa aactgtctat ggacttcttg atgattcatt 46740
   aagggcacca tattctctga atcaaaatgg gtcctgttgc tgcatcagaa taggaaaaaa 46800
   gtagaccaaa gagacaaaca tcatgataac aatgccaaag agatttgggg acatctccaa 46860
   tggtagcaca aaattactat ggcatataaa gtactacagc acggtgaggt ttagagttcg 46920
   gggactggag ccagaagtcc tgtgttcagg tctgccactt cttggctaga taaacttgag 46980
   taagctactt accttctctg tgcctcagtt tcttcatatg tcaaaccggg ctgtcgtgag 47040
   gattaaataa gttaacccat gggcagtaca taatgattgc tttcatatgt cataggcact 47100
   caatatatga aagcaatcat tacggttatc attagccttt ctatccaggt cagactccag 47160
   ttttagcaat catatcaaag tgtgcctggt gatgattgtt tggggttcgc tcaaggacct 47220
   agtattggtc aaggactgtg aaagctagcc tagagttagc aaaggaaaaa ccctggcctg 47280
   gtgtggtggc tcacgcccgt aattacaaaa ctttgggagg ctgaggtggg tggatcactg 47340
   gaggttagga gttcgagacc agcctggcca acatggtgaa acctcatctc tactaaaaat 47400
   acaacattag ccgggcgtag tggtgggcac ttgtaatccc agctatttgg gaggctgagg 47460
   caggagaatc gcctgaactt gggaggcaga ggttggagct gagatagtgc cactgcactc 47520
   cagcctgggc aacagagtga gactctgtct taaaaaaaga aaaagaaaaa ccctgatcaa 47580
   acacataaat atctctgcag cgtattctat cacacctgtc tgaagttcta gagatgccat 47640
   tcaagtaaat tacaatattg tatcggaaaa acttctcata atttatgtct caaacaacat 47700
   gtacttaatc tatattaagc ttgagtacca cagaattttt ttgtccatat gccacatatc 47760
   catatgagtc acattagaga acatatttct agtacttagc taattagaaa tacaaaacct 47820
   tattcagtgg tttggaaatc gtcatggtag aattacaaag agggctgcca aatcctttat 47880
   tctgctagtt aactctgaat ggtctttctt tacttatatc tcattgtaat gttaatgaat 47940
   tttcttatct tttcatcctt gcatattgtc attatgtcat tttatatttc cttccatttg 48000
   ctacatggct tcatttaggg ttactttgtg ttattttttt tctagaattt gtctttgatt 48060
   ttcatttcca acctctgcaa atcacactga tctttgtcac tatacacaaa aaccaaagct 48120
   tgagaggttt attttaaact ctgagtttat caaacatatt cacaattaaa tataatattt 48180
   aatatactga agaaaatcat ttcactggct gagtgaatgt gatatgccag agttaaagta 48240
   tctcccaatt gtccattttt gaaaatatca gtcaattcat taatcagttt tgaaatacat 48300
   aagatggagt tccattatat tttgcagatt tagaggaaag agattatgac tatatagatg 48360
   gtcaataatc cccttccaat gtcgcaggtt accataaagg caaataaaaa tttacattat 48420
   aaatttatct gttctttaaa agaaaattaa taaactgata tgcacatcaa taggagactg 48480
   gctaaggaaa atgtggtaca tatacaccat ggaatacaat gcaaccatga aaaagaacat 48540
   cagccaggca cggtggctca cgcctgtaat cccagcactt tgggaggcca aggtgggcag 48600
   atcacctgag gtcaggagtt cgagaccagc ctggccaata tgatgaaacc ccgtctctac 48660
   taaaacacaa aaattagcca ggcatggtgg catgcacctc taatcccagc tacttgggag 48720
   gctgaggcag gagaattgct tgaacctggg aggcagaggt tgcagtcagc caagatcgtg 48780
   ccactgtact ccaacctggg agacagtgag actctgtctc aaaaaaaaaa aaaaaaaaag 48840
   agatcatgtc ctttgcagga acatggatgc agctggagac catcatcctt agcaaactaa 48900
   tgcaggaaca gaaaagcaaa tactgcatgt tgtcacttat aagtgggagg taagatgaga 48960
   acacatggac acaaagaggg gaacaataga cactggggcc tacttgaggg tggagggtgg 49020
   gaggagggag aagataagaa aaaataacta ttggatacta agcttaatac ctgagtgatg 49080
   aaatcagcca tacaacaaac ccacatgaca cgaatttacc tacataacaa acctgcacat 49140
   gtacccttga acctaaaata aaagttaaaa aagaaaattg tgctattaaa caaaattatt 49200
   attaggtccc ttacctaaat atcttgagaa aaaaaattct accactgggg aactacatga 49260
   cctcctcaaa gtaggcagag taaattccca agacgccaat tgcacttggc aaattttcta 49320
   gctgtggcat aatctttttc tgtctacaaa aaaaaaaaaa aaaaaaaaaa aaaaaggaaa 49380
   aacaacatta taggccactg ggcaaatacc tgctagtttt cttatggtag tctaactcag 49440
   ccagtaacta aactgggatg atgtcattca gggctctttc aagtgacatg aggtttttat 49500
   aactggattg atgggttttt tgtttgtttt tggttgttac tgttttttga tcaagtgaac 49560
   taatactgaa aatctggctt taattctttg tccaacatca gctggaactt atttttgcac 49620
   tagatttctc ctccatttgt taattaaact cttgtgttcc ccctacatac ttaccacctt 49680
   ctctttcctc tgtcatagac tgtgaattag acattgatga agttaaaaaa tcacttatag 49740
   tctacaggga attgttagct atgttttccc cctcaaatgt tcaaagatac ctcccagaaa 49800
   gtgtctttaa ggtttttctt tcccttctga tgttttaaac actgttttag agaatcgtga 49860
   tgttcatgat tttatactag ctatgggaac aagactaaca cactcaaagc agctaaatga 49920
   caataaaaga ccatctactt tattccttta agggaagaga ttagaagtgc ttagaaatgc 49980
   tgtagaccct gggctatggt ctgatagtgg tcggtgggta ttagattctt gtaggagcgt 50040
   gaaccccact gtgaactgtg cataccaggg atccaggttg tgcgctcttt acgagaacat 50100
   aacgcctgat gatccgtcat tgtctcccat catccccaga tgggaccata tagttgcagg 50160
   aaaaccagct cagggctccc actgatttta cattatggtg agttgtataa ttatttcatt 50220
   atatattaca atgtaataat aatagaaatg tttgaatcat cctgaaacca tccccctgcc 50280
   tggtccatgg aaaaattgtc ttccatggaa tcggtccctg gtgccaaaaa ggctggagac 50340
   cactgctgca taatgtaaaa ccacactatc caattatagc tcaccttcat gagactataa 50400
   tttatataga ctagagaagg gaagatattt ccaggttaag ggacctgaat taaccgtctt 50460
   tcaactcaaa ttaactttcc aactaattat ttctgtctgg aatgcatcta ttctttcttt 50520
   tttcttcttt tttttttttt tttttttttt gagatggagc ctcattctgt cgccaggctg 50580
   gagtacaggg gcacaatctc ggctcactgc aacctccgcc tcctgggttc aagtgattct 50640
   cctgctccaa cctccctagt agctgggact acaggcgggc accaccaggc ccagctaatt 50700
   tttgtatttt tagtagagac agggtttcac gatgttaacc aggatggtct ccatctcttg 50760
   aactcatgat ccacccgcct gggtctccca aagtgctggg attacaggcg tgagccactg 50820
   cgcccagccg catctattct tttaattatc ctgttcacaa ggctcagagc ccactcgatt 50880
   tttcttatcc cctagccctt aaacatcaca attccttaaa tcatctccgc gtcaggctcc 50940
   ctttaccatc cacttgatct acctcaagca gacctcaaat cagcctcatt ctccacccct 51000
   aaaatacagc tacaagcatg tctttctcct agttgaaatt cacaatgcat tccctctgcc 51060
   caccacatta attatcaact ccttttcctg gcatttactc atccaacgca tggccccacg 51120
   ttaactttca gttccctttc tcccctacaa atactccata atccagcaac cctgggatta 51180
   ttcctgttcc ctaaatgtcc tcagcactct gccacccacc cagtttttgc ttatactgtt 51240
   tggtcagcac tagagcaacc tttctttgcc atgataagct gtagaaattg tacttattct 51300
   tcaaatacta tcttttctaa tgacattttc tgttctcccc caagtagata ggatcatttt 51360
   ttttctcaac aaaatttaac ttatttttag gtatagtatt atagtttttt tttttaattt 51420
   agatttaggg ggtacatgtg caagtttgtt acataggtat gttgtgtgat actgaggttt 51480
   gggcttctaa tgatatcgtt gcccaaatag tgaccatagt actcgatagg tggtctttca 51540
   atccttgcct cctccctccc ttcccacttt gggaatccct agtgtttact gtttccatct 51600
   ttgtgtctgt gtatactcaa tgtttagctc ccacttagaa gtgagaacat gcagtatttc 51660
   gttttctgtt tctgcattaa tttgcttagg tttatggttt ctacttgtat ccgtgttgct 51720
   acaaaggaca tgatttcatt ctttttaatg cttatgtaat attctacagt gtatatgtac 51780
   catgttttct ttatccaatc cactgttaat gggcacctga gttgattcca tgtctttgtt 51840
   attgtgaata gcgctgcaat gattatttct ccacctatgc tctttagcat tttatcctat 51900
   ctgtgctaca gcactcactt accacattct gctatgtatt ccaactcttt acctgctttc 51960
   taatccttat acaaagctat gagatcccta gaggcacaaa aagcacctta cttactgaaa 52020
   tatactaaga aacattgcag gcagtgcaaa aaaatatgaa tacatctatc tgttaaatgg 52080
   aatatgagga aagacctgag ggtgggggaa atgtactata ttcatggaag aagaaggagc 52140
   ttagggttct actgaaattg ctgcttgtgg ggaacagtgg aaaatgagga tagatatata 52200
   tacttgacat aacttccaaa ggccactagt taactatgat cctcaacctc ctctgcagga 52260
   aaatttctgt ttccctcata gtctttatca ccagttgcaa tcattttgtt tgttccttta 52320
   cgagtattgt ctctctgtat catatacata tacgcagact caacactatg ttccaagagg 52380
   ctttgggcca tacctgcttt ggtcccttcc ccagtgtcta ccagactgcc cggcacatgg 52440
   caaatactca ataagtatgt ttggagtaaa agaataaaag ttgtgtatca cgataccttg 52500
   tgaagattaa atgcagtcat gtctaaagtg actagcacaa tgcttaaaca taataagcac 52560
   cagtaaatat tagccccaca cctatatttc ttccttctat gaactactca tgtattaatg 52620
   tatatattcg acagatgtgc ctggcttttc tctagccact ggaaatacaa tagtcaacaa 52680
   aagagagaaa aattcctacc tgcccttagg gagattacat tccagtgagg aaatgagcaa 52740
   ttcctgccaa gaagtttaaa gaaggaagtt tttggccaaa tgtggaggct cacacattta 52800
   atcccaacat tttgggaggc cagtgctgga ggattgcttg agcccaggag ttcaagacca 52860
   gcttgggcaa catagcaaga ccctggctct acaaaaaata aaaagttagc tgggcatgga 52920
   tggcactccc agcttctagg gaggctgagg cagtaggaat acttgagctc aagagttaag 52980
   aggctgcagt gagctatgac tgcgccaccg cactccagcc taggcaacag agcaagcctt 53040
   tgtctctctc tctctctctc tctctctcta tatatatata tataagtttt aaagaaaaag 53100
   gggtgatggc ttgaagatag atgcattggt ggcagaaaag aaagcatgga ggggccgggc 53160
   gcagcggctc acacctgtaa tcccagcact tcgggaggcc aaggcgggtg gatcacgagg 53220
   tcaggagatt gagaccatcc tggctaacac ggtgaaaccc cgtctctact aaaaaaatac 53280
   aaaaacttag ccgggcttgg tggcgggcgc ctttggtccc agctactggg aaggctgagg 53340
   caggagaatg gcgtgaacct gggaagcgga gcttgcagtg agccgagatc gcgccactgc 53400
   actgcagccc cgggggacag agcgagaccc cgtctcaaaa aaaaaaaaaa aaagaaaaga 53460
   aagaaagcaa gaaagcatgg aggaacggaa gccaattgca gtattgcagg aaggaggtat 53520
   gaaatggctt ggatctgagt gacagcaacg taacagagga gtggatccag gaagagcggg 53580
   gcatgatacc cattggatct gagatggaag agttagttag gaagtggcaa tgatgaaagt 53640
   ttccaatctt gttgttttgc tgtattgccc tgaatagaca gggaagctgg caggggaagc 53700
   gatggtggtt catgggcttt agggcgtgag acgagctaaa cccagtcttg aatgtgattc 53760
   ctcctaagag gcaaaatgga atctaaatga aaatttttaa ctcactaatg aaagtaatgt 53820
   tttagaatgc aggtgaaaac aaatctccct tcttacatag tatgatcact gttacgtcat 53880
   ttgattttgt tttttacatc attttacatc gtaaacatgg tgttatagct gtctgccaaa 53940
   ttcctctaaa aatatgcccc ttcgcagcaa gatctgtgtt aatcatccca tctcttcagt 54000
   tcctggaaca cagtaggtcc ctaaatgata agtccagcac ttaatatttg tgaattgaac 54060
   tggttgatag taagcaaaat tttgttatca aggcaaacag aaaagagaag tgcatttaga 54120
   gtcagaaagg cctcgattca aattcttgtc cccttcttcc cacccctgct agtcatacaa 54180
   ccacaagaaa attgctagcc tgcatcttag gctcctcatt agtgaaatgg atgatcaagt 54240
   ctatcttgca ggagggtgtg agtgcatcaa gttaaattaa atatatacac agtgtctgtc 54300
   attcagttgg catcgacatt acctattata ctatttatta ttattacctg tatttccatg 54360
   gattttaggt tttgctatgg ttttcatgct ctcagtgtac cctccttctt ctttctaaca 54420
   ttccatccaa acagtcctgc tagatgctgt tctcttggca tggctagcca gctttcccaa 54480
   agcctccata ggccactaag cttctgtcca cacggctggc tggtccgtca gtttaagtca 54540
   cccaattcaa ctgacatcat gacagcttgc aaattccaaa atcactgttg gtaatttaga 54600
   tgtggtagtg tttatgcctg gcctatgaat tataccacca cgtctttatt tatatgcaac 54660
   ttgcatcatg ctgtgttgaa agcagcttcc cagtccagca cttgaaaata cttctcagat 54720
   aggttttcat agcaattaaa aaataaacac aaaacccggg aaagctatgt ctgtgtaccc 54780
   aaaagatacc taagaacaca gtagcataga gagcaggctg agttgtctca ctcaaggatg 54840
   tgagttcaga ttcattgatc tccctgactg aaaactatca cccattgatt gaggcactgt 54900
   atatcttatc tcagaatgaa atgaattctt aagtctcatg aattctacat gaatgaatga 54960
   aagtgtttct gcaactttca cttctcttgc agcccaccaa aaacaatggc attttctaat 55020
   aggaaaaaca cattgaacgc tgtatatgtg cgtatgtata tgtgtgtgta gtacatacat 55080
   atactttatt ttgagacagg ttctcactct gttgcacagg gtggagtgca gtggtgtgca 55140
   cagcctactg cagcctcaat ttcccaggct caagcaatcc ccctgcctta gtctcccaag 55200
   tagctgggac cacaggtgtg caccatcacg cctggctttt tttggtttca tatatatatg 55260
   ttgcctaagc tggtctcaaa ctcctgatct caagcaatcc tcctgctttg gcctcccaaa 55320
   gtgctgggac tacagaacac ctacactttt atacattcta tatgcatcaa acaaacatat 55380
   gtctgtgaca agagagcagc ctatcacagc ccagagactt cagaatgggg acaggttgga 55440
   aggaggagct acctatccct gagatgatga agaggaccag tgcccattcc aggagacatc 55500
   accgcagccc tgaggaatcg gctatgggca ccagcagggc acagtgccac acctcgccaa 55560
   tgccttgtcc tccttttcca tagtgagtca gtcagcaagc gtcgcattgc cctgggatcg 55620
   gcactgcacg tagaagtgag ttccacactc tcttcctccc atagggagat cacttttctc 55680
   attctaaggg ttccaggcac actcacaatg gtggcatttg ctgagcagtg gcttgaataa 55740
   agggctctca gaaagcaaga tgtaactcag agcataggct tgtcctccag ggcacccagc 55800
   cccaggaatg ctcttgggga atgacctgca gcctcccagt gaaagagaga ataaaagaaa 55860
   gccccagcag gcgagctggg cagtagagag tcctgtaatt ccaccttggc aagcaccatt 55920
   tgcaagaacg aactgggata aggtaaacaa aatattgcct aaaagaggct tgtccaaaga 55980
   agtcagaata cgctcttcat ttacctctaa attatgtttc ttaaatacca agtaacctgc 56040
   agtaccccct gccgccacca aaaaaaaaga gagaaatact tttttttctc ctatccactg 56100
   cagaatcaat tttacagtgg ttctgcagtt aatcctttca gtacaccata aatctaaata 56160
   ctcaaaaaaa cctgtgcctt ttcaattgct actaaatcac gagaagactg atttacatag 56220
   tctcctttta tctcccttgg cgggtaagta ctcagctctg ctcgttacta atattgaaac 56280
   aacagccctt gaattgagtg atttccctag aaaggttaag gtgaccgaat ctgaacactc 56340
   cctccatgtc ttggacacga agttttttgc tgcgtagaca gttttatccc cctcacccca 56400
   aggtcaattg cacgaattct tttggaaaac aggacctatg gcatttccca gacaaatcac 56460
   cgtgaaccct gtactgtgca ttgctgtcct aaaattaaca cataaatcta ttgccgccaa 56520
   agattctgtc atttgtgtta cataattgcc tttcatttga actcattaat caaattgggg 56580
   tttttaagca acacctaatt aattctttaa ctggctcata ttataccttt aatgacttcc 56640
   accagggtaa aaaccactga tcactgagtt ctattttgaa actacggacg tcgagtttcc 56700
   tctttcaccc agaattttca gatcttgttt aaaaagttgg gtgtggtttc atggggggag 56760
   ggggaagagc gagaggagac cagagggacg ggggcgggga ctctgcaaga aaaaccttcc 56820
   cggtgcaatc gtgatctggg aggcccacgt atggcgcctc tccaaaggct gcagaagttt 56880
   cttgctaaca aaaagtccgc acattcgagc aaagacaggc tttagcgagt tattaaaaac 56940
   ttaggggcgc tcttgtcccc cacagggccc gaccgcacac agcaaggcga tggcccagct 57000
   gtaagttggt agcactgaga actagcagcg cgcgcggagc ccgctgagac ttgaatcaat 57060
   ctggtctaac ggtttcccct aaaccgctag gagccctcaa tcggcgggac agcagggcgc 57120
   ggtgagtcac cgccggtgac taagcgaccc cacccctctc cctcgggctt tcctctgcca 57180
   ccgccgtctc gcaactcccg ccgtccgaag ctggactgag cccgttaggt ccctcgacag 57240
   aacctcccct ccccccaaca tctctccgcc aaggcaagtc gatggacaga ggcgcgggcc 57300
   ggagcagccc ccctttccaa gcgggcggcg cgcgaggctg cggcgaggcc tgagccctgc 57360
   gttcctgcgc tgtgcgcgcc cccaccccgc gttccaatct caggcgctct ttgtttcttt 57420
   ctccgcgact tcagatctga gggattcctt actctttcct cttcccgctc ctttgcccgc 57480
   gggtctcccc gcctgaccgc agccccgaga ccgccgcgca cctcctccca cgcccctttg 57540
   gcgtggtgcc accggacccc tctggttcag tcccaggcgg acccccccct caccgcgcga 57600
   ccccgccttt ttcagcaccc cagggtgagc ccagctcaga ctatcatccg gaaagccccc 57660
   aaaagtccca gcccagcgct gaagtaacgg gaccatgccc agtcccaggc cccggagcag 57720
   gaaggctcga gggcgccccc accccacccg cccaccctcc ccgcttctcg ctaggtccct 57780
   attggctggc gcgctccgcg gctgggatgg cagtgggagg ggaccctctt tcctaacggg 57840
   gttataaaaa cagcgccctc ggcggggtcc agtcctctgc cactctcgct ccgaggtccc 57900
   cgcgccagag acgcagccgc gctcccacca cccacaccca ccgcgccctc gttcgcctct 57960
   tctccgggag ccagtccgcg ccaccgccgc cgcccaggcc atcgccaccc tccgcagcca 58020
   tgtccaccag gtccgtgtcc tcgtcctcct accgcaggat gttcggcggc ccgggcaccg 58080
   cgagccggcc gagctccagc cggagctacg tgactacgtc cacccgcacc tacagcctgg 58140
   gcagcgcgct gcgccccagc accagccgca gcctctacgc ctcgtccccg ggcggcgtgt 58200
   atgccacgcg ctcctctgcc gtgcgcctgc ggagcagcgt gcccggggtg cggctcctgc 58260
   aggactcggt ggacttctcg ctggccgacg ccatcaacac cgagttcaag aacacccgca 58320
   ccaacgagaa ggtggagctg caggagctga atgaccgctt cgccaactac atcgacaagg 58380
   tgcgcttcct ggagcagcag aataagatcc tgctggccga gctcgagcag ctcaagggcc 58440
   aaggcaagtc gcgcctgggg gacctctacg aggaggagat gcgggagctg cgccggcagg 58500
   tggaccagct aaccaacgac aaagcccgcg tcgaggtgga gcgcgacaac ctggccgagg 58560
   acatcatgcg cctccgggag aagtaaggct gcgcccatgc aagtagctgg gcctcgggag 58620
   ggggctggag ggagagggga acgccccccc ggcccccgcg agagctgcca cgcccttggg 58680
   gatgtggccg gggggaggcc tgccagggag acagcggaga gcggggctgt ggctgtggtg 58740
   gcgcagcccc gcccagaacc cagaccttgc agttcgcatt tcctcctctg tccccacaca 58800
   ttgcccaagg acgctccgtt tcaagttaca gatttcttaa aactaccact ttgtgtgcag 58860
   ttgaaggccc ttgggcacaa tgagagccag tcctccaaac tttcagaaag tttcctgccc 58920
   cttctggcag gctgccaatc accgggcggg agaaggaagg aggggaaggc ggtggaggga 58980
   gcgagacaaa gggatggtcc ctcgggggcg gggatggcgg ggctgtcctg taggtctgtg 59040
   cggccaccgt gattgcccct ctgcgcggtg cccgaagtcc cgctgaaacc tgccgagggc 59100
   agcaggtctg aaagctgcag gcgctagttg cgcggaggtg gcgcagctgc tctggaggcg 59160
   cagagcgaat acgtggtgtt tgggtgtggc cgccccgccc ctggcggttt cctcgttccc 59220
   ctttggttaa tgcgcaactg tttcagattg caggaggaga tgcttcagag agaggaagcc 59280
   gaaaacaccc tgcaatcttt cagacaggtt tgtagactct cttcccactc gcagccgcct 59340
   gaccccaccc aacacaaccc acgagcaatt ctaaaagttg cttaactcac gtctaaaaag 59400
   tgcaaaactt cagggctgcg cgtaaagccc tctagtggcg ggaagaccac aggttggagc 59460
   ttctcatgat tagaaaaata ttaataaaac cccttgagcg attttttttt tttttttgag   59520
   acggagtctt actctgtcgc ccaggctgga gtgcagtggc gagatcttgg ctcactgcat   59580
   cctccgcctc ccgggttcaa gcgatccttg aatgatttct aagcagttcc ttgggacata   59640
   aagaaaaatc ttttaacttt ttactttgtt tcccaaatgt tgcacagttt tgcaacacat   59700
   tgaccttctg gtttcgaacg gttacaattt tagattgtgg tttgccaaag tcaagttgct   59760
   taatttttac aaggccacaa aaagcgcaat tatgccctgc agtttaaaat ggaaaacgtg   59820
   ttggaagata agaaaactta gtttccaact ggaatggagc cagcaagttt cttttcttct   59880
   ttgcaaattc tattgtgtca ttaaagttcg atggaagtat cactatgcac aactattttg   59940
   tgatctaata agggtgaaaa ggagccatct gtccccttgg ctaaggggta ttaatggttt   60000
   ctatgggctt cactatggaa tgtagataca gacattctgg caaatgtggt ggctctggac   60060
   agaaataata ggagtctttg tattcccagg gaagctttgc aacaggctac attcttactg   60120
   aatatgtaat gatgtaagca cggttctaat tggacacaag tatttgctaa catccgttat   60180
   ctaatatctg gcccagactt gagaagtagg taatgtaaaa agtttttaaa gctacaagca   60240
   tacctcacat tttaaaagtc ctttcttgat tgggttcttg tgttctttag cactcttgcc   60300
   ataaaaaata ataacagtaa taaacccaag gctgaaaaac tgaattttaa ctaaagggtt   60360
   tttgtgcgtg tttttttttt ttttcaccaa aattagatgg acttacagaa tttttaactt   60420
   aaaattggaa tccaaaagcc agaagatccc cattatagtt tatagttgta ttccctggaa   60480
   tatttactgg gattaactgc aaagcactct cagatgaata gtgtagtata acattttgaa   60540
   actgaaatac atttaccaaa ttaatttaac cacagcaatg tgtgtggttc attttagtcc   60600
   ttgagcattt ttgattatca tacctgtcat gttttctgca gtgtagtgag ttaacataaa   60660
   acaacatcaa tacaaaagat cctctgtttc gagattaagc aaaattcctc attctcttca   60720
   atgtgataga ataccacatt gatctttctt tggaggttag taaaatatct tttatgtatt   60780
   tttcagggct taacaagtaa aaatcaatgt tttcatcaag tctgatcttt ttgtcaccca   60840
   ctcttcattc atttttccac taaggtgata gaaaagtctc aacagtttaa gaccgtaagg   60900
   ctatgaactc caaatataat tgctgacaag ataagcaatc ctcacgcatc cttttgagag   60960
   gaaataaaat cttagttgca agattacata ttctgatttg gaatgctgag ctttttaaat   61020
   ggaaatatag aaggacggct gaatcagcaa aaatccttta tgtagtttca ttctttgcaa   61080
   gcttgaccag tcattctgaa acaggctaac tgaactgata cagtggcaag tgaaaaagac   61140
   atgcctttac aggatgagtc aaaggagttt tagaagaaaa atccaccaga gaaagccaag   61200
   caaatacagt tcagagttac atttcttttc cattttttcc tgaactgaat ctttggcatg   61260
   catatcctga attgggttat tgaatataaa tctagccttg tacaatggat gccagatgac   61320
   tacatatttg ctttggagcc taaggataag tttcaaaaga tttgagtgga gaagaaaagc   61380
   taaaactctt gaagcacaag tttctgttct ccatgtactc aagtgtacat gaagttgtga   61440
   aaatttgtcc acctctatca tcatgttatt ccatgaaatt acaaaacaaa tcttaaaaat   61500
   gttgtggcat agattttcta gatttaaaaa gtaattaaat taaatgaatt actttatttt   61560
   ttgagacaga gtgtcactct gttgcccagg ctggagtgca gtggcactat gttggctcac   61620
   tgcaacctct gcctcctggg ttgaagaaat tctcctgcct caacctccca agtagctggg   61680
   actacaggca tgtgccacca cacccagcta atttttgtat ttttggtaga gacggggttt   61740
   cgccatgttg gctaggctgg tctcgaactc ctgacctcaa gtgatccacc cgtctcagcc   61800
   tcccaaagtg ctgggattac aggcataagc caccatgacc agccttaaaa agtaatttta   61860
   aaatatcact ggtaaaatgt ggattcagtc atgattgagt gcagtttacc atgtgtgtgg   61920
   acatttattt attttaaaat tgtctgatca ccaccttgag taaaacacaa gcagtcacaa   61980
   ttaaaatata ttagtgagca ggagaaagca cagcatatta tagcactgaa tgatttataa   62040
   acctattcca gggtcataaa atgtgtcaac ggcttttcta tagtaaggag actaggttca   62100
   gatggttaat ctaagacaaa taaatgagat aagccataca cttttacatc ctccatgtcc   62160
   tgtcttttct ctgttcaaaa taggatgttg acaatgcgtc tctggcacgt cttgaccttg   62220
   aacgcaaagt ggaatctttg caagaagaga ttgccttttt gaagaaactc cacgaagagg   62280
   ttagtggagt gactttcggg gaatgaatga gggtaaggca gcccccacgg ttggcagagc   62340
   tgaccgtctg tctgttcttt ttgcaggaaa tccaggagct gcaggctcag attcaggaac   62400
   agcatgtcca aatcgatgtg gatgtttcca agcctgacct cacggctgcc ctgcgtgacg   62460
   tacgtcagca atatgaaagt gtggctgcca agaacctgca ggaggcagaa gaatggtaca   62520
   aatccaaggt aggaaacaaa tcagtgcggc ttcaaccaaa gaaaagcatt gtgttctcaa   62580
   aaccccatac ctgtgtgtga ttcctaaata tcctctagct ccaatgcaaa gctggctttg   62640
   acttcttgct catattgtgt ttgccaccac agcctcccca ccactcacat cacctccttt   62700
   atttatttat ttattttctt atttatttat gagacagagt cttgctctac tgcccaggct   62760
   ggagtgcagt ggcaacatct tggcacactg caacctccgc ctcccaggtt caagtgattc   62820
   tcctgcctca gccccctaag agctggaacc acaggcaagc accaccatgc ccggctaatt   62880
   tttgtatttt tagtagagat ggggtttcac catgttgacc aggcttctct caaactcctg   62940
   acctcaggtg atccaccctc ctcagcctcc caaaatgttg ggattacagg catgcgccac   63000
   cacgcctggc cacatcacct ccttcagaat agcagactct cttcccccta accttgcctc   63060
   caagtaaacc ccaatgccat acctttgacc tccactgtgt tgaaatgagc actgtagagt   63120
   gaactctgaa aatactaatg tcagtactcc actgctcttt ccctggcttt caaaacagaa   63180
   atttaaacct atactggaag acattcagtg agaaatatga tttttttttt ctaagagagt   63240
   caaaagactt gaatgtgagc aatctacatt tctgttttct tcccaacagt ttgctgacct   63300
   ctctgaggct gccaaccgga acaatgacgc cctgcgccag gcaaagcagg agtccactga   63360
   gtaccggaga caggtgcagt ccctcacctg tgaagtggat gcccttaaag gaaccgtgag 63420
   taccaaccct gcagtaaaag agggaaaata atgacccatt ctgctgacta ggctcatgat 63480
   gatacctgaa caaaatgttg agtgagtaaa aatgtatatc ataatgcaaa gaaaatgagt 63540
   tatcaagaca gactcaaaag ggacttcatg gaactcttga aggttttagc ttgcctatat 63600
   cattgcttct aatatgaagg acttggtact cgcattctcc acctaaaatt agagtggtcg 63660
   ccatttgccg ctaatggaaa ttattgcaga aggtctgtaa atggttctgg gaacagctgg 63720
   gtttttctga gaaataacac cagacatctt tctcaccccc tgcagaatga gtccctggaa 63780
   cgccagatgc gtgaaatgga agagaacttt gccgttgaag ctgctaacta ccaagacact 63840
   attggccgcc tgcaggatga gattcagaat atgaaggagg aaatggctcg tcaccttcgt 63900
   gaataccaag acctgctcaa tgttaagatg gcccttgaca ttgagattgc cacctacagg 63960
   aagctgctgg aaggcgagga gagcaggtag ggaactcaga cttggatgcg tgaactaatg 64020
   gtgaccattt gttaggccct gtgccactgg gctctaagca gtgtcacatt taatctttag 64080
   aaagtttctt tgaggtaact gctttccact ttttgtagag gaggaatttg aattgagaga 64140
   gagtaagtga cttgctgaaa aagggttaat caacagcaga gctgggattt gaacccataa 64200
   ctctgtcaaa gcctccactc ctaactcctg ttcatgctcc tgtggagaaa atgcttgtag 64260
   taacatattt taaatgtact aacaagacca gtcatgggaa aatgtttctg agacaaatct 64320
   ctagtttatg atttaaaaca gtacgttttc ttacgtgacg aaaacaaaaa gtgtgttaat 64380
   ttgttcccag tggttgaagt tatttgccaa caattttact gtttctcttc atctgtttat 64440
   aggatttctc tgcctcttcc aaacttttcc tccctgaacc tgaggggtaa gcattttatt 64500
   tccctttagg aaaaacgtca gctgcttgta accactgtgt ttatgtcaaa gcattcattt 64560
   tttttaggat atctgaaaaa atgccatata agagaaaact ctataaaaca tctataattt 64620
   tcgaacccaa gtacactctt gcattctatg ctttaagtta aatgcaaact cctttttcct 64680
   tcttcctgct gcaagtacta tctcatcctg atgctcaaga gtgtcagggc ctgggtttcc 64740
   aaacagagac taccctaaaa ttatttggcg agtagtactt tacacaattg cctctccccc 64800
   acaaatcata attgtttcag taaaatggtt acttggtttt tccaagaaaa aactcgtttt 64860
   tactcatttt tggcctgttt gtttatttag aaactaatct ggattcactc cctctggttg 64920
   atacccactc aaaaaggaca cttctgatta agacggttga aactagagat ggacaggttg 64980
   gtatctttta aggaaaaaat agggtaatct cagacaggag ttgatatatt ttaaaatcag 65040
   tgaatctgaa tctcagatac agctggctaa tttgagaggt tcaggtttca ttcatgccta 65100
   ctaaaaaaag aataggcttc ttcttccagc agtacacaca gccaactaat tatttggctc 65160
   ctggatgtga agttgagata gcagtcttcc tgtgctccag aattagtgat ttgctttggt 65220
   gcttaatttg aagtgggagt aagcttcctt aaaccacttc ctaaagcagc tacatgaaac 65280
   agcttcacta gactacctca atatgaggaa tgttttgatc ctggacatat ggtgtcttcc 65340
   tacctccata ctttatagat tcctaaaccc atctatataa tacaagcatg tgccatacga 65400
   tcatttagtt tcttattacc tccctatgcc aggaaagaaa tagttgcaat ttattgtagt 65460
   catcatgaaa tcttcccttg cacataaatt taaaatgtac ctgctgcaca ttttaatatg 65520
   tcttaattgc ttttaaactt ggctgtattg tgtacaacta ttataccatc ttttataaac 65580
   acagtttttt aagaaatttc tttttgtaag ttacaacatt ccactggatc cttatattgc 65640
   ctgtagtgga agagggtctt gtgtgtctgc cccttctagt tttcactcat gcagaagcaa 65700
   cataaccttc tgatttgcac aataaattac atatatttag caggattttt atttgccgtg 65760
   atatatagga taatttagtc tttggcatgt ggcattatat ttattttggt tttttttttt 65820
   aaacaggtta tcaacgaaac ttctcagcat cacgatgacc ttgaataaaa attgcacaca 65880
   ctcagtgcag caatatatta ccagcaagaa taaaaaagaa atccatatct taaagaaaca 65940
   gctttcaagt gcctttctgc agtttttcag gagcgcaaga tagatttgga ataggaataa 66000
   gctctagttc ttaacaaccg acactcctac aagatttaga aaaaagttta caacataatc 66060
   tagtttacag aaaaatcttg tgctagaata ctttttaaaa ggtattttga ataccattaa 66120
   aactgctttt ttttttccag caagtatcca accaacttgg ttctgcttca ataaatcttt 66180
   ggaaaaactc ttttgttgtg ttatttattg gataatatct aaacaattct ctacttggtc 66240
   ctattagtta atttgtcatt acaatcatgt aagttgataa attcaggtta tttatgcttg 66300
   agatgtagtt cttaattttg tcatttttga tagacctcac ttctttttat tattacttaa 66360
   aaacatttac aaataggtgg tgtcgaataa aataacttgt accaaaatga agataggtct 66420
   ctctaaaatg agctcaggtc tgtgatttta atcataacaa acagacttcc taaaattaaa 66480
   aaataaaaac tttttttagt acatatagca tatgagtaac acaaattcca ctttgggagt 66540
   taaagcatag gaagttgcca agatataggg gcttatcttc cgctagcaag atgcagagaa 66600
   atggaaaaag ttcaccaagt ttttctttta tttaagacag gatctcactt tgtcactcaa 66660
   gctggagtaa agtgactcaa tcatagttca ctgcagccaa caactcctag gctcaggcaa 66720
   tcctcccacc tcagcctcct cagtagctag gagtactcaa aacaaataaa cgaatgtcta 66780
   actttagcct aaaatgtgag ttgtcttccc aaaactagcc acacacctgg ctaatttttt 66840
   taattttttg tagagatggg gtctggaagt gttgtccagc ctagtctgga actcttgtgc 66900
   tcaagtgatc ctccctcggc ctcctaaaaa gctgggatta caggcatgag ccaccacact 66960
   cagcctagtt caccaagatt taatatatga aaccgttctg ctttattgtg tgtgtgtgtg 67020
   atagctatat ctatatccat ctatctatag atacagatat ttgtaaagac ctttgtgttt 67080
   ggagaatggc attaggcaca aaataaaacc tcctaccttt tcttccaggg agatgagatt 67140
   attacatgtg aatcgcatgg tgctaactgt aggtgtaata aggtttcaaa gtctggagca 67200
   ataaaaatga agtggggcag tcatggaaag ctccctagaa agcactcagt gtgagcatag 67260
   aacttacata aatagacgag agaaggtatt gccaagaggc aatagtgtag taatggtcaa 67320
   taatgtgtgt tctggagtga aaacttcgag tacattctcg gacatctcta agtttcaaat 67380
   tatggaaata ttattatctt ctctcctccg agaatgatgt ggtagacatt gtcataattt 67440
   ttctgtgttt gcatctgcct ctctctggag ttaactagtg agatggttct cacaaagccc 67500
   tgcccatttg caggaatgtt cactgtgatt ttcatcattt tggttaccgt gggtactgca 67560
   tgaataaaga aaggattggg gctggatgca gtggctcatg cctgtaatat cagcactttg 67620
   ggaggctgag gcgggtggat cacttgaagt caggagttcc ggactagcct ggccaacacg 67680
   gcgaaatcct gtctccacaa aaaatacaaa aattagccag ccgtggtggt ggatgcctat 67740
   aaccccagct actctggagg cagaggcagt agaattgctt cagcttggaa ggtagaggtt 67800
   tcagtgagcc aagactgtac cactaaactc cagcctgagt gacagagcga gatcctgtct 67860
   tagaaataaa gaaaaaaagg attggctgct gaagtaactc ctagccattc taaaggtaaa 67920
   ttctaaatgt aaaagtaaac tttacattct agtttacttg ggacattctt ggtttatgac 67980
   tgttgtgtaa gcataattat taataataac agtttggaca atagattgta tgtggtcagg 68040
   tcatgctacc cttagaataa tcttacagaa aatgtgtctg tcccctcagc ccccatattc 68100
   aaacaactga catggacaat ggcataatta attttattag tgtaaacatc caggtctttg 68160
   ggcccttatg aaaccttttc tatttgattt ttcgttttgc tttctgttct ttagccattc 68220
   tcaccactgt tccccgcagc ctcctaaccc taggatccct gtctcctctt ccacttgtat 68280
   cccacctttg gcttataagg gtcatccagg gaagacccca gccccatatt ggctttcttc 68340
   acacaccaga ctcattatta tttttttaaa aattgaaaaa caagccacag ttttctttca 68400
   ggggaaaaga accaggacag caagttctgc tcatacctat tgagattagt agactcccac 68460
   tcttaaaaat cctttacaga tggaggccag gcatggtgac caaggtgggt ggatcccctg 68520
   aggtcaggag tccaagacca gcctggccaa taaggcgaaa ccccgtctct actaaagaca 68580
   caaaaactag ccaggggtgg tggtgcacac ctgtaatctc aactactcgg gaggctgagg 68640
   cacgagaatt gcttgaaccc aggagatgga ggttgcagtg agccgagatt gtgccactgc 68700
   actccagcct gggcaacaga gtgagactct gtctcagaaa aagaaaaaat atcctttaca 68760
   gatggaaaca acagacactg ggcacttcaa aaaggaggag gttgggagga gggcaagggt 68820
   tgaaaaacca cctgttgagt acttgggtga tgggttcaat agaagcccaa gctctagcat 68880
   tacgcaatat atctatgtaa caaacctgca catatacccc ctgaatctca attaaaaaaa 68940
   aaaatatctt acagctcttt ctaaagtcac atcccagaaa tggaaaagag gagtgggcct 69000
   cccttatctg tgactgtcag aaattagacc ataatgaaaa gcttcacagg tgccccatct 69060
   ccgaagaatt caaatttatt tcagtcttct tctattttat ctttaattat actgttagtg 69120
   tttttcctta tcttccactt tgaaaaattt ataaaaagaa attatgttct caccacctat 69180
   ggtgaaagag agattaatgc ttggttcctt catgtataat attaatccaa gattaaagga 69240
   gacggaaaaa gtagggggat ggggagaaag aaatggtggc ttaaagtgtt taagtctgaa 69300
   aaaagttact aaggggatgg tgaaatccat ggaactcatg tgactgtcaa gtcctactaa 69360
   gttttctcac agtgacagat ggaaaaggct gctggtgaat gaaacaaatg tctactttta 69420
   gcctaaaatg tagtgagttg tcttcccaca actagccgct aacaaaggcg ctaaaaaaat 69480
   aaaatctctt cccaggctat gagactaagg gagtgagtgt gtgtagtgat gggggaggga 69540
   gacagacttt cactccaact atcgtctgaa tgtttgtgtc cccccaactt tgtatgttga 69600
   aacctaacct gcaacgtcat ggtattcaga tatgtctctt gtgaagtgat taggtcagga 69660
   ggctgaagcc cttatgaatg agattaatgc ccttataaaa gaaactccag gctgggcgtg 69720
   gtggctcaca cttgtaatct cagcaggttt ggaggctaag gcaggtggat tgtttgagtc 69780
   caggaattcc agaccagcct gagcaacatc gtgaaacgct atctctacaa aaaacaaaaa 69840
   acaaacaaac catgtgtggt ggtgcacacc tgtagtccca gttactcagg aggctgaggt 69900
   aggagaatca cttaagcccc ggaggcagaa gttgcatgag ccaagatcat gccactgcac 69960
   tctagcctgg gtgacagagt gagaccctac ctcaaaaaaa taaaataaaa taaaataaaa 70020
   taaaacaaaa aagcccataa tcccaacact ttgggatgct gaggcaggag gattgcttga 70080
   gaccagagtt caagaccagc ttgggcaaca ataagaggcc ccatatctac aaaaaataaa 70140
   aatataaaaa aaacaggcat ggtggtacgt gcgtatagtc ccagctactt tggaggctga 70200
   ggtgggagag gatcacagga acccaggaac tcaaatctgc agtgagctaa gaaggtgcca 70260
   ctgtactcca gcctgggtga cagaggagac cccatctcaa aaaagaccct taaaaaccca 70320
   cttgtccctt ccatcatgcg aggacacagc aagaaggcac cttctataaa ccaggaagtg 70380
   gccctcacca gacacggaat ctgctggtgc tttgatcatg aacttctcag cctccagaac 70440
   tgtgagaaat gtttctgttg tttaaaagcc acccagtgtg catgattttg ttacagcacc 70500
   cgaaacagcc taagacagct ccatcctaag gattactcat gagctttgtg aaggaagcaa 70560
   ggataaagca gacactatgc ccacacgaag tctccctctc caacctgcca gaccagctgc 70620
   atgaagaagg gtctgtacta ctcagctagt tttactgtaa cagaatacca cagactgggt 70680
   ggtttaaaca acagaatttt attgtttcac agtaacagag gctggaagtc caagatagag 70740
   gtgctggcag ggttggttta ttctgagcct ctcccattgg cttgcagatg acacgttctc 70800
   tctatgacct cacatggtca ccccttagtc tgtttggtct gtgtcctagt gtcttcttct 70860
   aaagacacta gtcccattgg attagggcct acccatatgg ctccatttta ccttaatcac 70920
   ctatttaaag gccttatctc caaatactgt tacaattttt tttttttttt ttgagatgga 70980
   gtctcactct gtcacccagg ctggggtgta gtggcacaat attggctcac tgcaacctct 71040
   gcctcctggg ttcaagcgat tctcctacct caacctccca agtagctagg attacagcta 71100
   cacaccacca tgcccagcta attcttgtat ttgaatagag acagggtttt gctatgtttg 71160
   ccaggctggt ctcgaactcc tgacctcaag tgatctgccc acctgggcct cccaaagtgc   71220
   tgggattaca agtgtgagcc accatgccca gccctccagt acattttgaa gtactggagg   71280
   ttagggcttc aacatatgaa ttttggggaa acacacttca gctcatacta ggatcaccac   71340
   taaacattgg gaaacactct ccttgtctgt actcacaata ggtctgtgat acagattttc   71400
   aaaagctgta aatttgcaaa atcattgata gggaatgcat ggcttacaga tttgttgatc   71460
   atttgtgact gcccagcttc tccatttttt tttttttttt tttttggtcc cttgtctaga   71520
   gtcagggcag tttgaggtca gttccatgga gtctttctac aaagatcttt catgtcaagc   71580
   aaatacttct tttagggcac tgtaggaggt gagcaggcag ccgtcaaaga ccttggaaga   71640
   gtcaataagt agggtacccg caagtgaaaa tttttctcat tccacaatac cagaaggccg   71700
   atttcttagg aagtttcagt ttcttgtatg tatcaggcac tatgctaggt gctggctgta   71760
   caatgataaa gtttcctccg ttactgagtt cacagtctat cagcctagtg ggaaagaaca   71820
   gagaacataa ggaaaatgca tgaggtgtac tatatatgtt taactgtaca tagtacatac   71880
   aaaagctata cagcacaagt aaagcctaca ggacatgctt acaggcaagc tctgtctact   71940
   tcttaaatca actgtaatcc attaattcca gtccccatat aacagctatc ttctccagga   72000
   atattgacca gtacggccta cccgttttac ttggtaaatg tggcaaagca gaccaccagg   72060
   tcaattcagt tcaattcaat tctccttgac gcaaggtaac ctgaaggagg gtcaagttct   72120
   gtccttggag ttcagacagt agattccagt cctgatgctg ttcataactc aatataggct   72180
   tctgtctctc cagaatatga gatatttttt ctattatctt ctcctttatg ttgttaatta   72240
   tataaattgt acttatatta ttttatttat ttatttttca tagtggctta aatgtccttc   72300
   tctgctaatg tcaacatgtg aataacatgt gaattacatt tatccatctt ttgtttttgt   72360
   tatgatcatt tggtctctct ttttttattt tttagcatgt ctcataattt ctccttaatg   72420
   ctggactttt taaatggata cttaggtagg acctggatga tgttctcttc ctctaaagaa   72480
   caagaaaagt tttctttttt acaggcaaag ggagaaccag gcattacctc gatcctggcc   72540
   agagttagtt ttagcctttg ttagggctgg tctattttaa cattatctta gtactggtac   72600
   ttacttccca actgcgttct ttagccctag tgtgggctat ttctcggcct caactacaac   72660
   ctgcgtgcgt ttatcaaagc ccttccacct tggcagagct aaactctaac ttctgtttct   72720
   ccaacactac tcaggtgctg aaatctctgc ccaactttta gcttcctggg tgctgttttc   72780
   tgctgtgttt cttggagcct cttcctgaac atgcagctta ggagacggcc aataattgga   72840
   atgaattcct gtcccctaag cccaataaga attccatttt atgtttgagc tctctctccc   72900
   tcatttggaa attaccctca tgggaaaagc caggcaaaca tgaattttac cttggatatt   72960
   tttcttcttt caaggagtat aactctccaa ctcaattcag gactgcattg tttgctttca   73020
   taggcattca aacaggtagg ggtgtgtgtg tggtggggca gggtgtgtgt gtgtgtgtgt   73080
   gtgtgttcca aatactatag ttgtttttat ggaggggaga ggtacagaca tccccagctt   73140
   acaatggttt gatgattttt ccactttaca atggtgtgaa agagatatgc attgagtatg   73200
   ctccttgact taagatttgt ttactgggag gtaaccttac cataagttga atagcatttg   73260
   cattccagtt atctattgcc actctgaaac atcgaactgc aaaactcagc agcttaaaac   73320
   aacaatttat gtttctgtga gttggctcag ctggagagtt tactagggaa tgttcatgca   73380
   attacaatga gatggcagct gagctgtggt tatctgaagc aaactgtact ggacttgtaa   73440
   gatgtcttct tcattcttct gtctctttct tcagtgctcc ttgacttatt ttactttcta   73500
   ctaattatct cagaatccag ggcctaggca tctcacagca agatctcagg ttatttatgc   73560
   ttctggcatt gtgactcact gctaagaggc agaaagcagt aatgtccagg tttgataagg   73620
   gttgcatctg taaatggcac aggataatta ccattatatt ttgtcagtca aataagtcac   73680
   aaggaccacc caaattccag gatagagaag cagattccac ttcttcatgg gagagtggca   73740
   aggtcacact acagaagagc atgtgagatg agataagatt gtgtgcaatt ttggcagata   73800
   caatctacca tagatacaac ctattcttct attgtttact ttatgatttt tccactttat   73860
   gatggtgcaa aagtaccatg catttggtgt gctccttgac tgaagaagag tttattgcag   73920
   ggtagctcaa ctaggttaat gaaacaccat aaatctttta aacaagaagt ctcaatcaaa   73980
   actgactgaa gaagatatga aaaatctaaa taggctggga gcggtggctc acacctgtaa   74040
   tccgagcact ttgggaggcc gaggagggtg gatcacctga ggtcagaagt tcaataccag   74100
   cctggccaac atggtgaaac cccgtctcta ctacaaaaat tcgctgggcc taatggcatg   74160
   cgcctgtaat gccaactaca tgggaagctg ggaaaggaga attgcttgca cccgggaggc   74220
   agaagttgca gtgagcagag gtgacgccat tgcactccag cctgggcaac aagagcaaaa   74280
   ctgtctcaaa aaaaaaaaag aaagaaagaa agaaagaaaa atctcaatag tctcgttacc   74340
   atcaagtcaa gtcaagttct gccaagcatt taatatacaa agtcaagtca aatgcttcca   74400
   gatcagaaaa tgaggaaaca ttatcaaact tactatattt gtgaatctat tcgtgctact   74460
   gtcatgaaat atctgagact gggtaattta taaagaacag aaatttattt ttcatagttc   74520
   tggagctggg aagtcgaaga tcaaggcgct agcaggttcc gagtctggtc aggtcagctc   74580
   ttcatttcca agatggtgcc ttgttgctgt aatgttcccc tctggaggaa tattgtgtct   74640
   tcagatggca aaagagatgg aaggggtgaa aagggttgta ttccctctgt caagaccttt   74700
   tataagaaac ctaatcccat tgatgagagt gtaactgtca tgacttaatc accccctcaa   74760
   gcccacgcct ctcaatactg ctgcattggg attaggtttc aacgtgaatt ttgaagggaa   74820
   caaaaacagt agcataacct taattccaaa actatacaga gatgcaacag gagatgaaaa   74880
   ttgcagccta actttattca gggatattga ggcaaacatc ctaatgaagt atcagcaaaa   74940
   caattgtgtt ggctaaggtt ctttgagaag caaaaagtgg gcttagatgt acaagagact   75000
   tgtagagaag gataaatggg aagaagtagg agtaggcagg taagacttca ggttttaatt   75060
   taactcttac accagcgaaa ggagagaggg aaggatggag gaataggagt acctcggatt 75120
   gcagggcaga ctcaagaaaa ttttggccaa gtcaatgagg gggtccttaa gtcagtcaca 75180
   gtttagatga attttatgtc ccataagaat gggtcagtat cataatcttt accacatgca 75240
   gtcattggtt gggaggaggc tgggggaagc atgcttttga cccagatgta gtgatgatcc 75300
   agaagggtgg caagtggagc tgtcagttaa tgatgctccc tgaaacaaga gatgttcatg 75360
   atctattttc cttgccacta cacaaatcta gcaatatatt ctaagatgtt taaatgtatt 75420
   atcagtgtat aaagataata tgcatgttgg atatagctca gaaaagtaat attattttaa 75480
   tattcaaaat tgatgtacac aatttaatac attaagaaat tagaggaaaa aaatggtgag 75540
   atgcaatggc tcacacctgt aatcctagtg ctttgggagg ctgaagtggg tggatcacct 75600
   gatcccagga gtttgaggcc agcctaagca atatagtgaa acctcatctg tgcaacaaat 75660
   taaaaaaaat taagaagaaa ataatttaaa aaacaattag atgggcatgg tggtgcatgc 75720
   ctgtagtctc agctacccag gaggatgaag tgggaggatt gcttgagccc cagggattga 75780
   ggctgcagtg agttatgatt gaaccagtag actccagcct gggcaacaga gtgagaccct 75840
   agctcaaaaa aaaaaaagag aggaagaaaa aaaattaaag cagaaaatca tgtgatggag 75900
   aggggaaaaa ggaaaaaacc tggcaggcag gcagttaggg tgggtcctca gttgaatttt 75960
   ttcaaacaaa agaacagcct gaaaactcaa gctgcaggca cagataaggg aacttgaaca 76020
   agggggttgc ttgagacatg cctacagcca cacagataag aaacactaca caggtgactt 76080
   gtccagacat gcctgcaatg gaaaattctg tcccctaaca tgtgcacacc aaggggaaca 76140
   aaacaatacg gagtaactca acctaatggc ctgcatgtgc attaggagga cagggtggag 76200
   ctaccagaaa tccacacctt atgcaaatga gatgtctagc cctcatcggt tttttataaa 76260
   agcttttgca ttcaattgta aaaatggcaa cccttttcca ggccctttct ccacagcaga 76320
   gcgctttctt cttttgttta ttaaactttt gctccaacct caccctttgt gtccacactc 76380
   cttaattctc ttggtcatga gacaaagaac tccaggcgaa acctcataac gagagactgc 76440
   tacattgtgg tgtattggct aggctgtaac atgatctgcc taatcatgtt gaaacatcta 76500
   atcattatga aaacttaaga aaaaaatagg aatataaaga cttttcttaa cttggtaaaa 76560
   gatctataag aaaccaaact ttgggaggct gaggtggggg ttcacttgaa gccaggagtt 76620
   caagaccagc atggtcaaca tggtgaaacc tcatctctgc taacaataca aaagaaattt 76680
   agctgggcat gattgtacat gcctgtaatc tcagctactc aggaggctga ggctggagaa 76740
   acacttgaac caaggagaca gaggttgcag tgagcagaga ttgtgccact atactctagc 76800
   ctgggtgagg gagtgatacc ctgtctcaga aaaaaaaaaa aagaaagaaa ccaagagtaa 76860
   atataaaaaa caatactgaa ataatataac tagtccctat aagatcaaaa gaatacccac 76920
   tatccatccc cactcctatc caatattgta ccacaggaaa aaaaaaaaaa aaggtcaggc 76980
   acagtggctc atccctgtaa tcccagcact ttgggaagcc aaagtgggag gattgcttaa 77040
   gcccagcagt ttgaggttaa cctgggcaac atagtgaaac cttatatcta caaaaaatta 77100
   aaaatttaac tagacatggt agtccatgcc tgtagtccca gctactcagt gcaggtaggg 77160
   ctgaggtggg aggatggctt gagcctggga agttgaggct gcagtgcact atgatcatat 77220
   cactgcattt ctggctgggt gaaagaatga gaccctgtct caaaaaaata aagacttaaa 77280
   aggcatagtg gcctggcaca gtggctcaca cctgtaatca cagcactttg ggaggctgag 77340
   gtgggcggat cacgaatcag gagatggaga ccatcctggc taacacggta aaaccccatt 77400
   tctactaaaa aatacaaaaa attagctagg catggtggtg ggcgcctgta gtcacagcta 77460
   ctcaggaggc tgaggcagga gaatggcgtg aacttgggag gcggagcttg cagtgagctg 77520
   agattgtgcc cctgcactct agcctgggca atagagcaat actccatctc aaaaaaaaaa 77580
   aaaaaaaaaa ggcatagtaa tagaagagat taaaatgtca tgaattacaa atgatactat 77640
   tgcttatttg gaaaatccaa aactatttac aggtaaatta tgtataaaga aaagcaaaag 77700
   gccaagagga gataagacac cccagacaag ataagataag ataagacaag ataagataag 77760
   ataaggtaag gtaagatgag ataaggtgag aatgggataa gataaggaga atatcaagat 77820
   agaaaaacac atcctactag atacggctgt agtaaaatgt aatagtgtag attggtacag 77880
   aaacaggcac caaggaaaca taataaagca ctcaagaatg aacccatgtg tatgaaattt 77940
   tatttttgat agagcaagca ttgctgtaac atgcattaat tagaaaatta cctcgaggag 78000
   gacccttaaa agggagatgc cagcattata taaccaaaag tttttctgac agttttctgt 78060
   aacatgtacc aacattctct ttctgtcccc tgtcttctga cctgaagaaa tgtgttcctt 78120
   ctttaaggaa aaaggatgat gaatggatgg ttttaatgtc cctttatgac ttttcaagga 78180
   tttctttgac aatacacaga tttcatacct aattgtccac caactacaaa caaaattaat 78240
   tttagataaa ttcatagtct gaagttaatt ctggaaagcc catattatgt ttgacaatct 78300
   taaaaacgtt tctctttcta tttgttgcca atttcacccc acaaatctta tctttgaatt 78360
   ccaattaaat atggataata taaattacca ttgtagaatc cagtgtatgt agggttacgg 78420
   gtgtagtcag ataactgaat gttctcctta ccacctccag ataaattctc ttcccctctg 78480
   tgattcactg atcataaatc ggtttactgt gaggaaaaaa agaaatggat aggttgacgt 78540
   tgtccccagg tggtaggtag gatgaactgc ctatgaaaca atgcaatcca ctccaggcag 78600
   tacacatttc aaaatgcttc tccctatggg gtttgacagg tacagacaat tgttgttcca 78660
   aatgccatgt gctttagcaa atcattctgc ctattgtgag tagtcacctt tcaagccacg 78720
   agttaccatt tgagttcttt tgcctcaaac tgatggagaa aatgaaaaca gaatgtcacc 78780
   catgcagttc cagtctgttg tattcaagaa tttgatatat tcagacctat gcctggcttt 78840
   cagtaccatg agctaatgac atttcttggc ttgactataa agttctcttg acaatactgt 78900
   gtggatgaga ttttaccgtt tgtgctaaag tcaacaacaa aagcaggacc tggtaatgcc 78960
   aacaactatc taaagaaggt cgatcagaaa aacgacaagg tggcctattc caaagcaact   79020
   ctgcattctt ataattttgt taatggacct tctttttaat aggtttctgc aaataaagtt   79080
   tgaggttata aaatgtgagc ctactagaat gacacaaagt tgaatttcaa atgttgtttt   79140
   tttggatgtt ctaaaaatac attgtaaatg gatttctgct aaggatggac tactgtcttc   79200
   aaagctagag aagataaatt gtggaatcaa gccagctgct cagagaacat agagaggttt   79260
   ggggtctctg gttatgcata ctaagtgcaa agctatcacg tggcccagag gcgggcaggc   79320
   ctgcttgact ttaataatca gtgcctgcag taggtagcct gtccttggga aatgggtgtc   79380
   tatagctttg ggcccatgtg tagcctctgg ttggctacca tgcccactcc atttttatgc   79440
   tcattttccc agttccagag agatcaatgc tgaggctggc taatattaat tggctgtgtc   79500
   attttgtcca cttgactgtt cagtgtgact tctgtggtgg gatgctctct ggtggccatt   79560
   aacatgtgat acaaaaactt tgtgtacaaa actttttgga tcacatgtta atgttttcat   79620
   tgctgtgcat ctccattttg tccatccacc tgcctctacc ccagccctcc ttgtgtctaa   79680
   tggtctggtc cttttcttta caggcctttg acccgctagc catgctgttc gtctcagcct   79740
   agggatatct atataatctt accttgggtt ccttctcctt ctatgccaga aacaccactt   79800
   gcatcctgct cattaggaaa ctttttttcc cctccccatt atcttttttt tctttttgag   79860
   atggagtctt gctctgtcgc ccaggctgga gcgcagtggt gcaatcttgg ctcactgaaa   79920
   cctctgcctc ctgggttcaa gcgattctcc tgtctcagcc tcctgagtag ctgtgattac   79980
   aggcacgtgc caccgtgtct gctaattttt gtatttttag tagagacagc gtttcaccat   80040
   gttggccagg gtggtctcga actcctgacc tcagatgatt tgcccacctc ggcctaccaa   80100
   agtgctggga ttacaggcct gagccactgt agccagcccc cactatcttc taaggccacc   80160
   tctgaatatg tcagcaatgg aaccaccagg tattttcatc atatacccac aaactgagcc   80220
   aacccatata taaaccaaac tcaagttgtt cctcgttcat ctggttgtgt ggcatctctc   80280
   cccatagggc cacgggtgtg agctggggaa gggggattaa tgcaaccatg gatggtgact   80340
   tggggacctc tgggctgcct gatcctgcag cttacctgtg ctctctggcc ctgttcaggc   80400
   ttaacttggg atgtatcatt ttcatcttta gtaagatgca gtaacggtaa ctattcctca   80460
   atgatgtgat tctctgcaga tgacatggcc ttccttcaga atccaggggt ctgcatagta   80520
   tttttcctgt ttggactttc tataaatgac acactacatc ttttctcacc gaggacccct   80580
   ccgacatcaa aacgtcagct ggatgttatg gccaatgaat caaggctgct cattccatgg   80640
   gctggacctg ctgcagagtc cttccttggg tgttatggcc aatgaatcaa ggctgctcat   80700
   tccatgggct ggacctgcgc agagtccttc cttgggtgtt atggccaatg aatcaaggct   80760
   gctcattcca tgggctggac ctgctgcaga gtccttcctt ggatgttatg gccaatgaat   80820
   caaggctgct cattccatgg gctggacctg ctgcagagtc cttccttgga tgttatggcc   80880
   aatgaatcaa ggctgctcat tccatgggct ggacctgctg cagagtcctt ccttggatgt   80940
   tatggccaat gaatcaaggc tgctcattcc atgggctgga cctgctgcag agcccttcct   81000
   tggatgttat ggccaatgaa tcaaggctgc tcattccatg ggctggacct gctgcagagt   81060
   ccttccttgg acgttatggc caatgaatca aggctgctca ttccatgggc tggacctgtg   81120
   cagagccctt ccttgctctg gactcacaaa gcaggcagcc ctctgtgtta gctggtgtat   81180
   ggacaagagc agcattccat aaaccaagaa ggttccaaag aggtttaaca ggctgtgtgc   81240
   tgttctttgg ggaaagggat gcaagatgca gcaattgttt ttcactttgg agggaatagc   81300
   caggcatttc cctaacctct gacccctaaa tactttataa agcagcaggt ccctgacttt   81360
   tcacagggtt tatcctccac cttctgctat gcatgtttct taccaaggcc ttcagtgtcc   81420
   tggccatctc ttcctggtcc tgctcaatca gtatgatggc attgatatgg ggaatggata   81480
   tgatattcta tgggaggtct agatctcctt ggattccacc acagctgaga gtggggaatt   81540
   aacagaagcc tggagcaaaa ctgtaaatga atgttcctgt ccattccacg tgaatgcaaa   81600
   ctgtttctga taagaataga agctgggcgc agttgctcat gcctgtaatc ccagcacttt   81660
   gggaggccga ggtgggcgaa tcacctgagg tcagtggttc aagaccagtc tggctaacat   81720
   ggcaaaactc gtctctacta aaaatacaaa aattagccag gcatggtggc acacgcctgt   81780
   agtcccagct acttgggagg ctgaggcagg agaatcactt gaacctggga ggtagaggtt   81840
   gcactgagcc atggttacac cactgcactc cagcttgggt gactaaatga gactccatct   81900
   caaaaaaaaa ccaaaaaaca cacacacaca aacccacatt tgccaaatca atggcagcac   81960
   gctcttactg gagacattat taatctgttc tagcaatgcc actttcagca ctgcagccaa   82020
   aataggacta ctacttggtt gagtgtgcat cattagtcta cagtcatttt cttagatcca   82080
   gccagtttct gcaaggctca ggccagtggt gaattgaaca gagataggac agagctcacc   82140
   attcctgcat tcctttggtt tttaatgatg gtatttatgt ttgccacccc ctggtatgtg   82200
   atattgtttg tgatttacta tcttgaccat ggagggggca agattcagac ttctacttga   82260
   ccttttctac tgtgatagct cttacctcgt aagctaatgt cccaaggtgg ggatactcct   82320
   gctgtcaagt acattcattt caactctaca cttgccgact agaaaaatga tgagtggact   82380
   tgtctatgca cccagtgggc ccactctgaa ttacacttta cccagaaccc tgtttgtttc   82440
   tttgtcccca aatcccccat tctaacagag gggccatagt gttgcttcag atctctgggt   82500
   actataatca gttgagagcc tgtgttcaaa agtcccccaa aaggtctaga gattccactt   82560
   accccagtga acagttgctc aggtaaacag ctacagatct gtctggagaa aagtcttggg   82620
   aatcattaca ggaattattg ctgcagcatt gcagagttct ttctcttagg gctccaacta   82680
   cctcttgagc caatagcttc tagatctgaa aattgagtaa agtctgagaa ctgagcaagg   82740
   tatgaaaact ttttattggg tgatcaccat tagttttctg ctcctccatt cttgcctttt   82800
   ttcttgttgt agaaattaag aaggcccctt gctggttgac tgtctatttt tcccctaaag   82860
   gagttaggtt ctattaacca tcttcacaac tttctgtagg ccaagactct ttggctgctc 82920
   ttccaagctt gctggtcact atggtcatcg tgactccctg gcttctaatg gttaagtgac 82980
   accccatcct ccaccatcag tccaggcctg cagaccttat cttactagct gttttctctg 83040
   atcactcctg gttccaactg tgtctgttca ggtcttctaa gaagtagaag ccaagacagg 83100
   attatatgta caaaagattt attggatgaa atgtctgtga tatggtttgg ctgtgtcccc 83160
   acccaaatct catcttgaat tcccatgtgt tgtgggaggg acccagtggg agatgattga 83220
   atcatggggg caggtctttc ctgtactgtt cttgtgacag tgaataagtc tcatgagatc 83280
   taatggtctt aaaaatgaga gtttacctgc acaagctctc tttttgcctg ctgccatcca 83340
   tgtaagacat gacttgcccc tccttgcctt ccaccatgat tgtgaagcca gccacgtgga 83400
   actgtaagta cactcgacca ctatcttttg taaattgccc agtcttgggt atgcctttat 83460
   cagcagcatg caaacagact aatacagtct gtgacagata aaagaagctg gagaagaagt 83520
   aggcaggaca agccttcaga tcacagtgta agttttatat ctgtaaaagg agaggaaaaa 83580
   ggaagaattg atctggaaga gtctgagctt acagagcttc tctaagaaaa gtcttggcta 83640
   ggactatggg gtgtcctgaa caaaagttac ccattagagg aatctttcaa caggcaaatg 83700
   gtcctatgcc agtgtacatg cccggctacg tcattgggtg ggaacagcct agtggtataa 83760
   ttggttcaga aagaatgcag gggtatgaca gcttgagact gtcaatcagc tgtattccat 83820
   ttctcaaagg gagctctgaa cctccatagt catctgtttg gtcatcaggg ccacacacta 83880
   attattatta aagaagaata tttccgtaaa tgcagcagaa tctgcaaaga ttcctctgag 83940
   cctccaatga ggagaaaata ttacattttg agacagaaca gtgtatgtgt tcattctcat 84000
   ttttcagact gtgccttggc tctctctgca tttgagaaaa gaaactgatt acatatccag 84060
   ccatcctttg atggcctcat cagctacacg aaggcccaat taaagtttta aaattattgt 84120
   tttcctttaa tgttattaat atggtttggc tccgtgtccc cagctaaatc tcatgtcaaa 84180
   ttgcaatcct catgtgttgg aggaggggca tggtaggtgg ccattgaatc atgggggcgg 84240
   acttccccct tgctgttctt gtggtaacaa tgagttctca gaagatctgc ttgtttaaaa 84300
   gtgtgtagca tttccctctt catgctctgt ctctcctgct gctgcgtgaa gaaatatttg 84360
   cttccccttc ccctttccac tctgattgta agtttcctga ggcctcccca ggcaccttcc 84420
   tgtacttcct gcagaactgt tagtcaatta aacctctttt ctttataaat ttatactagg 84480
   taatttatgt aattacccag tctcaggtag tgcttcatag caatgtgaga agggactaac 84540
   acagtcattt actcaaagta actttccttg atcttcttct ttactttgtt tccttatcct 84600
   accttacttt tcttctacac actcattact atctatgtta ttccgtattt atgtgtttgt 84660
   ttactttata attttctgtg tcctccatta gaatgtaagt ttcatgccag tagaagcctc 84720
   ttctgtcttg ttcactggag tatcttcggt gttcagttca tactgcctgg cacagagtat 84780
   gtgcataact tgtgattatt ttgaaagagt agataaaatc tgtggtaggc cagcctcccc 84840
   catacatacc taaaaccagt cctcagagtg gaggatgggg atagagaact aataacatta 84900
   tagacacttc acaatcatcc tacaaataaa gcattatcac ccccattttg cagataagga 84960
   aactgtggtt tatagaagtt aagtaatatt gacaaggtca tttggctagt aaattacaaa 85020
   gctgggctct gaatgcagtt acacactata tgtcctcaac cactatagag tattaatagc 85080
   aaaaaaaaaa aaaaactctc tagcttgtaa atagatgagg ttctgggaaa cactggacta 85140
   gttcaatttc caaacttctt gaaagtaatg actctcacca tcaagctaca taatcgctaa 85200
   attataaaac cggcagtgtg aaaaatccta aagcacttct gaattgtcca cctaacaact 85260
   gattacatcc tcttctgcaa agggaaataa aactgtttgc taatcaggcc tcagaaaggc 85320
   ttaagtgttc cacaccgaat atcagaaatc gggaaaatca ttaagtcatt acctcgtgcc 85380
   aaattgaatg ctacagtgta cttttctggt attttaacct gtctagcatc taatgactca 85440
   gaaagcattt ctcttattta atccctctca gcgttttttt tttccttact gagaagctac 85500
   tattaacctc tcttgtaaag aaagtgagtc tcttcagata tctgtcaaat aattttatgt 85560
   ggttaaccta aagcacagct ttttctaaaa aaaaaataga aggatcgttc tggaaaggag 85620
   acaagaaaaa gcagtccagt gggaagaaca taattgtctt agcatacccg ttgctggcag 85680
   atgcatgaaa aagccacaca aatgagacac cagcatctaa aatcaaatgc ctgtcagctt 85740
   aaattgtagt ctttggtaaa cctgatcaat tattcagcct tcttacacat ttaataaagc 85800
   tccagaacag agcccagaaa atagttgcat ataaaatatt ctttgagaaa taaattacgc 85860
   tttagtagaa agctacagtc tttacctaag ctaactcgga tgatactcca agtgagctat 85920
   ccttcctgct tacttctgcc tttttttctt caaagggcaa tcctttttga attggcattt 85980
   agcatggtag tattaggttg gggaaaaaaa tcgtgttttt ttgccattaa aagtaatagc 86040
   aataactttc gccccaacgt aatagttgct tcattcctgg agagtgggat ctgcttggtt 86100
   taccagccag agggcttgac aaaatctgac tttagtgtct tcgaatgtcc ttttctaaca 86160
   tctgcacaga aatatcttca tgggagaaaa tcatcttttt cattaaaaaa tgtcagtgtg 86220
   ggagtatggg gagaggaaat agggagagaa agagaaggaa agaggcgcac acatacagag 86280
   acagagagag acagggagag agagagggca agagagtact gccttggggt cactaacacc 86340
   cttgaagata tctcagaaga ttgttccaac caagtaaaaa tgtagacaaa aagtcgccgc 86400
   tccttcttca gacacaacgg atcttgtgaa gagcctggta gggcaaaaac ttgagaggcc 86460
   attgtggtgt ccaaagaaat actgacatct actggtgggt tcggaagtca caggactttt 86520
   ccaagggcca catttagttg cacggaattg aaagtattaa aatccagtgg atacaattag 86580
   gcattcctga ggatcccttc taaataaatt tttaaaatta acttatgacc atcaagagca 86640
   tttctgaatt gtctgttctt taatccacca gatagataat cattgactta gtctctgtac 86700
   ctgaaatatt tcacacagtc cagggaagag taaaatgaac aaaattcctt tctctttatg 86760
   accccaggac ctacctcatc aaaaacggtg ctttgaaaaa cagcaagaag aatgggaagc 86820
   aatattgctc agtcagaaga taaacctgtg ttcacatcca ggaactttca tttattggct 86880
   gtgtaatttc taccaagtta ctttgtctcc caaatctcat tctttctcag ctggaaatga 86940
   gaattgagga tacctgccct gaatgactct gtcagtaatt agagaccacg aactcaaagt 87000
   gcctgtggtt cacattacac actgagtaat tgccgggcgc ggtggctcac acctgtaatc 87060
   ctagcacttt gggaggccga ggcgggtgga tcacctgagg tcaggtagtc gagacaagcc 87120
   tgaccaacat ggtgaaaccc catctctact aaaaatacaa aaattagctg ggtgtggtgg 87180
   catgcacctg tagccccagc tactcaggag gctggggcag gagaattgct tgaacctggg 87240
   aagtggaggt tgcagtgagc tgagatggca ctactgcact ccagtctggg cgacaaagca 87300
   acgctccatc tcaaaaaaaa aacaaaaaca aacaaataaa agaaacaaaa aaactcagta 87360
   atgggattgt tgttacattt aaaggataga aacatcctgg gaccacttgc ttcttgattg 87420
   accaagctgt gatttaggag agtacttctc aaactttaat ttgcactcga ctcactaggg 87480
   aatcttgtta aaatgaaggt tctgtttcag ctgccaaggg cggggcctga gatactgcat 87540
   ctccagcaag ctcccaagag atattgatat gctggcctgg gacagcactg agtagcaaga 87600
   atctgtgggg tccaatcccc actagaggtc aagagcattg aggtggcccc tgtcaattgg 87660
   cttcctatgt gaggggaaat agctctcaaa atttcagttg tttcaaagta taaacaaagt 87720
   gtacataaga aaattgccag gaaagatggg tttgcctaat gtagaaacac taaaaattgg 87780
   ccgggtacag tggctcacac ctgtaatccc agcactttgg gaagccaagg tgggtggatc 87840
   acctgaggtc aggagttcaa gaccatcctg gccaacatgg cgaaacccca tctctattaa 87900
   aaatacaaaa aaacattagc taggcatggt ggcacgtgcc tgtagtccca gctactcggg 87960
   aagctgaggc aggagaatcg cttcaacccg ggaggtggaa gttgcactta gccgagattg 88020
   caccacggca ctccagcctg ggcaacagag caagagactc tctctccaaa taaataaata 88080
   aataaatact aaaaatttac ttcacaccca ttcccaatat acagattcta cttatcatga 88140
   tagagaagag gtgtttgggt tctgaatcag aaatgccccc ataggttaga catggtaggt 88200
   cacacctgca atcctagcac tttggggggc tgaagtggga agattactcg agccctggag 88260
   tttgagacca ggctagtcaa tgtagcgaga tcctatctct acaaaaattt taaaattagc 88320
   caggtgtggt ggtgcatgcc tgtagtccca gctactcagg aggctgaggt gggaggatcg 88380
   cctgagccca gaagttcatg gttgctccag ctagggtgac agaatgaaat cccagtagtt 88440
   aatttattct gtttcagctt taggctttct gtagaatctg gcaatggcaa gcaattaagc 88500
   taaggggatc tggagtcaca ggtgaatttg catcctgact cggtaacttg ccatctgcca 88560
   ggtcatagac aacttcctta acttttctag gcctcagatt tctcagagta tattgtgaat 88620
   attgtgaaca attcaatgag ataatacaga taacacatta gcacatatac tgctcaataa 88680
   atgattgtat tataattatt tttagacaaa ataatgcagc tgttatttct ccccagcaca 88740
   cttttttttg agaatagagt ctctctgtgt tcccaagact gatcttgaac tcctgggctc 88800
   aagcgatcag tctccaaatt agctggtact acaggcaaga gtcatccctt aatttttaag 88860
   tatagcacta tggctttggg gaagagtgtg gtagagaaag cagtggaaca gcacagaatg 88920
   ctgttttgca ggaagtaaga tgcaggttta aatcctagtt ccaccaccag aatggtggag 88980
   gactttgggc tgggttatat agtgtctctg ggcttcaggg ttcatctgca caatagagag 89040
   atgacaatgt aggcggtgtg aggattagaa aactctgtca agtcctgcta tgcaatcagc 89100
   cctcaaaatt agttgctatt aacaacagtg ggttttttgg tttatgtttt tgtttttgtt 89160
   ttgagacgga gttttgctct tcttgcccag gttgtaagtg cagtagcaca atctcagctc 89220
   actgtaacct ctgcctcctg agttcaagtt ctcctgcctc agcctcccga gtagctggga 89280
   ttacaggcac ccactactgt gcctggctag tttttagtat ttttagtaga aacagggttt 89340
   caccatgttg gccaggctgg tcttgaactc ctaacctcag gttatctaca agatccgaag 89400
   tgctgagatt acaggcataa gccaccgtgg gatattacat aatacagtgg gatattaata 89460
   ttgatatagt gttttctgac ttaggttcgg tagctcctta gactcccctg actcctctac 89520
   ctagattcct gctttttttt tttttcaccc aggctgcagt gcagtggagt gatcttagct 89580
   cactgccacc tccacctcta ggttcaagcg attctcctgg ttcagcctcc tgaggagctg 89640
   agggaggctg agggattgca ggcgtgtgcc accacgcccg gctaattttt gtatttttta 89700
   gtagggacag ggtttcacca aattggccag gttcatctca aactcccgat ctcaactgat 89760
   ctgcctgcct tagtctccca aagtgctggg attatgggct tgagccatca tgcccagccc 89820
   aaagtcatgt tttaattatt gtaattagtt aaatgatatt ttaaagtatt tttacacacg 89880
   gataattttt tacggtatac aaatgttttc cttacatatt agcatcatca ttttgtaaaa 89940
   caatggtgta aggtaggaag ggtgagtgtg tttttattta tttatgtatt ttacaagcaa 90000
   agcaacagga actcagagaa attaagcaac ttttttaaga ccatggagtt cgcagtgaaa 90060
   gatattctcc ctcccacaga acttatggta gagcctcatg gagcagaaag tagtatttaa 90120
   ttgggctggt gaaaatgccc tgtgttttta ccactattgg gtgattgaga cttgagcatg 90180
   attgacggaa aaagtcactg tatatcggct cagaagaaat gagttcaagg cccagcactg 90240
   ttacttttca ccggtgatgt tgagcaaaac catggttatc tcagattctt gctttacctg 90300
   tagaaggagg actttgaact agatcatagc caatagtctt ctttgctttt aaactggtgg 90360
   tagggatggt ggggttaaaa gaaaaactgt agccaagtta aatttaaaat aatttaattg 90420
   agcaaagaac aactcatgac tcagacagca tcctgagcca gagtgagctc agatactcca 90480
   ggacagccat gtggtggaag aggatttatg gacagaaaaa ggaaaatgac atacagaaaa 90540
   tggaagtgag ggacagaaac agccagattg gttgttgctt ggcatttgcc ttatctgaac 90600
   atggtttgaa cagttggcca atgtgattgg caaaacttgg tgactggcgc aagagtaggc 90660
   tacagtctgt ttacaattcc atttaggtta tggttcacta tgtacagaga aacctttagg    90720
   ctgaacttaa aatatgtagg gaggtaactt taggctaaac ttgatttaac agtgttcatt    90780
   gccagaggaa gagagatgta gaaagcaaaa agttcctctt caaattatcc cttcttgtta    90840
   aagaataaat cataagtgtt agaaatcata gtttctttta aagactaact ttcttcaaac    90900
   ctccttgctt cacactaata actcgttttt aagccctata ttatgtagct gttagatata    90960
   aaggaatgag tacattctat gtccttgtac tttaaccaag atatctgtgc tggacatgct    91020
   cacaggcatg tcccagctcg cagcctatac cccttcctta tttaggaatg ttactacttt    91080
   tctaagtctt ttcataaaca acttccactt ttcgtttgtt gtacattgct tttatctatt    91140
   tagaaaattt taagttgtta gtcaatcagg ttcagtttag attgtgtggt ctggctccag    91200
   ccaatggaga caggacacag tagcaagggc aagcacttta agggataaaa attgcttccc    91260
   tccttctcgc cattattcca tctgcgagga gcaccctttc tgcagaaagt aaatttgcct    91320
   tgctgagaaa actttttggc tgaatggtga tttttccttg cagtaccgag gaacaagcat    91380
   tctgtttctg aataaacatt ttacttataa caagagataa gattaaatat aagaatatat    91440
   tatattctct attttagata agcttgtcct tgaatgttga gcatataatg tcatccaaag    91500
   catcgtccaa cagcaacact attctcctgt tagtacccta ttctcccttt taggaaatct    91560
   cccatagtgt ggaagtcttt ctactgaaat ctaagtaaca gacaattcct ctttctacca    91620
   caaagcagtc agggatggag gactctttcc ttggacttca aatcttcagt gagtgttagg    91680
   aaggtagaga gatgtctgag gttcatgttc acggccacat ggttgatgat ttatggacta    91740
   gacacagcgt gctcttttgt gtgattccag ctctctagct tccccagact tcttggctgt    91800
   tttttccaag cctggtcgtc catctgccat ctcattccaa taaattggaa atgcagttca    91860
   agatagctag agttggagtc tgtggtattg ggggaacccg cccccaatat ttcaatgtag    91920
   gttctttcta ttttccctaa gtgtcggcgg gtctgagaaa taaagagaaa gagcacaaag    91980
   agaggaattt tacagctggg ccgctgtggg tgacatcacg tatcagtagg actgtaatgc    92040
   ccacctgagc ctgaaagcca gcaagtttta ttagggattt taaaagggga gggggtgcaa    92100
   gaacagggag taggtcacaa gatcacatgc ttcaaagggc aaaaaggaga acaaagatca    92160
   catgcttctg aggaaacagg gtaaggacaa aatcaggaac tcctgagaag ggtctatgtt    92220
   tagctgtgca cgtattgtct tgataaacat ctcaaacaac agaaaacagg gttcgagagc    92280
   agagatctag tctgatctta aatttaccag ggtggggttt cccaatccca gtaagcctga    92340
   gggtactgca ggagaccagg gcatatatca gtccttatct caaccgcata ggacagacac    92400
   tcccagagtg accgtttata gacctccccc caggaatgca attcttttcc tacggtctta    92460
   atgttaatat tccttgctag gaaaagaatt tagcgatatc tcttctactt gcacgtccat    92520
   ttgtaggctc tctgcaagaa gaaaaatatg gttttttttg cccgacccca caggcagtca    92580
   gaccatatgg ttgtcttccc ttgttcccta aaatcgctgt tattctgttt gttttcaagg    92640
   tgcactgatt tcacattgtt caaacacatg ttttacgatc aacttttaca gttaacacaa    92700
   tgatcacagt ggacctgtgg tgacgtacat cctcaactta cgaagataac aggattaaga    92760
   cattaaagta agacagtcgt aagaaatttt aagaatatta ttagggaagt gataaatgtc    92820
   cctgaaatct tcacaattta tgctcctctg ccatggctcc agccggtcct tccattcggg    92880
   atccctgact tcccataaca tctggcttgc cactaagacc ctgatgaacc aagcctgcat    92940
   tttttttttt tttttttttt tttttttttt tttttttttt tgctaaagct actgccaacc    93000
   ttcttgtgat gatctgaggg aatacaattt aaaccatgat tcataattgt gcgaggtaca    93060
   tgagtcacca agaaaggcag ggtaagtaat tacaaagtag ttatctctct ttccataatt    93120
   ttaaattgaa tatcacttta ttctccattt acagacagag agattacagg cagtaatctt    93180
   cctttctaaa gctgacagta actcccacag taataaatgg aagcctgttt aataagaaat    93240
   atttacgtgt gtgactgtga gcacagctga aaacgtggtg ttcttcgtaa atttagaact    93300
   gcattttcta aatgtcgagt gtttattacc tggaaaaaag aaaggaaaaa caactctaca    93360
   ttttagacgt gttccacagc ttccttgatg agacatttta aggaccatta gttgtctaaa    93420
   tagagattgt attttagagt ggagactatg aaagagtgtt ctctagttta aggttatatc    93480
   ctttaccttg tgatagagtc aagctgtaaa ctcaaagcaa gcatctggca ctctggttat    93540
   tctaagtcat ctttctttgc tctccctttt aagcatcttt cttcacttta aaaacaaaca    93600
   caaataaaag cacctatcac agggagggcc tcaaacgagg gcttaatagg tgttgctttg    93660
   cttcctcttc tcttttcttt agtaggcaga ctaaattcta tgactctgat ctgagggtta    93720
   acagtagtcc atctatggat tctatggact tgatgtatct tatactgtgg ttcctggaaa    93780
   ttcattttat caggagtgag tgatcttttc agaaaagctg ctcaagcttg agcctgggag    93840
   gtggaggttg cagtgagctg agatcatgcc actgcgttcc agcctggaca acagagtaag    93900
   accccatctc aaaagaaaaa aaaatctgct ccagattttc agaaaggaaa ataagatttt    93960
   acacacacac acacacacgg tttttgtgtc tggtaaacta gcaaactgtc tcatctgatt    94020
   tctttgccat catttctgct ttaaattaga actctagaga atgtaaatat ctaattatct    94080
   actggtcagc ttcagagatt tcaggcttca aagaagttca gtgtggtttt tgtgtgatgg    94140
   tttatgatat ggattattca aggaattc                                       94168
<210> 28
   <211> 106
   <212> DNA
   <213> Homo sapiens
<400> 28
   gagcaggagg atggcgtcct acaagccgcc gggcccgtgg cgctcggccg gctcgaggtc      60
   ggcctcgatg cactgatgca ggtgggcgtg gatgtcggac ccgtcg                    106
<210> 29
   <211> 106
   <212> DNA
   <213> Homo sapiens
<400> 29
   gttgtttagg ttgtaggtgc gggtggacgt agttacgtag tttcggttgg agttcggtcg      60
   gttcgcggtg ttcgggtcgt cgaatatttt gcggtaggag gacgag                    106

## Description

This invention was made using funding from the National Institutes of Health. The U.S. government retains certain rights in the invention according to the terms of CA 43460, CA 57345, CA 62924, and CA 121113.

### TECHNICAL FIELD OF THE INVENTION

This invention is related to the area of nucleic acid analysis. In particular, it relates to analysis of methylated nucleic acids.

### BACKGROUND OF THE INVENTION

In humans, DNA methylation is largely restricted to cytosines within 5'-CpG dinucleotides. This covalent modification of DNA functions as an important mediator of gene regulation and, together with covalent modifications of histone proteins, forms the cornerstone for the burgeoning field of epigenetics. In addition to its role in development, imprinting, and cellular differentiation, DNA methylation is altered in cancer cells. Though cancers are globally hypomethylated^{1, 2}, specific regions of genes have been shown to be hypermethylated in associated with transcription silencing^{3,4},. Reversal of the hypermethylation with pharmacological agents such as 5-azacytidine can reactivate such genes⁴. Though many regions of the cancer genome are abnormally methylated, the methylation and silencing of tumor suppressor genes are particularly important. In such cases, methylation serves as a heritable mechanism to inactivate a tumor suppressor gene, with the end result similar to that resulting from mutational inactivation of the gene.

In addition to its implications for gene regulation, DNA methylation is providing a new generation of cancer biomarkers^{5, 6}. Though mutant sequences provide exquisitely specific biomarkers of this class, their utility is compromised by their heterogeneity: the same gene can be mutationally inactivated through many different mechanisms or mutated at many different positions. In contrast, DNA hypermethylation in cancers often affects identical residues in the regulatory regions of particular genes, providing significant advantages in biomarker test design. Accordingly, many studies have employed DNA methylation of specific genes for diagnostics development⁷⁻¹³. Such diagnostic tests can in principle be used for early detection of cancers, prognosis, assessing the effects of therapy or detecting residual diseases.

Many diagnostic tests based on DNA methylation have employed bisulfite to convert cytosine residues to uracils. This conversion alters the sequence of DNA, providing an opportunity to assess DNA methylation with allele-specific PCR, restriction digestion or specific hybridization probes¹⁴⁻¹⁸. Such techniques have been enormously useful for establishing the position of hypermethylated sequences in the cancer genome as well as for documenting the potential utility of such methylation for diagnostic purposes. However, in many important diagnostic scenarios, DNA from the cancer represents only a small fraction of the total DNA in the clinical sample. Such scenarios include the use of DNA from plasma/serum, urine, feces, or sputum for early diagnosis or therapeutic monitoring and the use of DNA from surgical margins or lymph nodes to monitor the extent of disease¹⁹. Currently used techniques for assessing methylation generally do not provide direct quantitative information about the fraction of DNA fragments that are methylated or are incapable of detecting small fractions of methylated fragments. The actual fraction of methylated fragments can be determined through cloning and sequencing of PCR products or next-generation sequencing technologies^{20, 21}, but these approaches cannot easily be used in a clinical diagnostics setting.

There is a continuing need in the art for sensitive and accurate analytic methods.

### SUMMARY OF THE INVENTION

According to one embodiment of the invention a method is provided for determining fraction of molecules comprising a methylated sequence in a sample of analyte DNA molecules comprising the sequence. A sample of analyte DNA molecules is treated with a reagent which selectively modifies methylated cytosine residues or which selectively modifies unmethylated cytosine residues. Microemulsions are formed comprising the treated analyte DNA molecules. A portion of a treated analyte DNA molecule is amplified in the microemulsions in the presence of beads. The portion comprises one or more 5'-CpG methylation sites. The beads are bound to a plurality of molecules of a primer for amplifying the analyte DNA molecules. A plurality of copies of analyte DNA molecule are formed covalently attached to the plurality of molecules of the primer which are bound to beads. Nucleotide sequences at the one or more 5'-CpG methylation sites of analyte DNA molecules which are bound to beads are determined and beads that have modified 5'-CpG methylation sites and beads that have unmodified 5'-CpG methylation sites are quantified.

According to another aspect of the invention a bead is provided which is bound to a plurality of molecules of a primer for amplifying DNA molecules. The primer comprises at least 15 contiguous nucleotides selected from the group consisting of 5'-GTTGTTTAGG TTGTAGGTGN GGG-3 (SEQ ID NO: 2), 5'-CTCNTCCTCC TACCNCAAAA TATTC-3' (SEQ ID NO: 3), and the complements thereof.

These and other embodiments which will be apparent to those of skill in the art upon reading the specification provide the art with

### BRIEF DESCRIPTION OF THE DRAWINGS

Fig. 1 Figure 1 Schematic of Methyl-BEAMing. "Mixtures" represent beads from aqueous nanocompartments that contained both methylated and unmethylated vimentin fragments. "Virgin" beads represent those from aqueous nanocompartments that did not contain any vimentin fragments.
Fig. 2 Representative results of Methyl-BEAMing obtained with flow cytometry. The number of beads representing methylated, unmethylated, mixture, and virgin beads are indicated in the top right corner of each box.
Fig. 3 Vimentin Methyl-BEAMing of plasma from colorectal cancer patients. (a) Receiver operating characteristic (ROC) curves based on the number of methylated vimentin fragments for the indicated classes of samples. (b) The number of methylated vimentin fragments in 2 ml plasma from normal, age and sex-matched control patients. (c) The number of methylated vimentin fragments in 2 ml plasma from colorectal cancer patients of various stages. The doted line represents 1 methylated fragment per 2 ml plasma DNA.

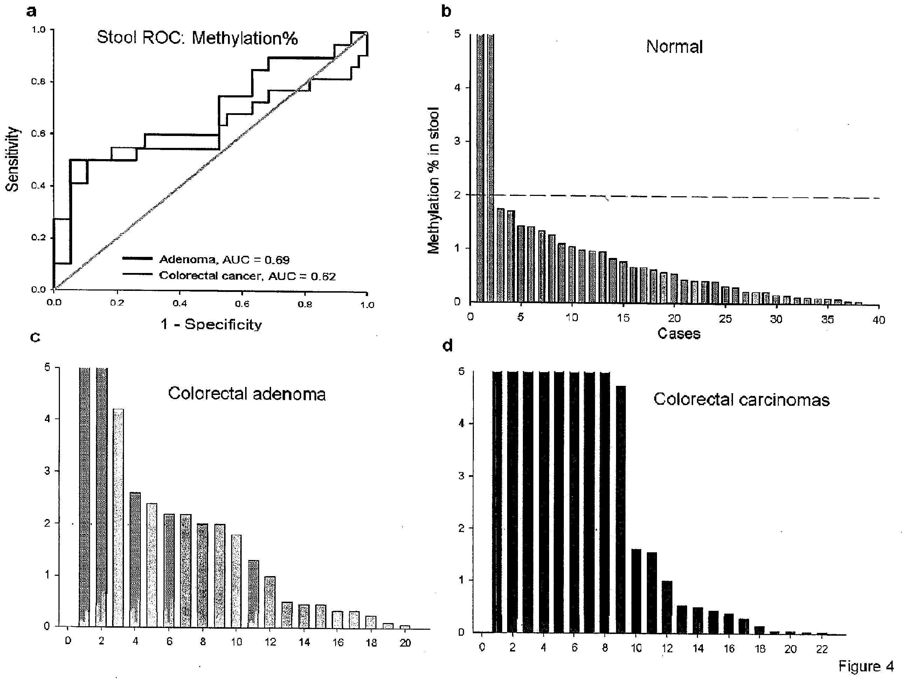

Fig. 4 Vimentin Methyl-BEAMing of fecal DNA from colorectal tumor patients. (a) Receiver operating characteristic (ROC) curves based on the fraction of methylated vimentin fragments for the indicated classes of samples. (b) The fraction of methylated vimentin fragments in 4 g feces from normal, age and sex-matched control patients. (c) The fraction of methylated vimentin fragments in 4 g feces from patients with colorectal adenomas (d) The fraction of methylated vimentin fragments in 4 g feces from patients with colorectal carcinomas (Duke's A or B red; Duke's C or D, blue). The dotted line represents 2% methylated fragments.
Fig. 5 (supp. Fig. 1) Correlation between the results of Methyl-BEAMing and the fraction of methylated fragments used as templates.
Fig. 6. (supp. Fig. 2) Genome equivalents of total DNA in 2 ml plasma from the indicated subgroups of patients.
Fig. 7 (supp. Fig. 3): Genome equivalents of total DNA in 4 g stool from the indicated subgroups of patients.

### DETAILED DESCRIPTION OF THE INVENTION

The inventors have developed a technology for direct quantification of DNA methylation at specific sites that is readily applicable to clinical samples, even when the fraction of methylated fragments in such samples is minute. This approach can be applied to determine the extent of methylation of marker genes in plasma and fecal DNA from cancer patients and healthy controls.

Beads are also known as microspheres or microparticles. Particle sizes can vary between about 0.1 and 10 microns in diameter. Typically beads are made of a polymeric material, such as polystyrene, although nonpolymeric materials such as silica can also be used. Other materials which can be used include styrene copolymers, methyl methacrylate, functionalized polystyrene, glass, silicon, and carboxylate. Optionally the particles are superparamagnetic, which facilitates their purification after being used in reactions.

Beads can be modified by covalent or non-covalent interactions with other materials, either to alter gross surface properties, such as hydrophobicity or hydrophilicity, or to attach molecules that impart binding specificity. Such molecules include without limitation, antibodies, ligands, members of a specific-binding protein pair, receptors, nucleic acids. Specific-binding protein pairs include avidin-biotin, streptavidin-biotin, and Factor VII-Tissue Factor. Primers can be attached via spacer moieties. The spacer moieties may be oligonucleotides.

Beads, after being prepared according to the present invention as product beads, have more than one copy of the same nucleic acid molecule bound to them. Preferably each bead is bound to at least 10, 50, 100, 500, 1000, or 10,000 molecules of the same nucleic acid sequence. In some circumstances some of the product beads are bound to more than one type of nucleic acid molecule. These product beads are generally less useful in the analysis of ratios of methylated sequences in a population of sequences. Such product beads can be readily discriminated and so do not distort the analysis.

A population of product beads will often comprise two or more types of nucleic acids. Such a population is heterogeneous with respect to the nucleic acids. Desirably, a substantial proportion of the product beads comprise only one type of nucleic acid per bead. A substantial proportion can be for example, at least 1 %, at least 5 %, at least 10 %, or at least 50 %. A product bead with only one type of nucleic acid per bead is termed homogeneous. A product bead with two types of nucleic acid per bead is termed heterogeneous. Heterogeneous product beads may result from aqueous compartments which have more than two molecules of template of non-identical sequence. This could result, for example from incomplete modification of methylated cytosine residues. A population of product beads can be heterogeneous as a population but contain individual product beads that are homogeneous.

Individual product beads preferably comprise more than one copy of template analyte molecule. Each bead may comprise at least 10, at least 50, at least 100, at least 500, at least 1000, or at least 10,000 copies of template analyte. If the bead is homogeneous, each of those copies will be identical.

Populations of product beads can be maintained in a liquid suspension. Alternatively they can be sedimented and dried or frozen. The latter alternatives may be beneficial for storage stability.

Analysis of populations of product beads can be used for distinguishing between methylated and non-methylated or hypomethylated templates. Polynucleotides can be distinguished which differ by as little as a single methylated residue, although differences in multiple residues may by better for detection sensitivity. Increased sensitivity obtained using multiple residues may decrease specificity, however.

One very convenient way for distinguishing between the products of methylated and non-methylated CpG islands is by differentially labeling probes to the expected products with fluorescent dyes. Such labeling can be accomplished by hybridization of a fluorescently labeled oligonucleotide probe to one species of polynucleotide. Alternatively, a fluorescently labeled antibody can be used to specifically attach to one oligonucleotide probe that hybridizes to a particular product. Such antibody binding can be, for example, mediated by a protein or polypeptide which is attached to an oligonucleotide hybridization probe. Of course, other means of labeling polynucleotides as are known in the art can be used without limitation. Another means of labeling different polynucleotide species is by primer extension. Primers can be extended using labeled deoxyribonucleotides, such as fluorescently labeled deoxyribonucleotides.

Populations of product beads can be used as templates. Template analyte molecules on the product beads can be analyzed to assess DNA sequence variations by hybridization, primer-extension methods, mass spectroscopy, and other methods commonly used in the art. Template analyte molecules on product beads can be employed for solid phase sequencing. In one solid phase sequencing technique, product beads are arrayed by placing them on slides spotted with complementary oligonucleotides. In another solid phase sequencing technique, product beads are placed into individual wells. In still another solid phase sequencing technique product beads are incorporated into acrylamide matrices (with or without subsequent polony formation). Sequencing reactions can be performed with any solid phase sequencing method, such as those using unlabeled nucleotide precursors (*e.g*., pyrosequencing, as described in Ronaghi et al., Anal. Biochem. 267: 65-71, 1999) or labeled nucleotides (*e.g*., photocleavable reagents described by Mitra et al., Anal. Biochem. 320:55-65, 2003). Product beads can thus be used for and facilitate massively parallel sequencing. Product beads can also be used in sequencing employing Type IIS restriction endonucleases. Product beads can also be used to provide templates for conventional dideoxynucleotide sequencing. To obtain useful data upon sequence analysis, a homogeneous template population is desirable. To provide a homogenous template population, product beads can be diluted, separated, or otherwise isolated so that each sequencing reaction contains a single product bead. Alternatively, product beads can be sorted to provide populations of beads with a single species of template.

Oligonucleotide primers can be bound to beads by any means known in the art. They can be bound covalently or non-covalently. They can be bound via an intermediary, such as via a protein-protein interaction, such as an antibody-antigen interaction or a biotin-avidin interaction. Other specific binding pairs as are known in the art can be used as well. To achieve optimum amplification, primers bound to the bead may be longer than necessary in a homogeneous, liquid phase reaction. Oligonucleotide primers may be at least 12, at least 15, at least 18, at least 25, at least 35, or at least 45 nucleotides in length. The length of the oligonucleotide primers which are bound to the beads need not be identical to that of the primers that are in the liquid phase. Primers can be used in any type of amplification reaction known in the art, including without limitation, polymerase chain reaction, isothermal amplification, rolling circle amplification, self-sustaining sequence replication (3SR), nucleic acid sequence-based amplification (NASBA), transcription-mediated amplification (TMA), strand-displacement amplification (SDA), and ligase chain reaction (LCR). Primers can be designed to hybridize only to modified, only to non-modified, or to both modified and non-modified sequences. Mixtures of primer sequences can be used to capture variants.

Microemulsions are made by stirring or agitation of oil, aqueous phase, and detergent. The microemulsions form small aqueous compartments (nanocompartments) which have an average diameter of 0.5 to 50 microns. The compartments may be from 1 to 10 microns, inclusive, from 11 to 100 microns, inclusive, or about 5 microns, on average. All such compartments need not comprise a bead. Desirably, at least one in 10,000 of said aqueous compartments comprise a bead. Typically from 1/100 to 1/1 or from 1/50 to 1/1 of said aqueous compartments comprise a bead. In order to maximize the proportion of beads which are homogeneous with respect to oligonucleotide, it is desirable that on average, each aqueous compartment contains less than 1 template molecule. Aqueous compartments will also desirably contain whatever reagents and enzymes are necessary to carry out amplification. For example, for polymerase chain reaction (PCR) the compartments will desirably contain a DNA polymerase and deoxyribonucleotides. For rolling circle amplification a DNA polymerase and a generic DNA circle may be present.

Emulsions can be "broken" or disrupted by any means known in the art. One particularly simple way to break the emulsions is to add more detergent. Detergents which can be used include, but are not limited to Triton X100, Laureth 4, Nonidet.

Sample DNA for amplification and analysis according to the present invention can be genomic DNA. Samples can be derived from a single individual, for example, from a body sample such as urine, blood, serum, plasma, sputum, stool, tissue or saliva. Samples can also be derived from a population of individuals. The individuals can be humans, but can be any organism, plant or animal, eukaryotic or prokaryotic, viral or non-viral.

Any type of probe can be used for specific hybridization to the amplified polynucleotides which are bound to the beads. Fluorescently labeled probes are useful because their analysis can be automated and can achieve high throughput. Fluorescence activated cell sorting (FACS) permits both the analysis and the isolation of different populations of beads. One type of fluorescently labeled probe that can be used is a modified molecular beacon probe. These probes have stem-loop structures and an attached fluorescent moiety on the probe, typically on one end of the probe, sometimes attached through a linker. Unlike standard molecular beacon probes, modified molecular beacon probes do not have a quenching moiety. The modified molecular beacon probe can have the fluorescent moiety attached on either end of the probe, 5' or 3'. One such probe will hybridize better to a sequence from a methylated gene than to a non-methylated gene. Another such probe will hybridize better to a sequence from a non-methylated gene than to a sequence from the methylated gene.

Microemulsions or nanocompartments are formed with beads and primers. Because BEAMing requires thermal cycling, an emulsifier which is thermostable can optionally be used. One such emulsifier is Abil^{®} EM90 (Degussa - Goldschmidt Chemical, Hopewell, VA). Other such emulsifiers can be used as are known in the art.

Chemical reagents can be used which selectively modify either the methylated or non-methylated form of CpG dinucleotide motifs. Alternatively, methylation-sensitive restriction endonucleases can be used to detect methylated CpG dinucleotide motifs. Such endonucleases may either impartially cleave within methylated CpG recognition sites relative to non-methylated CpG recognition sites or preferentially cleave within non-methylated relative to methylated CpG recognition sites. Examples of the former are Acc III, BstN I, and Msp I. Examples of the latter are Acc II, Ava I, BssH II, BstU I, Hpa II, and Not I.

Modified products can be detected directly, or after a further reaction which creates products which are easily distinguishable. Means which detect altered size and/or charge can be used to detect modified products, including but not limited to electrophoresis, chromatography, and mass spectrometry. Examples of such chemical reagents for selective modification include hydrazine and bisulfite ions. Hydrazine-modified DNA can be treated with piperidine to cleave it. Bisulfite ion-treated DNA can be treated with alkali.

One way to distinguish between modified and nonmodified DNA is to use oligonucleotide probes which are specific for certain products. Such probes can be hybridized directly to modified DNA or to amplification products of modified DNA. Oligonucleotide probes can be labeled using any detection system known in the art. These include but are not limited to fluorescent moieties, radioisotope labeled moieties, bioluminescent moieties, luminescent moieties, chemiluminescent moieties, enzymes, substrates, receptors, or ligands.

Test cells can be obtained from surgical samples, such as biopsies or fine needle aspirates, from paraffin embedded tissues, from a body fluid such as bone marrow, blood, serum, plasma, lymph, cerebrospinal fluid, saliva, sputum, stool, urine, or semen. Such sources are not meant to be exhaustive, but rather exemplary.

In one aspect of this embodiment, a gene can be contacted with hydrazine, which modifies cytosine residues, but not methylated cytosine residues, then the hydrazine treated gene sequence is contacted with a reagent such as piperidine, which cleaves the nucleic acid molecule at hydrazine modified cytosine residues, thereby generating a product comprising fragments. The fragments can then be used in amplification reactions and the products analyzed.

Bisulfite ions, for example, sodium bisulfite, convert non-methylated cytosine residues to bisulfite modified cytosine residues. The bisulfite ion treated gene sequence can be exposed to alkaline conditions, which convert bisulfite modified cytosine residues to uracil residues. Sodium bisulfite reacts readily with the 5,6-double bond of cytosine (but poorly with methylated cytosine) to form a sulfonated cytosine reaction intermediate that is susceptible to deamination, giving rise to a sulfonated uracil. The sulfonate group can be removed by exposure to alkaline conditions, resulting in the formation of uracil. The DNA can be amplified, for example, by PCR, and sequenced to determine whether CpG sites are methylated in the DNA of the sample. Uracil is recognized as a thymine by Taq polymerase and, upon PCR, the resultant product contains cytosine only at the position where 5-methylcytosine was present in the starting template DNA. One can compare the amount or distribution of uracil residues in the bisulfite ion treated gene sequence of the test cell with a similarly treated corresponding non-methylated gene sequence. A decrease in the amount or distribution of uracil residues in the gene from the test cell indicates methylation of cytosine residues in CpG dinucleotides in the gene of the test cell. The amount or distribution of uracil residues also can be detected by contacting the bisulfite ion treated target gene sequence, following exposure to alkaline conditions, with an oligonucleotide that selectively hybridizes to a nucleotide sequence of the target gene that either contains uracil residues or that lacks uracil residues, but not both, and detecting selective hybridization (or the absence thereof) of the oligonucleotide.

Methyl-BEAMing can quantitatively assess DNA methylation in clinical samples. In addition to its potential value as a platform for clinical diagnosis, these studies have illuminated several features with important implications for the use of DNA methylation in cancer diagnostics, regardless of the platform used. First, the correspondence between the number of methylated vimentin DNA fragments and mutant APC and PIK3CA DNA fragments in the plasma samples from cancer patients provides strong evidence that circulating methylated DNA fragments are derived from the cancer cells themselves. As DNA methylation is plastic within mammalian cells, it was possible that such circulating DNA was derived from non-neoplastic stromal or inflammatory cells adjacent to the tumor or from elsewhere in the host. As far as we know, this is the first study to provide evidence of this kind, as it requires quantitative measures of both methylation and mutation to make the argument.

Second, our results demonstrate that several methylation sites within a region of interest should be concurrently assessed to achieve the required sensitivity and specificity in a diagnostic test based on methylation. This conclusion is based on the fact that even with carefully optimized bisulfite treatments, ∼0.5% of individual cytosines are unconverted. PCR errors can further contribute to this background level³² . As less than 1 % of the DNA in some clinical samples, such as those from plasma, are derived from tumors, it is critical to be able to differentiate biologically methylated molecules from such technical artifacts.

Third, our results demonstrate that the fraction of methylated fragments derived from cancers is higher in fecal DNA than in plasma DNA. Moreover, the fraction of methylated DNA fragments derived from adenomas is sufficiently high in fecal DNA to be detected (Table 2). We did not attempt to detect circulating DNA in adenoma patients, as previous studies have shown that there is very little, if any, circulating DNA derived from pre-malignant lesions of the colon²². This result suggests that fecal DNA is the preferred analyte for colorectal cancer screening in methylation assays. However, the fraction of methylated vimentin molecules from normal individuals is substantially higher in feces than in plasma (mean fraction of 0.96% and 0.025% in feces and plasma, respectively). It is known that DNA methylation is tissue specific, and normal cells within the gastrointestinal tract presumably contribute more methylated DNA to the feces than to the circulation. This background limits the sensitivity and specificity of Methyl-BEAMing of fecal DNA by raising the threshold for positivity.

The ability to quantitatively measure the fraction of methylated molecules via Methyl-BEAMing provides several diagnostic opportunities. There is nearly a 2000-fold difference between the concentrations of methylated vimentin molecules in the plasma of advanced cancer patients compared to normal individuals (Table 1). This difference could easily be translated into simple tests for assessing the extent of disease following therapeutic procedures ³⁰. One could also imagine a prenatal test for Down's Syndrome based on the assessment of methylation of DNA fragments of chromosome 21³³. The development of such a test would require discovery of sequences that are methylated in the fetus but not in adults. Both these applications require a quantitative, rather than qualitative, assessment of DNA methylation such as afforded by Methyl-BEAMing. Though sequencing-by-synthesis instruments, such as the one employed in this study, also provide such quantification, they are not well-suited for high-throughput clinical analyses because of relatively long process times and high cost.

One of the most important applications of Methyl-BEAMing is in early diagnosis. In this study, 59 % of patients with colorectal cancer could be detected by a plasma-based Methyl-BEAMing assay. Note that this sensitivity is close to the maximal detectable with the vimentin biomarker, which has been shown to be methylated in 53-83 % of colorectal cancers²⁵. The fact that 50 % of presumably curable Duke's A and B stage cancers could be detected by this method was particularly encouraging.

Equally encouraging was the observation that 45% of patients with clinically significant pre-malignancies could be detected by Methyl-BEAMing of fecal DNA. Patients with these lesions would generally be treated by endoscopic excision without abdominal surgery. In screening an asymptomatic cohort of adults over age 50, the prevalence of colon cancers and of adenomas ≥ 1 centimeter is expected to be 0.5-1.0 % and 6.0 %, respectively^{34, 35}. Detection of 41% of cancers and 45 % of adenomas by Methyl-BEAMing of stool DNA, at 95 % specificity, would translate into a positive predictive value of 40%. This value exceeds that achieved in many studies of mammography, a cancer screening test of established clinical value in reducing mortality³⁶.

How could the Methyl-BEAMing approach be improved? As it could in principle be applied to any gene that is hypermethylated in cancers, the sensitivity for early-stage tumors could easily be raised by including one or a few additional genes in Methyl-BEAMing assays. The specificities of plasma-based and fecal-based Methyl-BEAMing assays were 95 % and 92 %, respectively. Though specificities in this range are typical of diagnostic screening tests that are widely used³⁶, the inclusion of additional genes in a methylation panel would undoubtedly lower the aggregate specificity. One challenge is therefore the identification of sequences in addition to vimentin that are methylated in tumor DNA but very rarely methylated in any normal adult cells. We have tested several commonly used methylated biomarkers in the context of selecting a biomarker that would be useful for testing in a body fluid and found most of them to be unsuitable. Hopefully, currently ongoing genome-wide epigenetic projects will identify new, more specific methylated sequences in the future^{37, 38}. Another potential enhancement would involve the combined analysis of vimentin methylation with mutations of commonly mutated genes, such as KRAS, PIK3CA and TP53, which could in theory increase sensitivity without compromising specificity.

The above disclosure generally describes the present invention. A more complete understanding can be obtained by reference to the following specific examples which are provided herein for purposes of illustration only, and are not intended to limit the scope of the invention.

### EXAMPLE 1

### Methods

Sample collection and DNA purification. Plasma samples were collected by study nurses of Indivumed GmbH, Hamburg, Germany, from surgery patients treated in hospitals of Indivumeds' collaborative network, in particular from patients at the Israelitic Hospital and Clinic Alten Eichen (both in Hamburg, Germany) following strictly controlled SOP criteria. IRB approval was provided by the Ethical Board of the Physicians Association of Hamburg and patient samples and data were collected only after informed, written consent was obtained. The samples used in the current study were randomly chosen from those contributing through this protocol. Shortly before surgery, 18 ml of blood was taken from a central catheter and loaded into a standard blood collection tube containing EDTA. The tube was immediately chilled to 8 °C and transported to the laboratories within 30 min for plasma preparation. The blood cells were pelleted for 15 min at 200 g in a Leucosep tube (Greiner, Frickenhausen, Germany) filled with 15 ml of Ficoll-Paque solution. After centrifugation, the supernatant (i.e., plasma) was transferred into 1.5 ml tubes, immediately frozen, and stored at -80 °C. The plasma samples were thawed at room temperature for 5 min, and any remaining debris was pelleted at 16,000 g for 5 min. The supernatant was transferred to a new tube. DNA was purified from the plasma supernatant using the QIAamp MinElute Virus Vacuum Kit (Qiagen) as recommended by the manufacturer. The DNA was eluted in EB buffer (Qiagen), and stored at -20 °C.

Stool samples and clinical data were collected from patients undergoing colonoscopy at Aarhus University Hospital. The study was approved by the Regional Scientific Ethics Committee, Aarhus, Denmark and by the Danish Data Protection Agency and informed, written consent was obtained in every case. Stool samples were collected on a weekday before bowel preparation for colonoscopy or operation for colorectal cancer. The patients placed the container in an insulated box containing frozen bags which had been stored in a freezer at least 24 hours. The box was collected at the patient's home within eight hours. Immediately after the stool sample arrived at Aarhus University Hospital, the container was frozen at -80 °C until it was thawed for DNA purification. Stool samples were homogenized with a stool shaker (Exact Sciences Corp, Marlborough, MA). After homogenization, a 4 g stool equivalent of each sample was subjected to 2 centrifugations (5 min at 2536 g and 10 min at 16,500 g). The supernatant was incubated with 20 µL RNase (0.5 mg/mL) for one hour at 37 °C, followed by precipitation with 1/10 volume of sodium acetate (3 M) and an equal volume of isopropanol. Stool DNA was dissolved in 4 mL of TE buffer and prepared for electrophoretic capture by mixing with 4 mL of water, 1 mL of 10× TBE (890 mM Tris base, 890 mM boric acid, 20 mM EDTA), and 1 mL of 10% SDS. DNA samples were denatured at 95 °C for 15 min and then cooled on ice for 15 min. Vimentin fragments were then purified using a Reversible Electrophoretic Capture Affinity Protocol (RECAP)³¹. This was accomplished by repeatedly driving the DNA back and forth through the capture device loaded with oligonucleotide probes for the Vimentin gene target (capture probe sequences were 5'-Biotin-CGCCACCCTCCGCAGCCATGTCCACCAGGTCCG-3' (SEQ ID NO: 4) and 5'-Biotin-GGCCATCGCCACCCTCCGCAGCCATGTCCACCAGG-3'(SEQ ID NO: 5)). After completion of electrophoretic cycling, the capture device was moved to a wash plate and washed four times with 500 µL of ST buffer (150 mM NaCl, 15 mM Tris-HCl, pH 7.4). The captured human DNA was eluted by adding 100 µL of 0.1 M NaOH to the capture device. The eluate was then neutralized with 10 µL of neutralization buffer (60 mM Tris pH 9.0, 6 mM EDTA, 0.5 M HCl).

Real-Time PCR. The amount of DNA per 2 ml plasma samples was quantified using a modified version of a human LINE-1 quantitative real-time PCR assay 39. Two primer sets were designed to amplify differently sized regions within the most abundant consensus region of the human LINE-1 family (for the 91-bp product, the primers were 5'-TGGCACATATACACCATGGAA-3'(SEQ ID NO: 6) and 5'-TGATGGTTTCCAATTTCATCC-3'(SEQ ID NO: 7); for the 69-bp product, the primers were: 5'-TGGCACATATACACCATGGAA-3' (SEQ ID NO: 8) and 5'-TGTCCCTACAAAGGATATGAACTC-3' (SEQ ID NO: 9)). PCR was performed in 25 µL reaction volumes consisting of template DNA equivalent to 25 µL of plasma, 0.5 U of Platinum Taq DNA Polymerase, 1× PCR buffer (see above), 6% (v/v) DMSO, 1 mM of each dNTP, 1:100,000 dilution of SYBR Green I (Invitrogen), and 0.2 µM of each primer. Amplification was carried out in an iCycler (Bio-Rad) using the following cycling conditions: 94 °C for 2 min; 3 cycles of 94 °C for 10 s, 67 °C for 15 s, 70 °C for 15 s; 3 cycles of 94 °C for 10 s, 64 °C for 15 s, 70 °C for 15 s; 3 cycles of 94 °C for 10 s, 61 °C for 15 s, 70 °C for 15 s; 35 cycles of 94 °C for 10 s, 58 °C for 15 s, 70 °C for 15 s. Various dilutions of normal human lymphocyte DNA were incorporated into each plate to serve as standards (0.006 to 1.56 ng). The threshold cycle number was determined using Bio-Rad analysis software. Each quantification for each LINE-1 product was performed in duplicate and the average value was used to determine the number of genome equivalents in the sample by comparison to the standards.

The amount of DNA per 4 g stool samples was quantified by real-time PCR assay using three sets of Vimentin specific primers (for the 81-bp product, the primers were 5'-GGGTGGACGTAGTCACGTAG-3 (SEQ ID NO: 10)and 5'-CCTCCTACCGCAGGATGTT-3' (SEQ ID NO: 11); for the 91-bp product, the primers were 5'-CTGTAGGTGCGGGTGGAC-3' (SEQ ID NO: 12) and 5'-CCTCCTACCGCAGGATGTT-3' (SEQ ID NO: 13); for the 100-bp product, the primers were 5'-CCTCCTACCGCAGGATGTT-3' (SEQ ID NO: 14) and 5'-CTGCCCAGGCTGTAGGTG-3' (SEQ ID NO: 15)). PCR was performed in 25 µL reaction volumes consisting of template DNA equivalent to 0.016g stool, 0.5 U of Platinum Taq DNA Polymerase, 1× PCR buffer, 6 % (v/v) DMSO, 1 mM of each dNTP, 1:100,000 dilution of SYBR Green I (Invitrogen), and 0.2 µM of each primer. Amplification was carried out in an iCycler (Bio-Rad) using the following cycling conditions: 94 °C for 2 min; 3 cycles of 94 °C for 10 s, 67 °C for 15 s, 70 °C for 15 s; 3 cycles of 94 °C for 10 s, 64 °C for 15 s, 70 °C for 15 s; 3 cycles of 94 °C for 10 s, 61 °C for 15 s, 70 °C for 15 s; 35 cycles of 94 °C for 10 s, 59 °C for 15 s, 70 °C for 15 s. Various dilutions of normal human lymphocyte DNA were incorporated in each plate to serve as standards (0.39 to 25 ng). The threshold cycle number was determined using Bio-Rad analysis software. Each quantification for each Vimentin fragment was performed in duplicate and the average value was used to determine the number of genome equivalents in the sample by comparison to the standards.

Bisulfite conversion. DNA isolated from plasma and stool was treated with bisulfite using an Epitect Bisulfite Conversion kit (Qiagen), modified for isolating degraded DNA such as that found in plasma or stool. In brief, each bisulfite conversion reaction contained 18 µl purified DNA solution, 2 µl 0.5 g/ml single-stranded sonicated salmon sperm DNA (5S DNA), 85 µl bisulfite mix and 35 µl DNA protect buffer. Bisulfite conversion was carried out in a thermocycler at 99 °C for 5 min, 60 °C for 25 min, 99 °C for 5 min, 60 °C for 85 min, 99 °C for 5 min, and 60 °C for 175 min. The cleanup of bisulfited converted DNA followed the manufacturer's recommendations for formalin-fixed, paraffin-embedded (FFPE) DNA samples with the modification that MinElute Spin Columns (Qiagen) instead of Epitect spin columns were used for DNA disulfonation and purification. Bisulfite treated DNA was eluted into EB buffer (Qiagen) and stored at -20 °C.

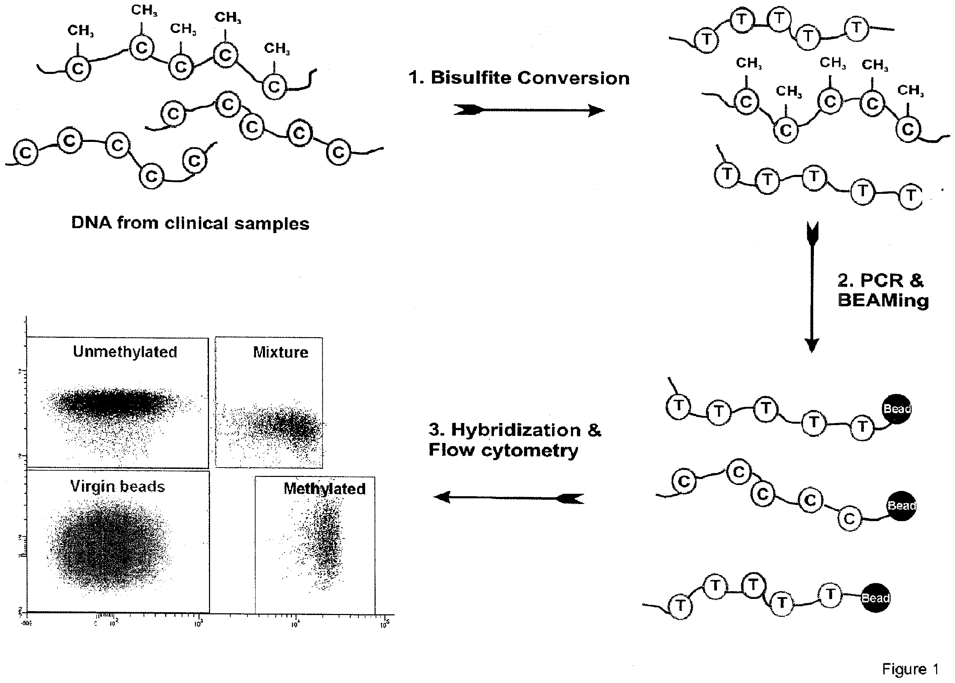

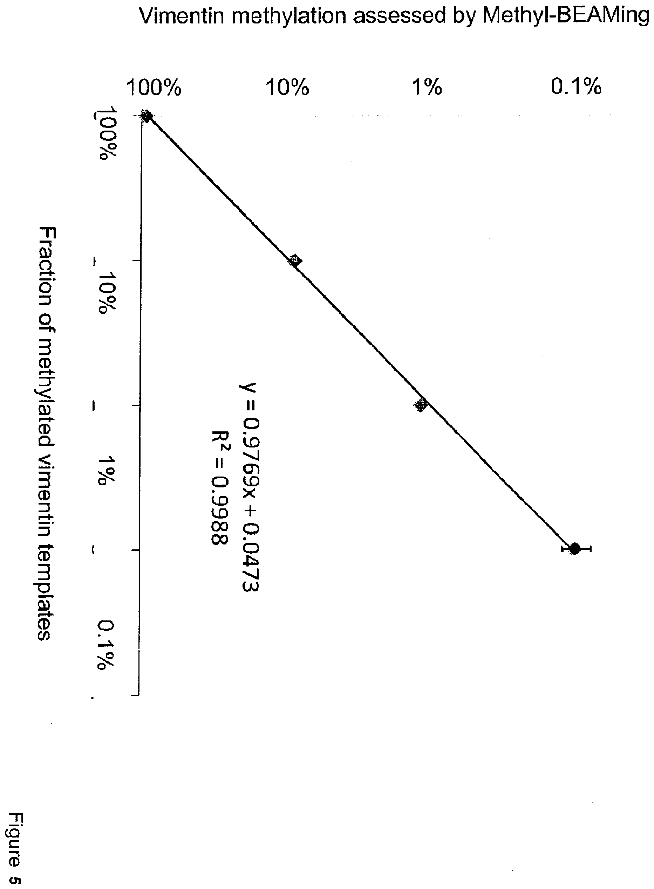

PCR amplification of bisulfite converted DNA. PCR was carried out by using a mixture of four primers that amplified both the methylated and the unmethylated Vimentin sequences (primers for methylated sequences were 5'-tcccgcgaaattaatacgacCTCGTCCTCCTACCGCAAAATATTC-3' (SEQ ID NO: 16) and 5'-GTTGTTTAGGTTGTAGGTGCGGG-3' (SEQ ID NO: 17); primers for unmethylated sequences were 5'-tcccgcgaaattaatacgacCTCATCCTCCTACCACAAAATATTC-3' (SEQ ID NO: 18) and 5'-GTTGTTTAGGTTGTAGGTGTGGG-3' (SEQ ID NO: 19)). PCR was performed in 50 µL reaction volumes consisting of 8 µl bisulfite-converted DNA, .2.5 U of AmpliTaq Gold DNA Polymerase (Applied Biosystems), 1× PCR Gold Buffer, 1mM MgCl₂, 0.2 mM dNTP mix, 0.1 uM of each primer. Amplification was carried out in a Thermo Hybrid thermocycler using the following conditions: 95 °C for 5 min; 3 cycles of 95 °C for 45 s, 67 °C for 45 s, 72 °C for 45 s; 3 cycles of 95 °C for 45 s, 64 °C for 45 s, 72 °C for 45 s; 3 cycles of 95 °C for 45 s, 61 °C for 45 s, 72 °C for 45 s; 45 cycles of 95 °C for 45 s, 58 °C for 45 s, 72 °C for 45 s; 1 cycle of 72 °C for 10 min. Note that these primers and PCR conditions result in a preferential amplification of methylated sequences ⁴⁰. This preferential amplification was confirmed using mixtures of fully methylated and fully unmethylated sequences (Fig. 5; supp, fig. 1). Values for the fraction of methylated fragments found in the plasma and stool were accordingly adjusted (*i.e*., the actual fractions of methylated Vimentin fragments determined experimentally were divided by 7). This normalization had no effect on the calculated sensitivity or specificity because all samples were adjusted equivalently.

BEAMing. Emulsion PCR was performed using modifications of the procedures described by Diehl, et al. ²⁹. We first prepared a 150 µL PCR mixture containing 15 pg template DNA, 45 U of Platinum Taq DNA polymerase (Invitrogen), 1× PCR buffer (67 mM Tris-HCl (pH 8.8), 16.6 mM (NH4)2SO4, 10 mM β-mercaptoethanol, 1.17 mM MgCl2), 0.2 mM dNTPs, 5 mM MgCl2, 0.05 µM universal forward primer (5'-tcccgcgaaattaatacgac-3' (SEQ ID NO: 20)), 8 µM Vimentin specific reverse primers (5'-GTTGTTTAGGTTGTAGGTGCGGG-3' (SEQ ID NO: 21) and 5'-GTTGTTTAGGTTGTAGGTGTGGG-3' (SEQ ID NO: 22)) and 6×107 magnetic streptavidin beads (MyOne, Invitrogen) coated with universal forward primer (5'-dual biotin-T-Spacer18-tcccgcgaaattaatacgac-3' (SEQ ID NO: 23)). This PCR reaction mixture was combined with 600 µL of an oil/emulsifier mix (7% ABIL WE09, 20% mineral oil, 73% Tegosoft DEC; Degussa Goldschmidt Chemical, Hopewell, VA) and one 5 mm steel bead (Qiagen) in one well of a 96 deep-well plate (Abgene). Emulsions were prepared by shaking the plate in a TissueLyser (Qiagen) for 10 s at 15 Hz and 7 s at 17 Hz and dispensed into eight wells of a 96 well PCR plate. Temperature cycling was performed at 94 °C for 2 min; 3 cycles of 94 °C for 10 s, 68 °C for 45 s, 70 °C for 75 s; 3 cycles of 94 °C for 10 s, 65 °C for 45 s, 70 °C for 75 s; 3 cycles of 94 °C for 10 s, 62 °C for 45 s, 70 °C for 75 s; 50 cycles of 94 °C for 10 s, 59 °C for 45 s, 70 °C for 75 s.

To break the emulsions, 150 µL breaking buffer (10 mM Tris-HCl, pH 7.5, 1% Triton-X 100, 1% SDS, 100 mM NaCl, 1 mM EDTA) was added to each well and mixed in a TissueLyser at 20 Hz for 20 s. Beads were recovered by centrifuging the suspension at 3200 g for 2 min and removing the oil phase. The breaking step was repeated twice. All beads from eight PCR wells were combined and resuspended in 100 µL wash buffer (20 mM Tris-HCl, pH 8.4, 50 mM KCl). The DNA on the beads was denatured for 5 min with 0.1 M NaOH. Finally, beads were washed with 100 µL wash buffer and resuspended in 100 µL of wash buffer.

Probe hybridization. To determine the fraction of beads containing PCR products from methylated DNA templates, 0.5 uM Cy5-labeled oligonucleotide (5'-Cy5-TCGGTCGGTTCGCGGTGTTCGA-3' (SEQ ID NO: 24), complimentary to the bisulfite converted methylated sequence), and 0.5 uM FITC labeled oligonucleotide (5'-FAM-TTGGTTGGTTTGTGGTGTTTGA-3' (SEQ ID NO: 25), complimentary to the bisulfite converted unmethylated sequence) were hybridized to the DNA on ∼107 beads in 100 µl hybridization buffer (30 mM Tris-HCl, pH 9.5, 13.4 mM MgCl2, 5% formamide) at 50 °C for 15 minutes. Beads were then collected with a magnet and resuspended in 200 µl 10 mM Tris, pH 7.5, 1 mM EDTA, pH 7.5.

Flow Cytometry. Beads were analyzed with a LSR II flow cytometer and data were analyzed with FACSDiva software (BD Biosciences). The flow rate was typically set at 5,000-10,000 events per second. Events were gated to exclude doublets and aggregates.

Massively parallel sequencing-by-synthesis. DNA isolated from normal lymphocytes (control) and stool was treated with bisulfite as described above. The converted DNA was PCR amplified using the same protocol as used for Methyl-BEAMing. The PCR product was purified with a QIAquick PCR purification column (Qiagen) and used to construct libraries for Solexa sequencing following Illumina's standard DNA sample preparation instructions. Briefly, (1) the PCR products were blunt-ended with T4 DNA polymerase, Klenow polymerase, and T4 polynucleotide kinase; (2) a dA was added to the 3' end of each strand with Klenow (exo-) polymerase; (3) adapters designed for library construction were ligated to the PCR fragments; (4) ligation products were gel-purified to select for ∼180bp fragments; and (5) PCR amplification was performed to enrich ligated fragments. The amplified library was quantified by real-time PCR and was denatured with 0.1 M NaOH to generate single-stranded DNA molecules, captured on Illumina flow cells, and amplified in situ. A Vimentin-specific oligonucleotide (5-GTGC/TGGGTGGAC/TGTAGTTAC/TGTAGT TTC/TGGTTGGA-3 (SEQ ID NO: 26), C/T indicates that the position was an equimolar mix of cytosine and thymidine) was used to prime sequencing for 36 cycles on an Illumina Genome Analyzer. Data analysis: The sequences generated were aligned to the bisulfite converted unmethylated Vimentin sequence. The following criterions were used to filter the tags for further analysis. Each tag was required (i) to pass the Illumina chastity filter; (ii) to have an average Phred sequence quality score above 20; and (iii) to have perfect match to the reference sequence at A,G, and T positions (allowing mismatched at C positions). At least 80,000 tags that met these criteria were evaluated in each case.

### EXAMPLE 2

### Technical overview of Methyl-BEAMing

The first hurdle to overcome in developing Methyl-BEAMing was bisulfite conversion. DNA in clinical samples such as plasma is already degraded to small size by circulating nucleases and is present at only a few nanograms per mililiter²². Conventional methods for bisulfite conversion further degrade DNA and are difficult to implement with samples containing only small amounts of DNA because of cumulative losses during the procedure^{23, 24}. After much optimization, we identified conditions that resulted in nearly complete (Mean + SEM: 99.4% + 0.4%) conversion of dC to dU residues while preserving the majority of the initial DNA in a fashion permitting subsequent PCR amplification (see Experimental Methods for details).

The exon 1 region of the vimentin gene has been shown to be hypermethylated in colorectal cancers when compared to normal colorectal mucosae and other normal tissues^{25, 26}. Moreover, this difference is the basis for the only commercially available diagnostic test based on DNA methylation (ColoSure, LabCorp). We therefore chose this region of vimentin to assess the dynamic range and accuracy of Methyl-BEAMing. The region of vimentin queried contains 5'- CpG sites that have been shown to be methylated in cancer cells. Primers surrounding this region were designed to amplify it, whether it was methylated or unmethylated, following bisulfite conversion. The PCR products were only ∼100 bp in length so as to accommodate the small size of circulating DNA molecules^{22, 27}. These amplicons were then used for BEAMing (Beads, Emulsion, Amplification and Magnetics)^{28, 29}.

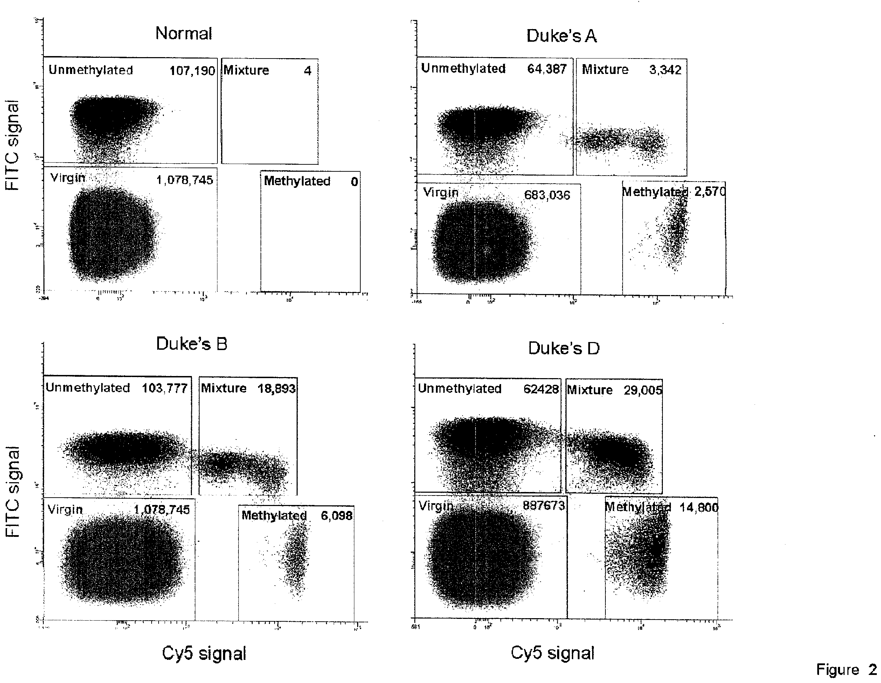

BEAMing employs aqueous nanocompartments suspended in a continuous oil phase (Fig. 1). Each aqueous nanocompartment contains DNA polymerase, cofactors, and dNTP's required for PCR. When a compartment contains a single DNA template molecule as well as a bead, the PCR product within the compartment becomes bound to the bead. Each bead thereby ends up with thousands of identical copies of the template within its nanocompartment - a process similar to that resulting from cloning an individual DNA fragment into a plasmid vector to form a bacterial colony. After PCR, the beads are collected by breaking the emulsion and incubated with fluorescent probes that specifically hybridize to either methylated or unmethylated sequences. Flow cytometry then provides an accurate read-out of the fraction of original template molecules that were methylated or unmethylated within the queried sequence (examples in Fig. 2).

As a prelude to the experiments reported below, we tested Methyl-BEAMing on mixtures of templates representing DNA from peripheral blood lymphocytes (unmethylated vimentin) and a colorectal cancer cell line (fully methylated vimentin). We found that the fraction of beads containing methylated vimentin sequences was directly proportional to the fraction of methylated input DNA when the latter represented 0.1% to 100% of the input DNA (R2=1.00, Fig. 5; supp. fig. 1).

### EXAMPLE 3

### Methylated vimentin gene fragments in the circulation come from cancer cells

Some of the most important uses of cancer biomarkers involve the assessment of circulating molecules, either for early detection or disease-monitoring following therapy. To use methyl-BEAMing for such purposes, or indeed to use any test based on DNA methylation, it is assumed that the methylated molecules in the circulation emanate from cancer cells. To explicitly test this assumption, we compared the fraction of methylated vimentin molecules with the fraction of mutant molecules (APC G4189T and PIK3CA G1624A) in the plasma of two colorectal cancer patients. In these patients, the mutations were somatic and therefore exclusively present in the patient's neoplastic cells. Methyl-BEAMing was used to assess methylation and standard BEAMing was used to assess mutations³⁰. The fraction of methylated vimentin templates in the first sample was 13.6%, quite similar to the fraction of mutated APC (17.5%). In the second patient's plasma, methylated vimentin represented 3.5% of the total vimentin templates while mutated PIK3CA represented 3.0% of the PIK3CA templates.

### EXAMPLE 4

### Methyl-BEAMing of circulating DNA

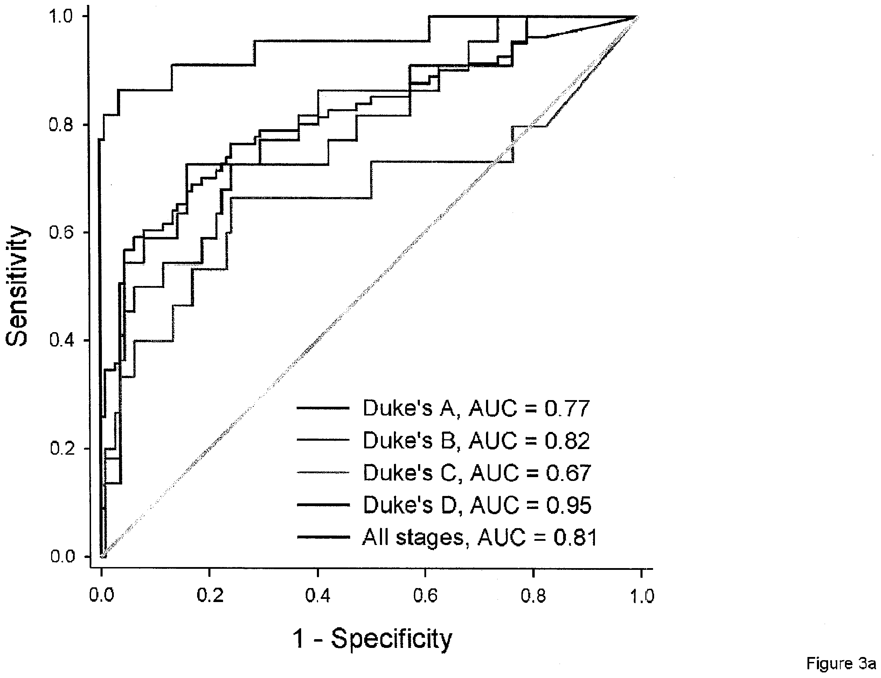

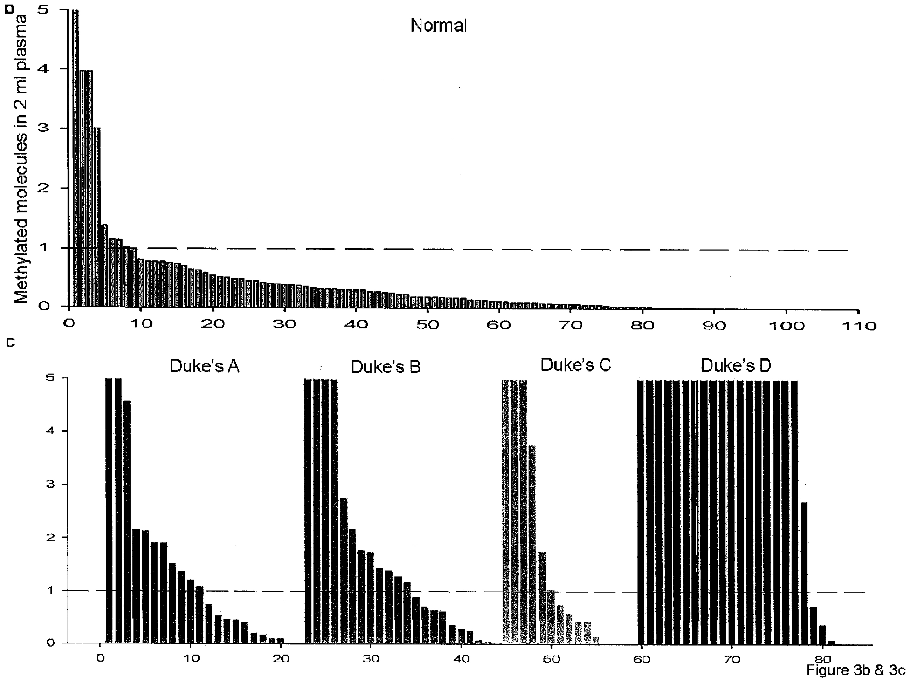

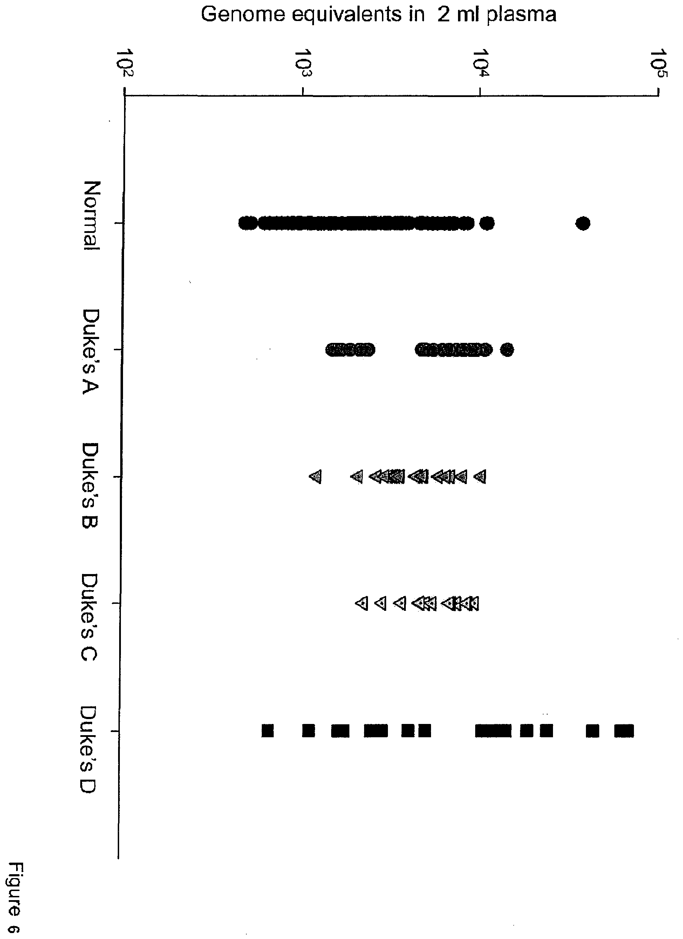

To determine the sensitivity and specificity of Methyl-BEAMing in plasma samples, we evaluated 191 samples, 81 from patients with colorectal cancer and 110 from age- and sex-matched controls. The total amount of DNA in the plasma was somewhat higher in patients with cancer than in the cancer-free controls, as noted previously12 (Fig. 6; supp. fig. 2). The average fraction of methylated vimentin fragments in these patients varied greatly but in a specific way. It was very low in the normal controls with a mean of 0.026% and gradually increased with cancer stage up to a mean of 4.4% in the most advanced disease (Duke's D; Table 1). The theoretical limit of detection in any digital assay is one event, *i.e*., in the current study, the limit of detection was one methylated vimentin fragment in the 2 ml plasma that was assayed for each patient. Using this cutoff, the sensitivity of Methyl-BEAMing was 59% in cancer patients, and the specificity was 93% (eight of the 110 normal samples contained ≥ 1 methylated vimentin fragment per 2 ml plasma, Table 1, Fig. 3). The Area under the Receiver Operating Characteristic Curve (AUC) of this assay in cancers was 0.81, and varied from 0.67 to 0.95 among the four cancer stages (Fig. 3a). As expected, the absolute number of methylated vimentin fragments in plasma increased with tumor stage, ranging from a mean of 1.8 in Duke's A patients with detectable circulating methylated vimentin to 1195 in Duke's D patients (Table 1 and Fig. 3c). Importantly, the sensitivity for detecting colorectal cancer was ∼50% in the patients with Duke's A and B cancers, nearly all of which were likely to be curable by conventional surgery (Table 1 and Fig. 3c).

**Table 1 Methylated vimentin DNA fragments in plasma**

| **Sample Type** | **Number of Patients** | **Total vimentin fragments (methylated plus unmethylated) (mean ± SEM)** | **Fraction of methylated fragments (mean ± SEM)** | **# of Vimentin methylated fragments in 2 ml plasma (mean ± SEM)** | **# of samples with ≥ 1.00 methylated fragment in 2 ml plasma (fraction in parentheses)** |
|---|---|---|---|---|---|
| Normal | 110 | 3170 ± 380 | 0.026% ± 0.013% | 0.60 ± 0.27 | 8 (7%) |
| Colorectal cancers (all stages) | 81 | 8240 ± 1180 | 1.4% ± 0.40% | 334.6 ± 177.2 | 48 (59%) |
| Duke's A | 22 | 5830 ± 781 | 0.052% ± 0.025% | 1.8 ± 0.54 | 11 (50%) |
| Duke's B | 22 | 4560 ± 458 | 0.31% ± 0.23% | 8.9 ± 5.3 | 12 (55%) |
| Duke's C | 15 | 6530 ± 632 | 0.76% ± 0.65% | 38.4 ± 31.6 | 6 (40%) |
| Duke's D | 22 | 15,500 ± 4270 | 4.4% ± 1.2% | 1195 ± 626 | 19 (86%) |

### EXAMPLE 5

### Methyl-BEAMing of fecal DNA

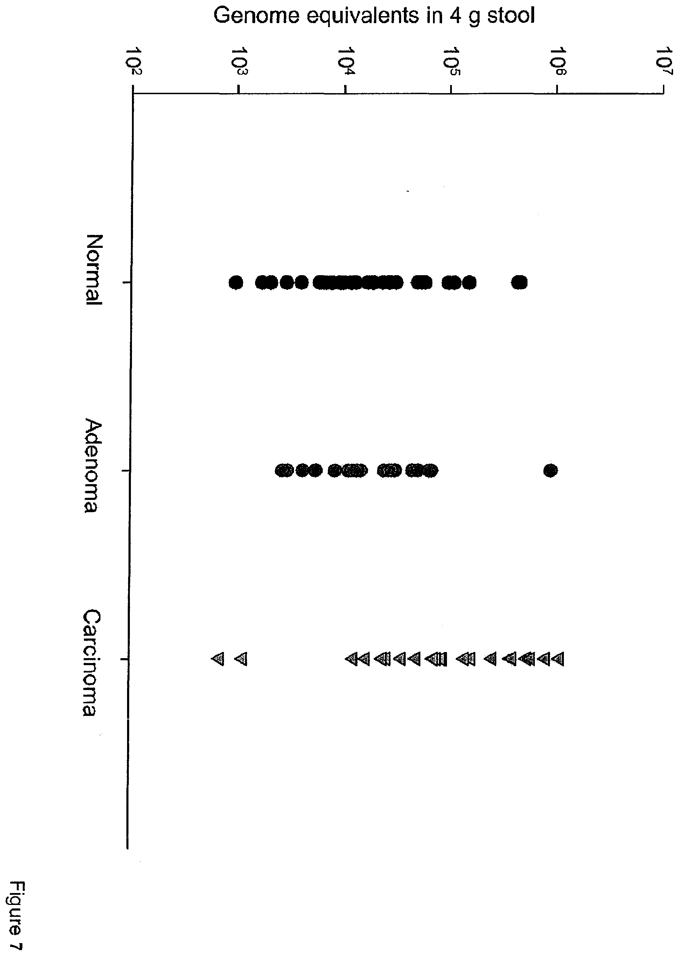

Next, we used Vimentin Methyl-BEAMing to screen 80 stool samples, including 38 from normal individuals, 20 from patients with > 1 centimeter adenomas, and 22 from colorectal cancer patients of various stages. In each case, we homogenized 4 g of stool and captured vimentin fragments through electrophoresis-driven hybridization to oligonucleotides on a solid phase matrix 31. The concentration of vimentin DNA fragments in stool varied widely, as expected for samples from individuals with various diets and bowel habits (Fig. 7; supp. fig. 3). Though there were no reproducible differences between the concentrations of total vimentin DNA fragments in the three patient groups, there was a significant difference in the fraction of methylated fragments. This fraction increased from a mean of 0.96% in normal individuals to 3.8% and 7.3% in patients with adenomas or carcinomas, respectively (Table 2). Using a threshold of 2% methylation, 45% and 41% of the patients with adenomas and carcinomas, respectively, scored positive in the Methyl-BEAMing assay, while only 5% of the samples from normal individuals did so (Table 2, Fig. 4). The corresponding areas under the ROC curves were 0.69 and 0.62 for adenomas and carcinomas (Fig. 4).

**Table 2 Methylated vimentin DNA fragments in fecal DNA**

| **Sample type** | **Number of Patients** | **Total vimentin fragments (methylated plus unmethylated) (mean ± SEM)** | **Fraction of methylated fragments (mean ± SEM)** | **# of samples with ≥ 2.0% methylated fragment in 4 g stool (fraction in parentheses)** |
|---|---|---|---|---|
| Normal | 38 | 47342.3 ± 16731.6 | 0.96% ± 0.25% | 2 (5.3%) |
| Adenoma | 20 | 69588.8 ± 45185.7 | 3.83% ± 0.86% | 9 (45%) |
| Colorectal cancer (mixed stages) | 22 | 236182.6 ± 66576.8 | 7.30% ± 3.28% | 9 (41%) |

### EXAMPLE 6

### Validation of Methyl-BEAMing through massively parallel sequencing

To further validate the accuracy of Methyl-BEAMing for the quantification of methylated vimetin fragments in clinical samples, we evaluated samples with low and high fractions of methylated fragments on the Illumina Genome Analyzer sequencing instrument. Following bisulfite conversion and PCR amplification (Fig. 1), the PCR products were ligated to adapters and sequenced using a standard Illumina protocol. The 36-base region sequenced encompassed the five core CpG's that were assessed with the hybridization probes employed in Methyl-BEAMing. Through the analysis of C residues not present within CpG motifs from normal lymphocyte DNA, we found that the bisulfite conversion efficiency used for Methyl-BEAMing was ∼99.4%. In this lymphocyte sample, the fraction of sequenced fragments in which four or five of the five core CpG sites were methylated was 0.015%. This fraction was determined to be 0.018% in the Methyl-BEAMing assay. Likewise, the fraction of methylated fragments in a fecal DNA sample with a high degree of methylation was similar when assessed by either sequencing or Methyl-BEAMing (11.3% vs. 10.8%, respectively).
1. Feinberg, A.P. & Vogelstein, B. Hypomethylation distinguishes genes of some human cancers from their normal counterparts. Nature 301, 89-92 (1983).
2. Feinberg, A.P. & Tycko, B. The history of cancer epigenetics. Nat Rev Cancer 4, 143-153 (2004).
3. Herman, J.G. & Baylin, S.B. Gene silencing in cancer in association with promoter hypermethylation. N Engl J Med 349, 2042-2054 (2003).
4. Jones, P.A. & Baylin, S.B. The epigenomics of cancer. Cell 128, 683-692 (2007).
5. Esteller, M., Corn, P.G., Baylin, S.B. & Herman, J.G. A gene hypermethylation profile of human cancer. Cancer Res 61, 3225-3229 (2001).
6. Laird, P.W. The power and the promise of DNA methylation markers. Nat Rev Cancer 3, 253-266 (2003).
7. Grady, W.M., Rajput, A., Lutterbaugh, J.D. & Markowitz, S.D. Detection of aberrantly methylated hMLH1 promoter DNA in the serum of patients with microsatellite unstable colon cancer. Cancer Res 61, 900-902 (2001).
8. Kawakami, K. et al. Hypermethylated APC DNA in plasma and prognosis of patients with esophageal adenocarcinoma. J Natl Cancer Inst 92, 1805-1811 (2000).
9. Wong, I.H., Zhang, J., Lai, P.B., Lau, W.Y. & Lo, Y.M. Quantitative analysis of tumor-derived methylated p16INK4a sequences in plasma, serum, and blood cells of hepatocellular carcinoma patients. Clin Cancer Res 9, 1047-1052 (2003).
10. Hoque, M.O. et al. Quantitation of promoter methylation of multiple genes in urine DNA and bladder cancer detection. J Natl Cancer Inst 98, 996-1004 (2006).
11. Machida, E.O. et al. Hypermethylation of ASC/TMS1 is a sputum marker for late-stage lung cancer. Cancer Res 66, 6210-6218 (2006).
12. Fleischhacker, M. & Schmidt, B. Circulating nucleic acids (CNAs) and cancer--a survey. Biochim Biophys Acta 1775, 181-232 (2007).
13. Lofton-Day, C. et al. DNA methylation biomarkers for blood-based colorectal cancer screening. Clin Chem 54, 414-423 (2008).
14. Herman, J.G., Graff, J.R., Myohanen, S., Nelkin, B.D. & Baylin, S.B. Methylation-specific PCR: a novel PCR assay for methylation status of CpG islands. Proc Natl Acad Sci U S A 93, 9821-9826 (1996).
15. Xiong, Z. & Laird, P.W. COBRA: a sensitive and quantitative DNA methylation assay. Nucleic Acids Res 25, 2532-2534 (1997).
16. Gonzalgo, M.L. & Jones, P.A. Rapid quantitation of methylation differences at specific sites using methylation-sensitive single nucleotide primer extension (Ms-SNuPE). Nucleic Acids Res 25, 2529-2531 (1997).
17. Lo, Y.M. et al. Quantitative analysis of aberrant p 16 methylation using real-time quantitative methylation-specific polymerase chain reaction. Cancer Res 59, 3899-3903 (1999).
18. Eads, C.A. et al. MethyLight: a high-throughput assay to measure DNA methylation. Nucleic Acids Res 28, E32 (2000).
19. Sidransky, D. Emerging molecular markers of cancer. Nat Rev Cancer 2, 210-219 (2002).
20. Korshunova, Y. et al. Massively parallel bisulphite pyrosequencing reveals the molecular complexity of breast cancer-associated cytosine-methylation patterns obtained from tissue and serum DNA. Genome Res 18, 19-29 (2008).
21. Weisenberger, D.J. et al. DNA methylation analysis by digital bisulfite genomic sequencing and digital MethyLight. Nucleic Acids Res 36, 4689-4698 (2008).
22. Diehl, F. et al. Detection and quantification of mutations in the plasma of patients with colorectal tumors. Proc Natl Acad Sci U S A 102, 16368-16373 (2005).
23. Grunau, C., Clark, S.J. & Rosenthal, A. Bisulfite genomic sequencing: systematic investigation of critical experimental parameters. Nucleic Acids Res 29, E65-65 (2001).
24. Okamoto, A. Chemical approach toward efficient DNA methylation analysis. Org Biomol Chem 7, 21-26 (2009).
25. Chen, W.D. et al. Detection in fecal DNA of colon cancer-specific methylation of the nonexpressed vimentin gene. J Natl Cancer Inst 97, 1124-1132 (2005).
26. Zou, H. et al. Highly methylated genes in colorectal neoplasia: implications for screening. Cancer Epidemiol Biomarkers Prev 16, 2686-2696 (2007).
27. Jahr, S. et al. DNA fragments in the blood plasma of cancer patients: quantitations and evidence for their origin from apoptotic and necrotic cells. Cancer Res 61, 1659-1665 (2001).
28. Dressman, D., Yan, H., Traverso, G., Kinzler, K.W. & Vogelstein, B. Transforming single DNA molecules into fluorescent magnetic particles for detection and enumeration of genetic variations. Proc Natl Acad Sci U S A 100, 8817-8822 (2003).
29. Diehl, F. et al. BEAMing: single-molecule PCR on microparticles in water-in-oil emulsions. Nat Methods 3, 551-559 (2006).
30. Diehl, F. et al. Circulating mutant DNA to assess tumor dynamics. Nat Med 14, 985-990 (2008).
31. Kent Moore, J., Smith, J.A., Whitney, D.H., Durkee, K.H. & Shuber, A.P. An electrophoretic capture method for efficient recovery of rare sequences from heterogeneous DNA. Biotechniques 44, 363-374 (2008).
32. Li, M., Diehl, F., Dressman, D., Vogelstein, B. & Kinzler, K.W. BEAMing up for detection and quantification of rare sequence variants. Nat Methods 3, 95-97 (2006).
33. Lo, Y.M. Noninvasive prenatal detection of fetal chromosomal aneuploidies by maternal plasma nucleic acid analysis: a review of the current state of the art. Bjog 116, 152-157 (2009).
34. Lieberman, D.A. et al. Use of colonoscopy to screen asymptomatic adults for colorectal cancer. Veterans Affairs Cooperative Study Group 380. N Engl J Med 343, 162-168 (2000).
35. Kahi, C.J., Rex, D.K. & Imperiale, T.F. Screening, surveillance, and primary prevention for colorectal cancer: a review of the recent literature. Gastroenterology 135, 380-399 (2008).
36. Humphrey, L.L., Helfand, M., Chan, B.K. & Woolf, S.H. Breast cancer screening: a summary of the evidence for the U.S. Preventive Services Task Force. Ann Intern Med 137, 347-360 (2002).
37. Esteller, M. Cancer epigenomics: DNA methylomes and histone-modification maps. Nat Rev Genet 8, 286-298 (2007).
38. Moving AHEAD with an international human epigenome project. Nature 454, 711-715 (2008).
39. Rago, C. et al. Serial assessment of human tumor burdens in mice by the analysis of circulating DNA. Cancer Res 67, 9364-9370 (2007).
40. Warnecke, P.M. et al. Detection and measurement of PCR bias in quantitative methylation analysis of bisulphite-treated DNA. Nucleic Acids Res 25, 4422-4426 (1997).

## Claims

1. A method for determining fraction of molecules comprising a methylated sequence in a sample of analyte DNA molecules comprising the sequence, comprising:
treating a sample of analyte DNA molecules from plasma or stool with bisulfite ions to selectively modify unmethylated cytosine residues, without degrading the majority of the analyte DNA molecules, wherein the sample is from a human who is being screened for or suspected of having a colon adenoma or colon cancer;
forming microemulsions comprising the treated analyte DNA molecules; amplifying a portion of a treated analyte DNA molecule in the microemulsions in the presence of beads, wherein said portion comprises one or more 5 '-CpG methylation sites that are hypermethylated in cancer cells, wherein the beads are bound to a plurality of molecules of a primer for amplifying the analyte DNA molecules, whereby a plurality of copies of analyte DNA molecule are formed covalently attached to the plurality of molecules of the primer which are bound to beads;
determining nucleotide sequences at the one or more 5 '-CpG methylation sites of analyte DNA molecules which are bound to beads and quantitating beads that have modified 5'-CpG methylation sites and beads that have unmodified 5'-CpG methylation sites.

2. The method of claim 1 wherein:
(1) the amplified portion of analyte DNA molecule comprises at least two 5 '-CpG sites; or
(2) the amplified portion of analyte DNA molecule comprises at least three 5'-CpG sites.

3. The method of claim 1 further comprising the step of isolating beads which are bound to modified analyte DNA molecule from beads which are bound to unmodified analyte DNA molecule, wherein optionally the step of isolating is performed using fluorescence activated cell sorting.

4. The method of claim 1 wherein prior to the step of determining, the microemulsions are broken by addition of one or more detergents.

5. The method of claim 1 wherein the step of determining is performed by:
(1) hybridization to oligonucleotide probes which are differentially labeled;
(2) nucleotide sequencing; or
(3) primer extension.

6. The method of claim 1 wherein the analyte DNA molecules are genomic DNA.

7. The method of claim 1 wherein
the bisulfite ion-treated sample is exposed to
alkaline conditions, whereby modified cytosine residues are converted to uracil residues.

8. The method of claim 1 wherein the sample is from:
a patient who previously was treated to remove or ablate a cancer.

9. The method of claim 1 wherein the amplified DNA comprises:
(1) a portion of a vimentin gene;
(2) a bisulfite-converted sequence derived from all or at least 15 contiguous nucleotides of
(3) a converted sequence derived from 5'-CTCGTCCTCCT ACCGCAGGAT GTTCGGCGGC CCGGGCACCG CGAGCCGGCC GAGCTCCAGC CGGAGCTACG TGACTACGTC CACCCGCACC TACAGCCTGG GCAGC- 3 ' (SEQ ID NO: 1), in which the converted sequence is derived by replacing each of one or more unmethylated C bases with a T base;
(4) a bisulfate-converted sequence derived from all or at least 15 contiguous nucleotides the complement of SEQ ID NO: 1, 5'-GAGCAGGAGGATGGCGTCCTACAAGCCGCCGGGCCCGTGGCGCTCGGCCGG CTCGA GGTCGGCCTCGA TGCACTGA TGCAGGTGGGCGTGGA TGTCGGACCCGTCG -3' (SEQ ID NO: 28); or
(5) a converted sequence derived from all or at least 15 contiguous nucleotides of the complement of SEQ ID NO: 1 in which the converted sequence is derived by replacing each of one or more unmethylated C bases with a T base.

10. The method of claim 1 wherein the amplified DNA comprises

11. The method of claim 1 wherein:
(1) the primer for amplifying the analyte DNA molecules is selected from the group consisting of 5'-GTTGTTTAGG TTGTAGGTGN GGG-3 (SEQ ID NO: 2) and 5'-CTCNTCCTCC TACCNCAAAA TATTC-3'(SEQ ID NO: 3); or
(2) the primer for amplifying the analyte DNA molecules comprises at least 15 contiguous nucleotides selected from the group consisting of 5'-GTTGTTTAGG TTGTAGGTGN GGG-3 (SEQ ID NO: 2) and 5'-CTCNTCCTCC TACCNCAAAA TATTC-3' (SEQ ID NO: 3).

12. The method of claim 1 wherein nucleotide sequences are determined by hybridization to fluorescent probes.

13. The method of claim 1 wherein the amplified portion of analyte DNA comprises between 50 and 200 nucleotides or between 75 and 125 nucleotides.

14. The method of claim 1 further comprising testing for the presence of one or more mutations in a tumor suppressor or oncogene in the sample.

15. The method of claim 11 wherein:
(1) the primer is covalently bound to the bead;
(2) the primer is non covalently bound to the bead; or
(3) the bead is superparamagnetic.

16. The method of claim 1 where between 99 and 99.8% of the unmethylated cytosine residues are modified.

17. The method of claim 1 wherein the majority of the treated analyte DNA molecules can be amplified by said primers.

## Patentansprüche

1. Verfahren zum Bestimmen von Molekülfraktionen, die eine methylierte Sequenz umfassen, in einer Probe von Analyt-DNA-Molekülen, die die Sequenz umfassen, umfassend:
Behandeln einer Probe von Analyt-DNA-Molekülen aus Plasma oder Stuhl mit Bisulfit-Ionen, um unmethylierte Cytosinreste selektiv zu modifizieren, ohne den Großteil der Analyt-DNA-Moleküle abzubauen, wobei die Probe von einem Menschen ist, der auf ein Kolonadenom oder Kolonkarzinom gescreent wird oder bei dem Verdacht hierauf besteht;
Bilden von Mikroemulsionen, die die behandelten Analyt-DNA-Moleküle umfassen;
Amplifizieren eines Teils eines behandelten Analyt-DNA-Moleküls in den Mikroemulsionen in Gegenwart von Beads, wobei der Teil ein oder mehrere 5'-CpG-Methylierungsstellen umfasst, die in Krebszellen hypermethyliert sind, wobei die Beads zum Amplifizieren der Analyt-DNA-Moleküle an eine Vielzahl von Molekülen eines Primers gebunden sind, wodurch kovalent an die Vielzahl von Molekülen des Primers gebunden eine Vielzahl von Analyt-DNA-Molekül-Kopien gebildet werden, die an Beads gebunden sind;
Bestimmen von Nukleotidsequenzen an den ein oder mehreren 5'-CpG-Methylierungsstellen von Analyt-DNA-Molekülen, die an Beads gebunden sind, und Quantifizieren von Beads, die modifizierte 5'-CpG-Methylierungsstellen haben, und von Beads, die unmodifizierte 5'-CpG-Methylierungsstellen haben.

2. Verfahren nach Anspruch 1, wobei:
(1) der amplifizierte Analyt-DNA-Molekül-Teil wenigstens zwei 5'-CpG-Methylierungsstellen umfasst; oder
(2) der amplifizierte Analyt-DNA-Molekül-Teil wenigstens drei 5'-CpG-Methylierungsstellen umfasst.

3. Verfahren nach Anspruch 1, weiterhin umfassend den Schritt des Isolierens von Beads, die an modifiziertes Analyt-DNA-Molekül gebunden sind, von Beads, die an unmodifiziertes Analyt-DNA-Molekül gebunden sind, wobei der Isolierschritt gegebenenfalls durch fluoreszenzaktivierte Zellsortierung erfolgt.

4. Verfahren nach Anspruch 1, wobei die Mikroemulsionen vor dem Bestimmungsschritt durch Zugabe von ein oder mehreren Detergenzien gebrochen werden.

5. Verfahren nach Anspruch 1, wobei der Bestimmungsschritt erfolgt durch:
(1) Hybridisierung an Oligonukleotidsonden, die unterschiedlich markiert sind;
(2) Nukleotidsequenzierung; oder
(3) Primer-Extension.

6. Verfahren nach Anspruch 1, wobei die Analyt-DNA-Moleküle genomische DNA sind.

7. Verfahren nach Anspruch 1, wobei die mit Bisulfit-Ionen behandelte Probe alkalischen Bedingungen ausgesetzt wird, wodurch modifizierte Cytosinreste in Uracilreste umgewandelt werden.

8. Verfahren nach Anspruch 1, wobei die Probe von einem Patienten ist, der zuvor zur Entfernung oder Ablation eines Karzinoms behandelt wurde.

9. Verfahren nach Anspruch 1, wobei die amplifizierte DNA umfasst:
(1) einen Teil eines Vimentin-Gens;
(2) eine durch Bisulfit umgewandelte Sequenz, die sich ableitet von allen oder wenigstens 15 aufeinanderfolgenden Nukleotiden von
(3) eine umgewandelte Sequenz, die sich ableitet von 5'-CTCGTCCTCCT ACCGCAGGAT GTTCGGCGGC CCGGGCACCG CGAGCCGGCC GAGCTCCAGC CGGAGCTACG TGACTACGTC CACCCGCACC TACAGCCTGG GCAGC-3' (SEQ ID NO: 1), in der sich die umgewandelte Sequenz durch Austauschen einer jeden von ein oder mehreren unmethylierten C-Basen durch eine T-Base ableitet;
(4) eine durch Bisulfit umgewandelte Sequenz, die sich ableitet von allen oder wenigstens 15 aufeinanderfolgenden Nukleotiden des Komplementärstrangs von (SEQ ID NO: 1), 5'-GAGCAGGAGGATGGCGTCCTACAAGCCGCCGGGCCCGTGGCGCTC GGCCGGCTCGA GGTCGGCCTCGA TGCACTGA TGCAGGTGGGCGTGGA TGTCGGACCCGTCG-3' (SEQ ID NO: 28); oder
(5) eine umgewandelte Sequenz, die sich ableitet von allen oder wenigstens 15 aufeinanderfolgenden Nukleotiden des Komplementärstrangs von (SEQ ID NO: 1), in der sich die umgewandelte Sequenz durch Austauschen einer jeden von ein oder mehreren unmethylierten C-Basen durch eine T-Base ableitet.

10. Verfahren nach Anspruch 1, wobei die amplifizierte DNA umfasst:

11. Verfahren nach Anspruch 1, wobei:
(1) der Primer zum Amplifizieren der Analyt-DNA-Moleküle ausgewählt ist aus der Gruppe bestehend aus 5'-GTTGTTTAGG TTGTAGGTGN GGG-3' (SEQ ID NO: 2) und 5'-CTCNTCCTCC TACCNCAAAA TATTC-3' (SEQ ID NO: 3); oder
(2) der Primer zum Amplifizieren der Analyt-DNA-Moleküle wenigstens 15 aufeinanderfolgende Nukleotide umfasst, ausgewählt aus der Gruppe bestehend aus 5'-GTTGTTTAGG TTGTAGGTGN GGG-3' (SEQ ID NO: 2) und 5'-CTCNTCCTCC TACCNCAAAA TATTC-3' (SEQ ID NO: 3).

12. Verfahren nach Anspruch 1, wobei Nukleotidsequenzen durch Hybridisierung an Fluoreszenzsonden bestimmt werden.

13. Verfahren nach Anspruch 1, wobei der amplifizierte Analyt-DNA-Teil zwischen 50 und 200 Nukleotide oder zwischen 75 und 125 Nukleotide umfasst.

14. Verfahren nach Anspruch 1, weiterhin umfassend Testen auf Vorliegen ein oder mehrerer Mutationen in einem Tumorsuppressor oder Onkogen in der Probe.

15. Verfahren nach Anspruch 11, wobei:
(1) der Primer kovalent an das Bead gebunden ist;
(2) der Primer nicht kovalent an das Bead gebunden ist; oder
(3) das Bead superparamagnetisch ist.

16. Verfahren nach Anspruch 1, wobei zwischen 99 und 99,8 % der unmethylierten Cytosinreste modifiziert werden.

17. Verfahren nach Anspruch 1, wobei der Großteil der behandelten Analyt-DNA-Moleküle durch die Primer amplifiziert werden kann.

## Revendications

1. Procédé de détermination de la fraction de molécules comprenant une séquence méthylée dans un échantillon de molécules d'ADN à analyser comprenant la séquence, comprenant :
le traitement d'un échantillon de molécules d'ADN à analyser provenant d'un plasma ou de selles avec des ions bisulfite pour modifier sélectivement les résidus cytosine non méthylés, sans dégrader la majorité des molécules d'ADN à analyser, dans lequel l'échantillon provient d'un humain qui est soumis à un dépistage pour ou suspecté d'avoir un adénome du côlon ou un cancer du côlon ;
la formation de microémulsions comprenant les molécules d'ADN à analyser traitées ;
l'amplification d'une partie d'une molécule d'ADN à analyser traitées dans les microémulsions en présence de billes, dans lequel ladite partie comprend un ou plusieurs sites de méthylation de 5'-CpG qui sont hyperméthylés dans les cellules cancéreuses, dans lequel les billes sont liées à une pluralité de molécules d'une amorce pour amplifier les molécules d'ADN à analyser, moyennant quoi il se forme une pluralité de copies de molécule d'ADN à analyser liées par liaison covalente à la pluralité de molécules de l'amorce qui sont liées aux billes ;
la détermination des séquences nucléotidiques au niveau du ou des sites de méthylation de 5'-CpG des molécules d'ADN à analyser qui sont liées aux billes et la quantification des billes qui présentent des sites de méthylation de 5'-CpG modifiés et des billes qui présentent des sites de méthylation de 5'-CpG non modifiés.

2. Procédé selon la revendication 1 dans lequel :
(1) la partie amplifiée de la molécule d'ADN à analyser comprend au moins deux sites de 5'-CpG ; ou
(2) la partie amplifiée de la molécule d'ADN à analyser comprend au moins trois sites de 5'-CpG.

3. Procédé selon la revendication 1 comprenant en outre l'étape d'isolement des billes qui sont liées à la molécule d'ADN à analyser modifiée par rapport aux billes qui sont liées à une molécule d'ADN à analyser non modifiées, dans lequel facultativement l'étape d'isolement est réalisée au moyen d'un tri de cellules activées par fluorescence.

4. Procédé selon la revendication 1 dans lequel avant l'étape de détermination, les microémulsions sont cassées par l'ajout d'un ou de plusieurs détergents.

5. Procédé selon la revendication 1 dans lequel l'étape de détermination est réalisée par :
(1) hybridation à des sondes oligonucléotidiques qui sont marquées de manière différente ;
(2) séquençage des nucléotides ; ou
(3) extension d'amorce.

6. Procédé selon la revendication 1 dans lequel les molécules d'ADN à analyser sont un ADN génomique.

7. Procédé selon la revendication 1 dans lequel
l'échantillon traité par les ions bisulfite est exposé à des conditions alcalines, moyennant quoi les résidus cytosine modifiés sont convertis en résidus uracile.

8. Procédé selon la revendication 1 dans lequel l'échantillon provient :
d'un patient qui a été précédemment traité pour éliminer ou enlever un cancer.

9. Procédé selon la revendication 1 dans lequel l'ADN amplifié comprend :
(1) une partie d'un gène de vimentine ;
(2) une séquence convertie par le bisulfite dérivée de la totalité ou d'au moins 15 nucléotides contigus de
(3) une séquence convertie dérivée de 5'-CTCGTCCTCCT ACCGCAGGAT GTTCGGCGGC CCGGGCACCG CGAGCCGGCC GAGCTCCAGC CGGAGCTACGTGACTACGTC CACCCGCACC TACAGCCTGG GCAGC-3' (SEQ ID NO: 1), dans laquelle la séquence convertie est dérivée en remplaçant chacune d'une ou de plusieurs bases C non méthylées par une base T ;
(4) une séquence convertie par le bisulfate dérivée de la totalité ou d'au moins 15 nucléotides contigus du complément de SEQ ID NO : 1, 5'-GAGCAGGAGGATGGCGTCCTACAAGCCGCCGGGCCCGTGGCGCTCGGCCGG CTCGA GGTCGGCCTCGA TGCACTGA TGCAGGTGGGCGTGGA TGTCGGACCCGTCG -3' (SEQ ID NO: 28); ou
(5) une séquence convertie dérivée de la totalité ou d'au moins 15 nucléotides contigus du complément de SEQ ID NO : 1 dans laquelle la séquence convertie est dérivée en remplaçant chacune d'une ou de plusieurs bases C non méthylées par une base T.

10. Procédé selon la revendication 1 dans lequel l'ADN amplifié comprend

11. Procédé selon la revendication 1 dans lequel :
(1) l'amorce pour amplifier les molécules d'ADN à analyser est choisie dans le groupe constitué de 5'-GTTGTTTAGG TTGTAGGTGN GGG-3 (SEQ ID NO: 2) et de 5'-CTCNTCCTCC TACCNCAAAA TATTC-3'(SEQ ID NO: 3) ; ou
(2) l'amorce pour amplifier les molécules d'ADN à analyser comprend au moins 15 nucléotides contigus choisis dans le groupe constitué de 5'-GTTGTTTAGG TTGTAGGTGN GGG-3 (SEQ ID NO: 2) et de 5'-CTCNTCCTCC TACCNCAAAA TATTC-3' (SEQ ID NO: 3).

12. Procédé selon la revendication 1 dans lequel les séquences nucléotidiques sont déterminées par hybridation à des sondes fluorescentes.

13. Procédé selon la revendication 1 dans lequel la partie amplifiée de l'ADN à analyser comprend entre 50 et 200 nucléotides ou entre 75 et 125 nucléotides.

14. Procédé selon la revendication 1 comprenant en outre un test pour déterminer la présence d'une ou de plusieurs mutations dans un suppresseur de tumeur ou un oncogène dans l'échantillon.

15. Procédé selon la revendication 11 dans lequel :
(1) l'amorce est liée par liaison covalente à la bille ;
(2) l'amorce n'est pas liée par liaison covalente à la bille ; ou
(3) la bille est superparamagnétique.

16. Procédé selon la revendication 1 dans lequel entre 99 et 99,8 % des résidus cytosine non méthylés sont modifiés.

17. Procédé selon la revendication 1 dans lequel la majorité des molécules d'ADN à analyser traitées peut être amplifiée par lesdites amorces.
